# EUROPÄISCHE PATENTSCHRIFT

(11) **EP 0 596 298 B1**
(45) Veröffentlichungstag und Bekanntmachung des Hinweises auf die Patenterteilung: **09.01.2002**
(21) Anmeldenummer: 93116687.0
(22) Anmeldetag: 15.10.1993
(51) Int. Cl.: C07D 209/54, A01N 43/38, C07C 255/46, C07C 233/52

(54) **5-Spiro-substituierte 1-H-3-Aryl-pyrrolidin-2,4-dion-Derivate als Herbizide, Insektizide und Akarizide**
5-Spirosubstituted 1-H-3-aryl-pyrrolidin-2,4-dione derivatives as herbicides, insecticides and acaricides
Dérivés de 1-H-3-aryl-pyrrolidin-2,4-dione 5-spirosubstitués comme herbicides, insecticides et acaricides

(30) Priorität: 28.10.1992 DE 4236401; 11.08.1993 DE 4326909
(43) Veröffentlichungstag der Anmeldung: 11.05.1994
(73) Patentinhaber: BAYER AG, 51368 Leverkusen (DE)
(72) Erfinder: Fischer, Reiner, Dr., D-40789 Monheim (DE); Bretschneider, Thomas, Dr., D-51731 Siegburg (DE); Krüger, Bernd-Wieland, Dr., D-51467 Bergisch Gladbach (DE); Erdelen, Christoph, Dr., D-42799 Leichlingen (DE); Santel, Hans-Joachim, Dr., D-51371 Leverkusen (DE); Lürssen, Klaus, Dr., D-51469 Bergisch Gladbach (DE); Schmidt, Robert R., Dr., D-51467 Bergisch Gladbach (DE); Wachendorff-Neumann, Ulrike, Dr., D-40789 Monheim (DE); Stendel, Wilhelm, Dr., D-42113 Wuppertal (DE)

(56) Entgegenhaltungen:
- EP-A- 0 442 073
- EP-A- 0 456 063
- EP-A- 0 501 129
- EP-A- 0 521 334
- CHEMICAL ABSTRACTS, vol. 53, no. 16, 25. August 1959, Columbus, Ohio, US; abstract no. 14961c, Spalte L ; & ACAD. REP. POPULARE ROMINE, INST. BIOCHIM., STUDII CERCETARI BIOCHIM. Bd. 1 , 1958 Seiten 97 - 107

## Beschreibung

Die Erfindung betrifft neue 1-H-3-Aryl-pyrrolidin-2,4-dion-Derivate, mehrere Verfahren zu ihrer Herstellung und ihre Verwendung als Schädlingsbekämpfungsmittel (insbesondere als Insektizide und Akarizide) und als Herbizide.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985 1095) synthetisiert. Eine biologische Wirksamkeit dieser Verbindungen wurde nicht beschrieben.

In EP-A 0 262 399 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind außerdem unsubstituierte, bicyclische 3-Aryl-pyrrolidin-2,4-dion-Derivate (EP-A 355 599) und (EP 415 211) sowie substituierte mono-cyclische 3-Aryl-pyrrolidin-2,4-dion-Derivate (EP-A 377 893), (EP 442 077) und (EP 497 127).

Weiterhin bekannt sind polycyclische 3-Arylpyrrolidin-2,4-dion-Derivate (EP 442 073), substituierte bicyclische 3-Arylpyrrolidin-2,4-dion Derivate (EP 501 129) sowie 1-H-3-Arylpyrrolidin-dion-Derivate (EP 456 063) und (EP 521 334).

Es wurden nun neue substituierte spirocyclische 1-H-3-Aryl-pyrrolidin-2,4-dion-Derivate der Formel (I) 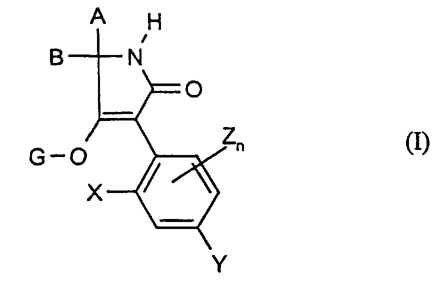 gefunden,
in welcher
- A, B: und das Kohlenstoffatom an das sie gebunden sind für einen C₃-C₆-Spirocyclus, der einfach oder mehrfach durch C₁-C₆-Alkyl, C₃-C₈-Cycloalkyl, C₁-C₃Haloalkyl, C₁-C₆-Alkoxy, C₁-C₄-Thioalkyl, Fluor, Chlor oder Phenyl substituiert sein kann stehen oder
- A, B: und das Kohlenstoffatom, an das sie gebunden sind für einen C₃-C₆-Spirocyclus stehen, der mit einer gegebenenfalls durch ein oder zwei Sauerstoff- oder Schwefelatome unterbrochenen Alkylendiyl- oder durch eine Alkylendioxyl- oder durch eine Alkylendithiooxyl-Gruppe substiuiert ist, die mit dem Kohlenstoffatom, an das sie gebunden ist, einen weiteren fünf- bis siebengliedrigen Spirocyclus bildet oder
- A,B: und das Kohlenstoffatom, an das sie gebunden sind für einen C₃-C₆-Spirocyclus stehen, bei dem zwei Substituenten gemeinsam mit den C-Atomen, an die sie gebunden sind für eine durch Alkyl(C₁-C₃), Alkoxy(C₁-C₃) oder Fluor, Chlor, Brom substituierten gesättigten oder ungesättigten Cyclus stehen, der durch Sauerstoff oder Schwefel unterbrochen sein kann.
- X: für C₁-C₄-Alkyl, Halogen oder C₁-C₄-Alkoxy steht,
- Y: für Wasserstoff, Methyl, Ethyl, Propyl, i-Propyl, Butyl, i-Butyl, Fluor, Chlor, Brom, Methoxy, Ethoxy oder Trifluormethyl steht,
- Z: für Methyl, Ethyl, Propyl, i-Propyl, Butyl, i-Butyl, tert.-Butyl, Fluor, Chlor, Brom, Methoxy oder Ethoxy steht,
- n: für eine Zahl von 0 - 2 steht,
- G: für Wasserstoff (a) oder für die Gruppen der Formel 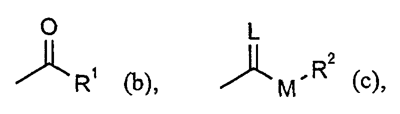 oder E (f) steht, in welcher
- E: für ein Metallionäquivalent oder ein Ammoniumion steht und
- L und M: für Sauerstoff stehen,
- R¹: für gegebenenfalls durch Halogen substituiertes C₁-C₁₆-Alkyl, C₂-C₁₆-Alkenyl, C₁-C₆-Alkoxy-C₂-C₆-alkyl, C₁-C₁₆-Alkylthio-C₂-C₆-alkyl, C₁-C₆-Polyalkoxy-C₂-C₆-alkyl oder Cycloalkyl mit 3 bis 7 Ringatomen, das durch 1 bis 2 Sauerstoff- und/oder Schwefelatome unterbrochen sein kann, steht,
für gegebenenfalls durch Halogen-, Nitro-, C₁-C₄-Alkyl, C₁-C₄-Alkoxy, C₁-C₃-Halogenalkyl-, C₁-C₃-Halogenalkoxy-substituiertes Phenyl,
für gegebenenfalls durch Halogen-, C₁-C₄-Alkyl, C₁-C₄-Alkoxy, C₁-C₃-Halogenalkyl-, C₁-C₃-Halogenalkoxy-substituiertes Phenyl-C₁-C₄-alkyl steht,
für gegebenenfalls durch Fluor, Chlor, Brom- und/oder C₁-C₄-Alkyl-substituiertes Hetaryl steht,
für gegebenenfalls durch Fluor, Chlor, Brom und C₁-C₄-Alkyl-substituiertes Phenoxy-C₁-C₅-alkyl steht,
für gegebenenfalls durch Fluor, Chlor, Brom, Amino und C₁-C₄-Alkyl-substituiertes Hetaryloxy-C₁-C₅-alkyl steht,
- R²: für gegebenenfalls durch Halogen substituiertes C₁-C₁₆-Alkyl, C₃-C₁₆-Alkenyl, C₁-C₆-Alkoxy-C₂-C₆-alkyl, C₁-C₆-Polyalkoxy-C₂-C₆-alkyl steht,
für gegebenenfalls durch Halogen, Nitro, C₁-C₄-Alkyl-, C₁-C₃-Alkoxy-, C₁-C₃-Halogenalkyl-substituiertes Phenyl oder Benzyl steht,
Unter Einbeziehung der verschiedenen Bedeutungen (a), (b), (c) und (f) der Gruppe G der allgemeinen Formel (I) ergeben sich folgende hauptsächlichen Strukturen (Ia) bis (Ig): 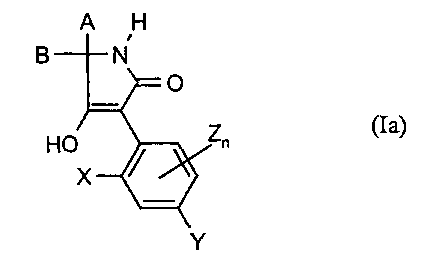 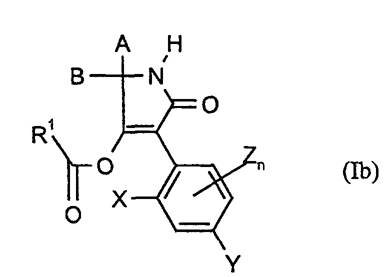 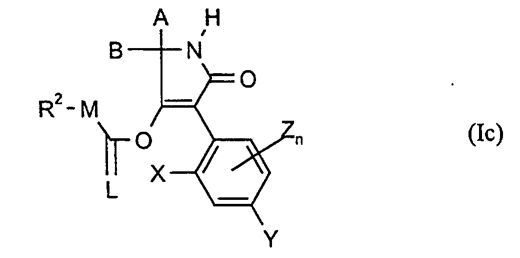 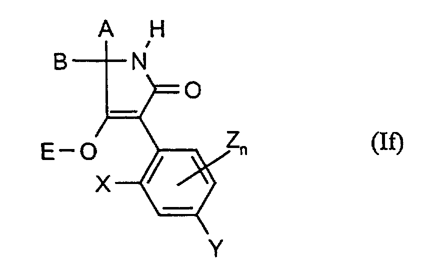 worin
A, B, E, L, M, X, Y, Z, R¹, R² und n die oben angegebenen Bedeutungen besitzen.

Aufgrund eines oder mehrerer Chiralitätszentren, fallen die Verbindungen der Formel (Ia), (Ib), (Ic) und (If) im allgemeinen als Stereoisomerengemisch an, die gegebenenfalls in üblicher Art und Weise getrennt werden können. Sie können sowohl in Form ihrer Diastereomerengemische als auch als reine Diastereomere oder Enantiomere verwendet werden. Im folgenden wird der Einfachheit halber stets von Verbindungen der Formel (Ia) bis (Ic) und (If) gesprochen, obwohl sowohl die reinen Verbindungen, als auch die Gemische mit unterschiedlichen Anteilen an isomeren, enantiomeren und stereomeren Verbindungen gemeint sind.

Weiterhin wurde gefunden, daß man die neuen substituierten 1-H-3-Aryl-pyrrolidin-2,4-dion-Derivate der Formel (I) nach einem der im folgenden beschriebenen Verfahren erhält.
(A) Man erhält 1-H-3-Aryl-pyrrolidin-2,4-dione bzw. deren Enole der Formel (Ia) 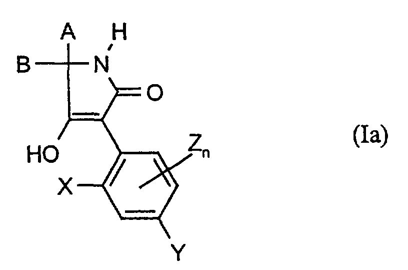 in welcher
   - A, B, X, Y, Z und n: die oben angegebene Bedeutung haben,
   wenn man
   N-Acylaminosäureester der Formel (II) 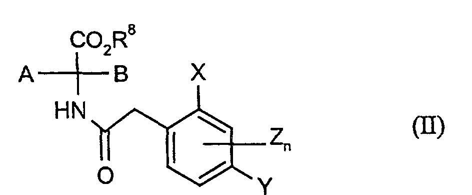 in welcher
   - A, B, X, Y, Z und n: die oben angegebene Bedeutung haben, und
   - R⁸: für Alkyl steht,
   in Gegenwart eines Verdünnungsmittels und in Gegenwart einer Base intramolekular kondensiert;
   oder
(B) man erhält Verbindungen der Formel (Ib) 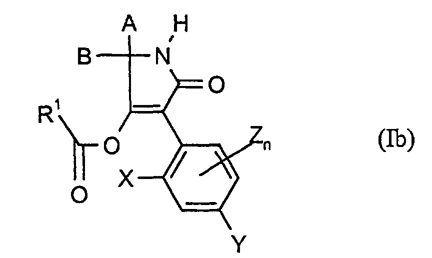 in welcher
   - A, B, X, Y, Z, R¹ und n: die oben angegebene Bedeutung haben,
   wenn man Verbindungen der Formel (Ia), 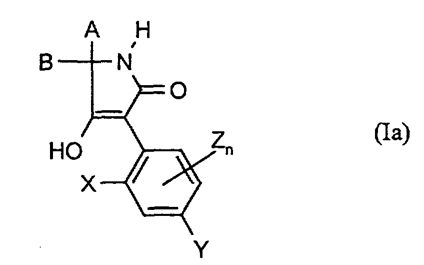 in welcher
   - A, B, X, Y, Z und n: die oben angegebene Bedeutung haben,

   a) mit Säurehalogeniden der allgemeinen Formel (III) 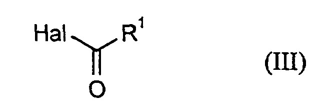 in welcher
      - R¹: die oben angegebene Bedeutung hat und
      - Hal: für Halogen, insbesondere Chlor und Brom steht,
      gegebenenfalls in Gegenwart eines Verdünnungsmittels und gegebenenfalls in Gegenwart eines Säurebindemittels umsetzt
      oder
   β) mit Carbonsäureanhydriden der allgemeinen Formel (IV)

      R¹-CO-O-CO-R¹ (IV)

      in welcher
      - R¹: die oben angegebene Bedeutung hat,
      gegebenenfalls in Gegenwart eines Verdünnungsmittels und gegebenenfalls in Gegenwart eines Säurebindemittels,
   umsetzt;
   oder
(C) man erhält Verbindungen der Formel (Ic-1) 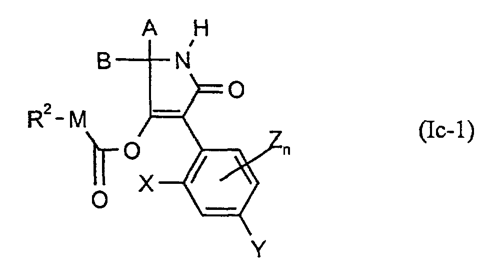 in welcher
   - A, B, X, Y, Z, R² und n: die oben angegebene Bedeutung haben,
   und
   - M: für Sauerstoff oder Schwefel steht,
   wenn man Verbindungen der Formel (Ia) 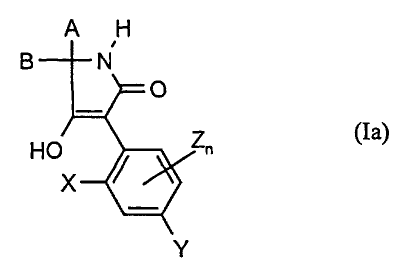 in welcher
   - A, B, X, Y, Z und n: die oben angegebene Bedeutung haben,
   mit Chlorameisensäureester oder Chlorameisensäurethiolester der allgemeinen Formel (V)

   R²-M-CO-Cl (V)

   in welcher
   - R² und M: die oben angegebene Bedeutung haben,
   gegebenenfalls in Gegenwart eines Verdünnungsmittels und gegebenenfalls in Gegenwart eines Säurebindemittels umsetzt;
   oder
(G) man erhält Verbindungen der Formel (If) 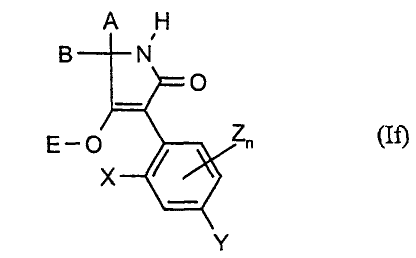 in welcher
   - A, B, X, Y, Z und n: die oben angegebene Bedeutung haben,
   und
   - E: für ein Metallionäquivalent oder für ein Ammoniumion steht,
   wenn man Verbindungen der Formel (Ia) 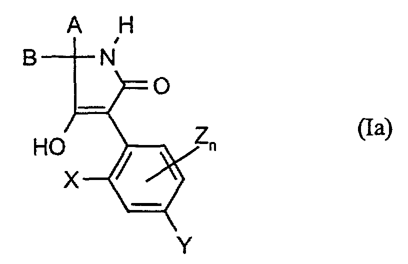 in welcher
   - A, B, X, Y, Z und n: die oben angegebene Bedeutung haben,
   mit Metallhydroxiden oder Aminen der allgemeinen Formeln (X) und (XI)

   MeₛOHₜ (X)

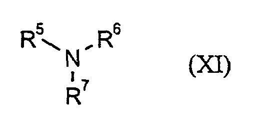

   in welchen
   Me für ein- oder zweiwertige Metallionen,
   s und t für die Zahl 1 und 2 und
   R⁵, R⁶ und R⁷ unabhängig voneinander für Wasserstoff und Alkyl stehen,
   gegebenenfalls in Gegenwart eines Verdünnungsmittels, umsetzt.
Weiterhin wurde gefunden, daß sich die neuen 1-H-3-Arylpyrrolidin-2,4-dion-Derivate der Formel (I) durch hervorragende insektizide, akarizide und herbizide Wirkungen auszeichnen.

Bevorzugt für die allgemeinen Formeln der vorliegenden Anmeldung gilt, daß:
- A, B: und das Kohlenstoffatom an das sie gebunden sind für einen C₃-C₆-Spirocyclus, der mindestens einfach oder mehrfach durch Methyl, Ethyl, Propyl, Isopropyl, Butyl, iso-Butyl, sec.-Butyl, tert,-Butyl, Cyclohexyl, Trifluormethyl, Methoxy, Ethoxy, Propoxy, Isopropoxy, Butoxy, Isobutoxy, sek.-Butoxy, tert.-Butoxy, Methylthio, Fluor, Chlor oder Phenyl substituiert sein kann stehen oder
- A, B: und das Kohlenstoffatom, an das sie gebunden sind für einen C₃-C₆-Spirocyclus stehen, der mit einer gegebenenfalls durch ein Sauerstoff- oder Schwefelatom unterbrochenen Alkylendiyl- oder durch eine Alkylendioxyl-Gruppe substituiert ist, die mit dem Kohlenstoffatom, an das sie gebunden ist, einen weiteren fünf- bis siebengliedrigen Spirocyclus bildet oder
- A,B: und das Kohlenstoffatom, an das sie gebunden sind für einen C₃-C₆-Spirocyclus stehen, bei dem zwei Substituenten gemeinsam mit den C-Atomen, an die sie gebunden sind für einen gesättigten oder ungesättigten fünf- oder sechsgliedrigen Cyclus stehen, der durch Sauerstoff oder Schwefel unterbrochen sein kann.
- X: für Methyl, Ethyl, Propyl, 2-Propyl, Fluor, Chlor, Brom, Methoxy oder Ethoxy steht,
- Y: für Wasserstoff, Methyl, Ethyl, Propyl, i-Propyl, Butyl, i-Butyl, tert.-Butyl, Fluor, Chlor, Brom, Methoxy, Ethoxy oder Trifluormethyl steht,
- Z: für Methyl, Ethyl, Propyl, i-Propyl, Butyl, i-Butyl, tert.-Butyl, Fluor, Chlor, Brom, Methoxy oder Ethoxy steht,
- n: für eine Zahl von 0 - 1 steht,
- G: für Wasserstoff (a) oder für die Gruppen der Formeln 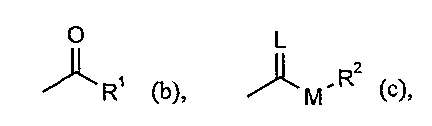 oder E (f) steht, in welchen
- E: für ein Metallionäquivalent oder ein Ammoniumion steht und
- L und M: für Sauerstoff stehen,
- R¹: für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Alkylthio-C₂-C₆-alkyl, C₁-C₄-Polyalkoxy-C₂-C₄-alkyl oder Cycloalkyl mit 3 bis 6 Ringatomen, das durch 1 bis 2 Sauerstoff- und/oder Schwefelatome unterbrochen sein kann, steht,
für gegebenenfalls durch Fluor, Chlor, Brom, Methyl, Ethyl, Propyl, i-Propyl, Methoxy, Ethoxy, Trifluormethyl, Trifluormethoxy, Nitro-substituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy-substituiertes Phenyl-C₁-C₃-alkyl steht,
für gegebenenfalls durch Fluor, Chlor, Brom, Methyl, Ethyl-substituiertes Furanoyl, Pyridyl, Pyrimidyl, Thiazolyl und Pyrazolyl steht,
für gegebenenfalls durch Fluor, Chlor, Methyl, Ethyl-substituiertes Phenoxy-C₁-C₄-alkyl steht,
für gegebenenfalls durch Fluor, Chlor, Amino, Methyl-, Ethyl, substituiertes Pyridyloxy-C₁-C₄-alkyl, Pyrimidyloxy-C₁-C₄-alkyl und Thiazolyloxy-C₁-C₄-alkyl steht,
- R²: für gegebenenfalls durch Fluor oder Chlor substituiertes C₁-C₁₄-Alkyl, C₃-C₁₄-Alkenyl, C₁-C₄-Alkoxy-C₂-C₆-alkyl, C₁-C₄-Polyalkoxy-C₂-C₆-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl oder Benzyl steht.

Im einzelnen seien außer den bei den Herstellungsbeispielen genannten Verbindungen die folgenden Verbindungen der Formel (Ia) genannt:

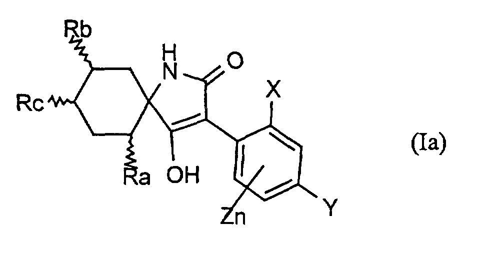

**Tabelle 1:**

| **X** | **Y** | **Z**_{**n**} | **R**_{**a**} | **R**_{**b**} | **R**_{**c**} |
|---|---|---|---|---|---|
| Cl | Cl | H | H | CH₃ | H |
| Cl | Cl | H | H | H | CH₃ |
| Cl | Cl | H | H | H | C₂H₅ |
| Cl | Cl | H | H | H | i-C₃H₇ |
| Cl | Cl | H | H | H | t-C₄H₉ |
| Cl | Cl | H | H | CH₃ | CH₃ |
| Cl | H | 6-F | CH₃ | H | H |
| Cl | H | 6-F | H | CH₃ | H |
| Cl | H | 6-F | H | H | CH₃ |
| Cl | H | 6-F | H | H | C₂H₅ |
| Cl | H | 6-F | H | H | i-C₃H₇ |
| Cl | H | 6-F | H | H | t-C₄H₉ |
| Cl | H | 6-F | H | CH₃ | CH₃ |
| Cl | H | 6-Cl | CH₃ | H | H |
| Cl | H | 6-Cl | H | CH₃ | H |
| Cl | H | 6-Cl | H | H | CH₃ |
| Cl | H | 6-Cl | H | H | C₂H₅ |
| Cl | H | 6-Cl | H | H | i-C₃H₇ |
| Cl | H | 6-Cl | H | H | t-C₄H₉ |
| Cl | H | 6-Cl | H | CH₃ | CH₃ |
| CH₃ | CH₃ | H | CH₃ | H | H |
| CH₃ | CH₃ | H | H | CH₃ | H |
| CH₃ | CH₃ | H | H | H | CH₃ |
| CH₃ | CH₃ | H | H | H | C₂H₅ |
| CH₃ | CH₃ | H | H | H | i-C₃H₇ |
| CH₃ | CH₃ | H | H | H | t-C₄H₉ |
| CH₃ | CH₃ | H | H | CH₃ | CH₃ |
| CH₃ | CH₃ | 6-CH₃ | H | CH₃ | H |
| CH₃ | CH₃ | 6-CH₃ | H | H | C₂H₅ |
| CH₃ | CH₃ | 6-CH₃ | H | H | i-C₃H₇ |
| CH₃ | CH₃ | 6-CH₃ | H | H | t-C₄H₉ |
| CH₃ | CH₃ | 6-CH₃ | H | CH₃ | CH₃ |

Im einzelnen seien außer den bei den Herstellungsbeispielen genannten Verbindungen die folgenden Verbindungen der Formel (Ib) genannt:

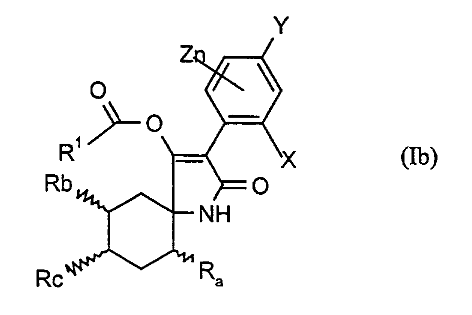

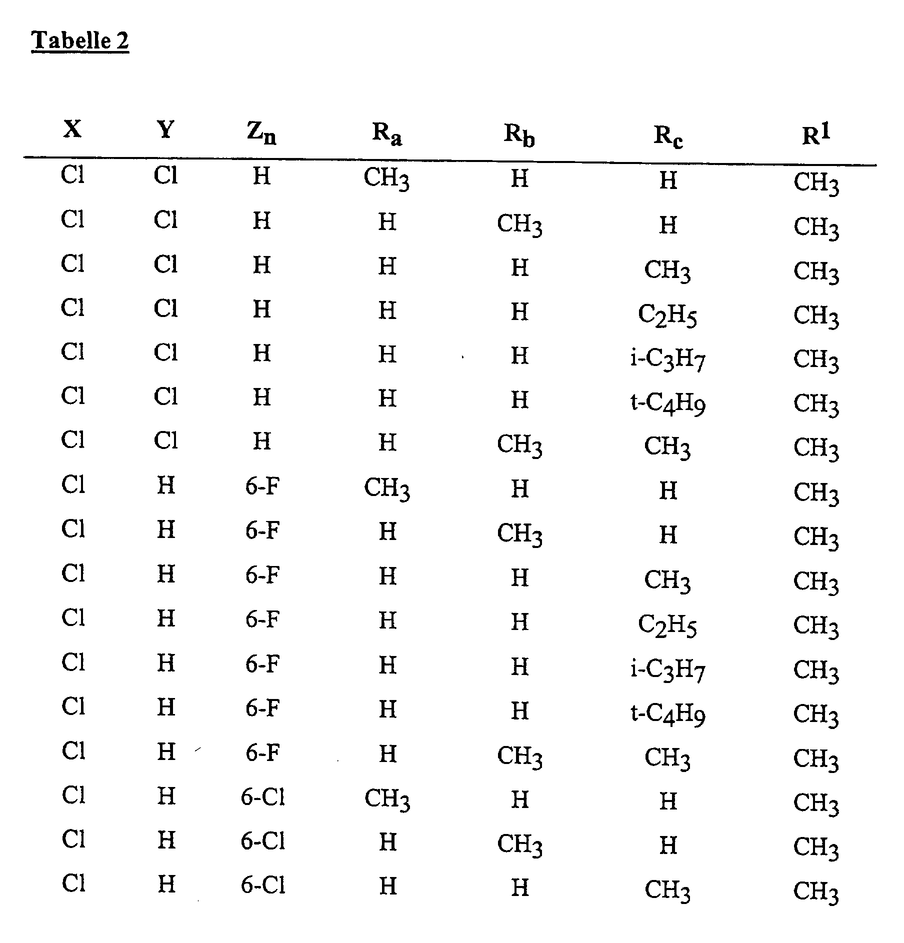

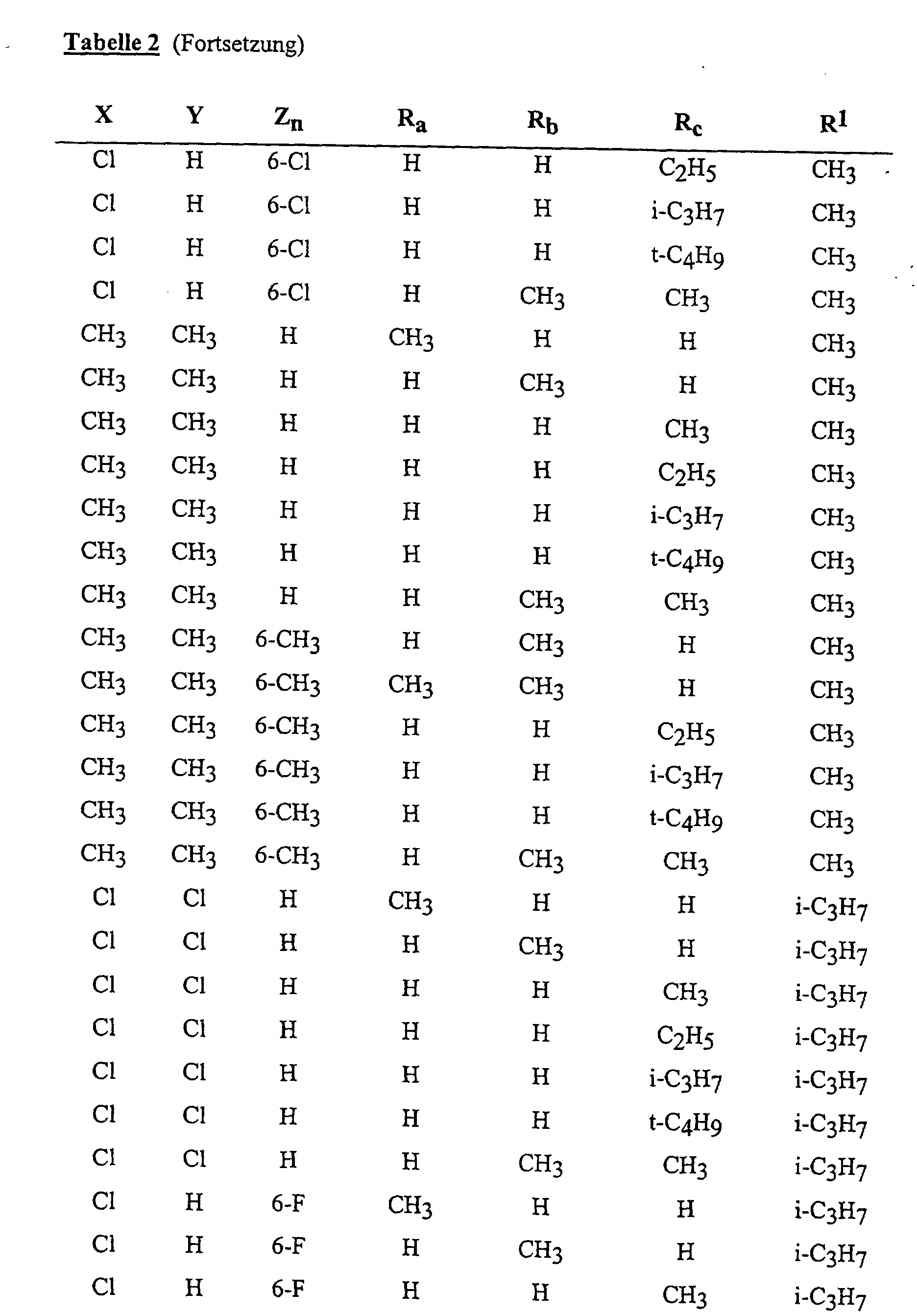

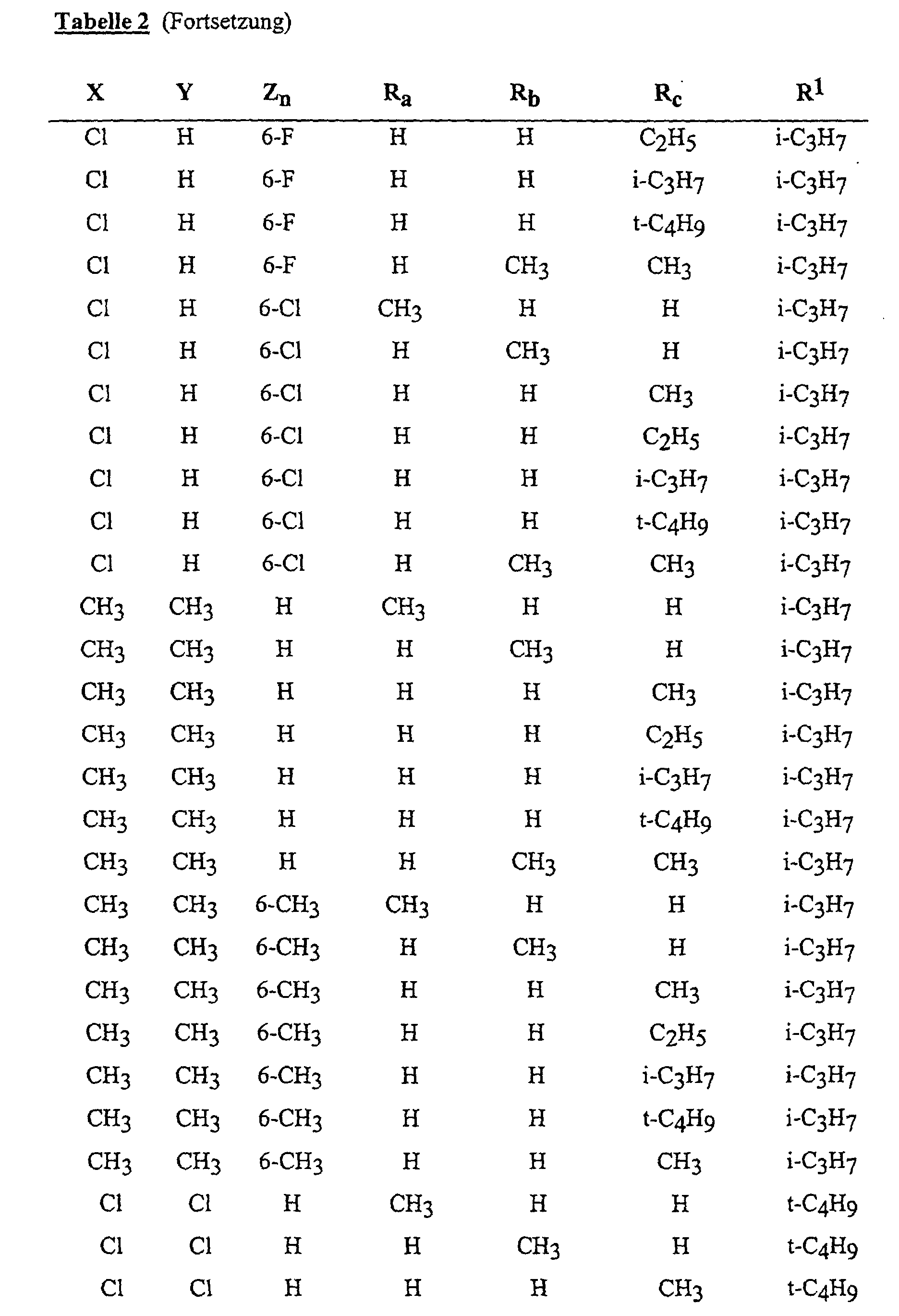

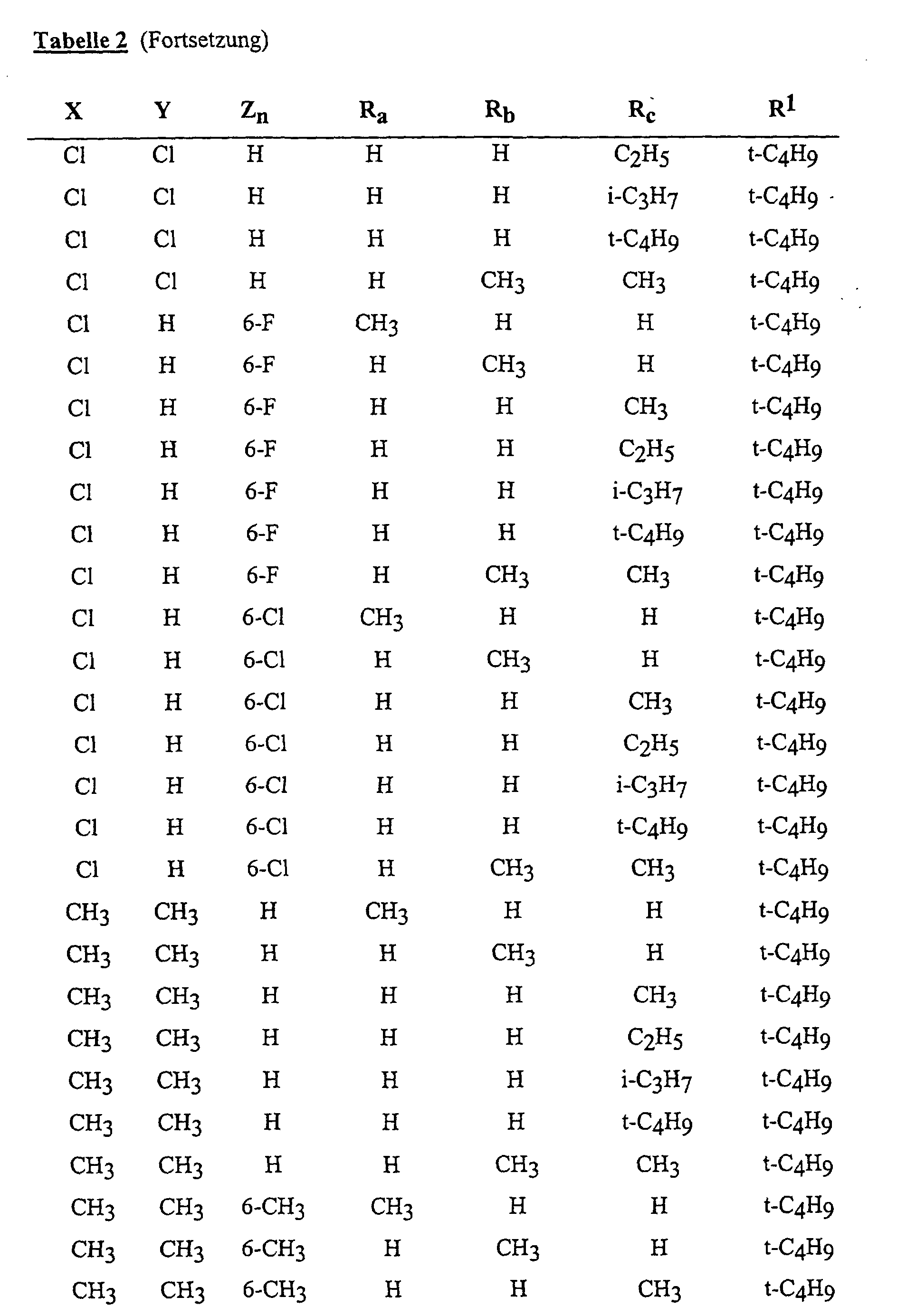

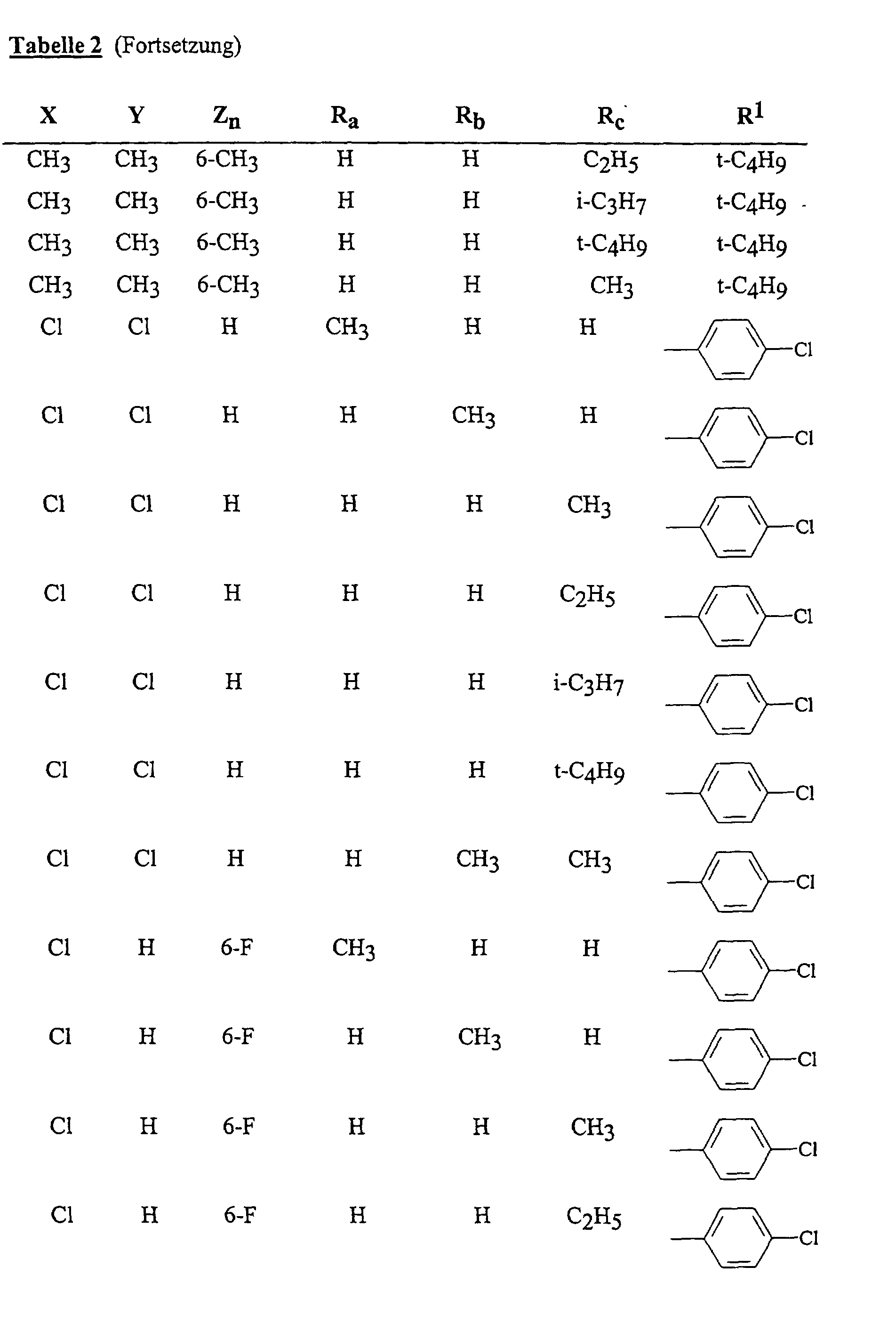

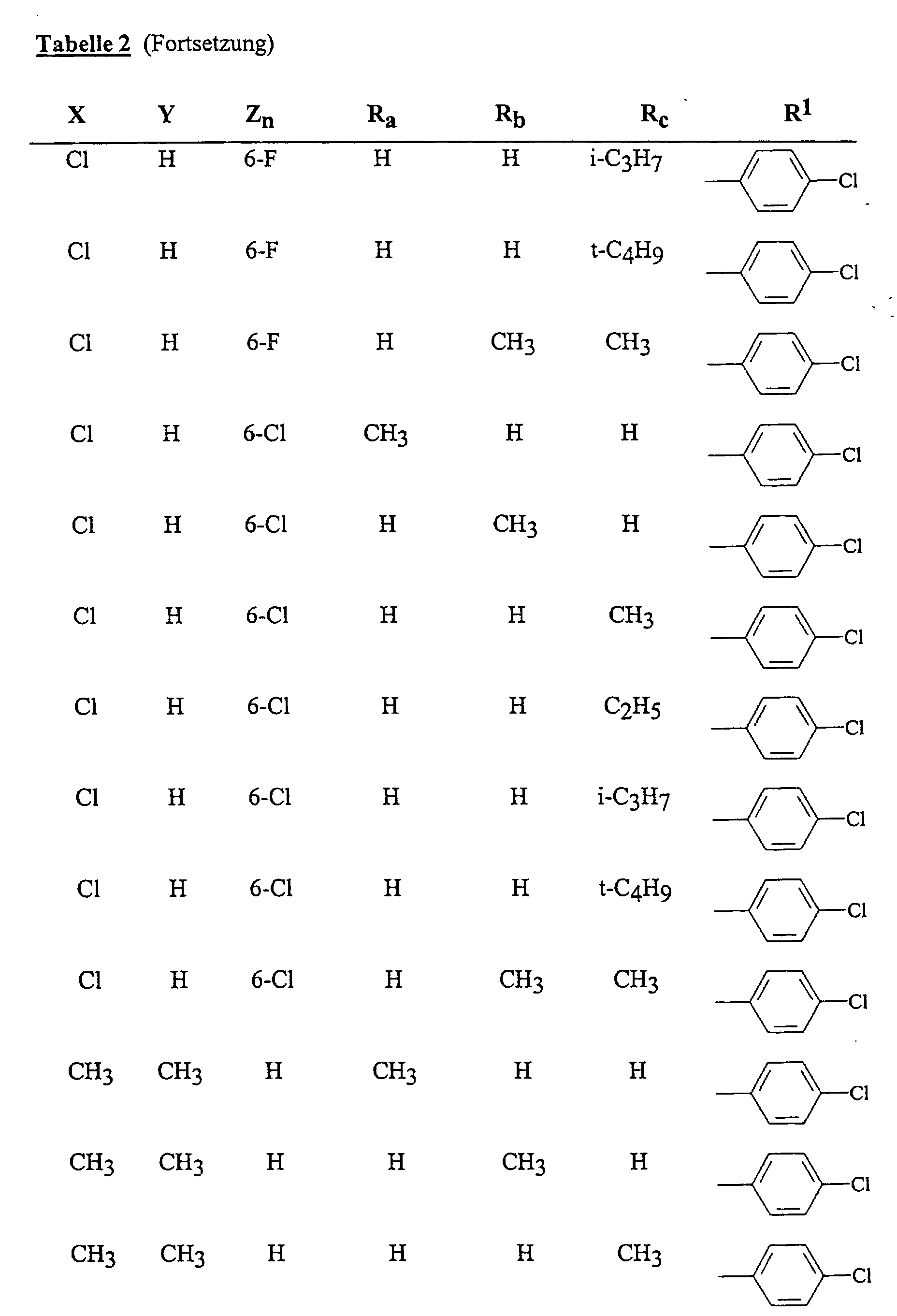

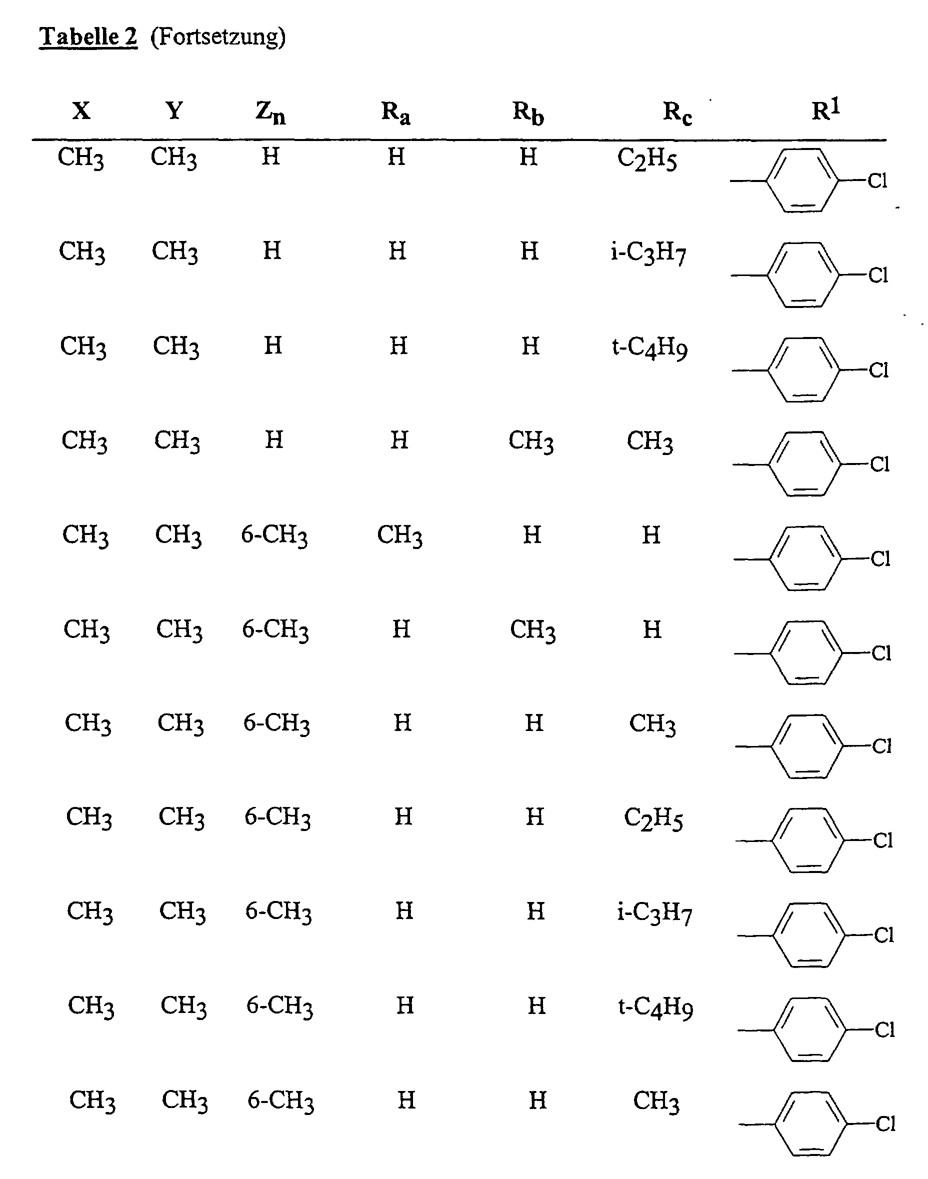

Im einzelnen seien außer den bei den Herstellungsbeispielen genannten Verbindungen die folgenden Verbindungen der Formel (Ic) genannt:

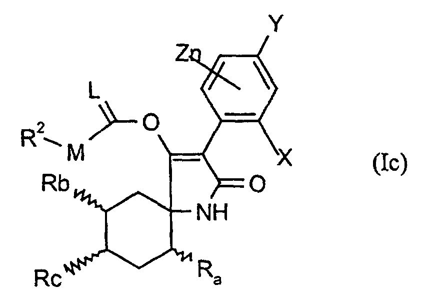

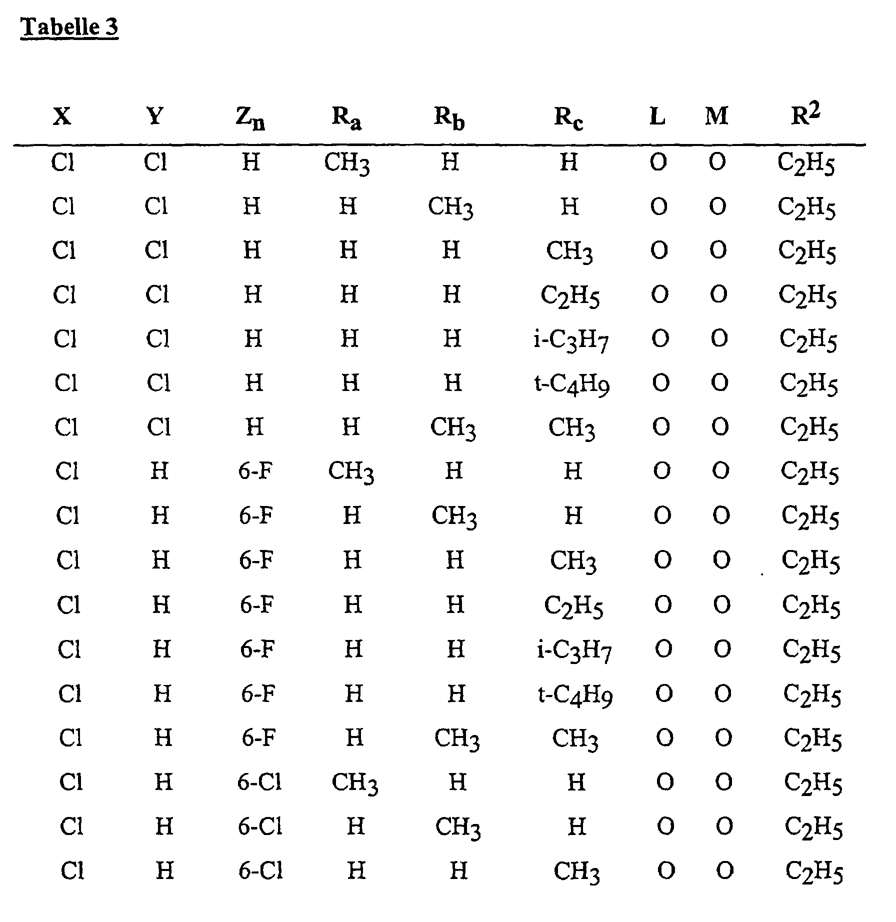

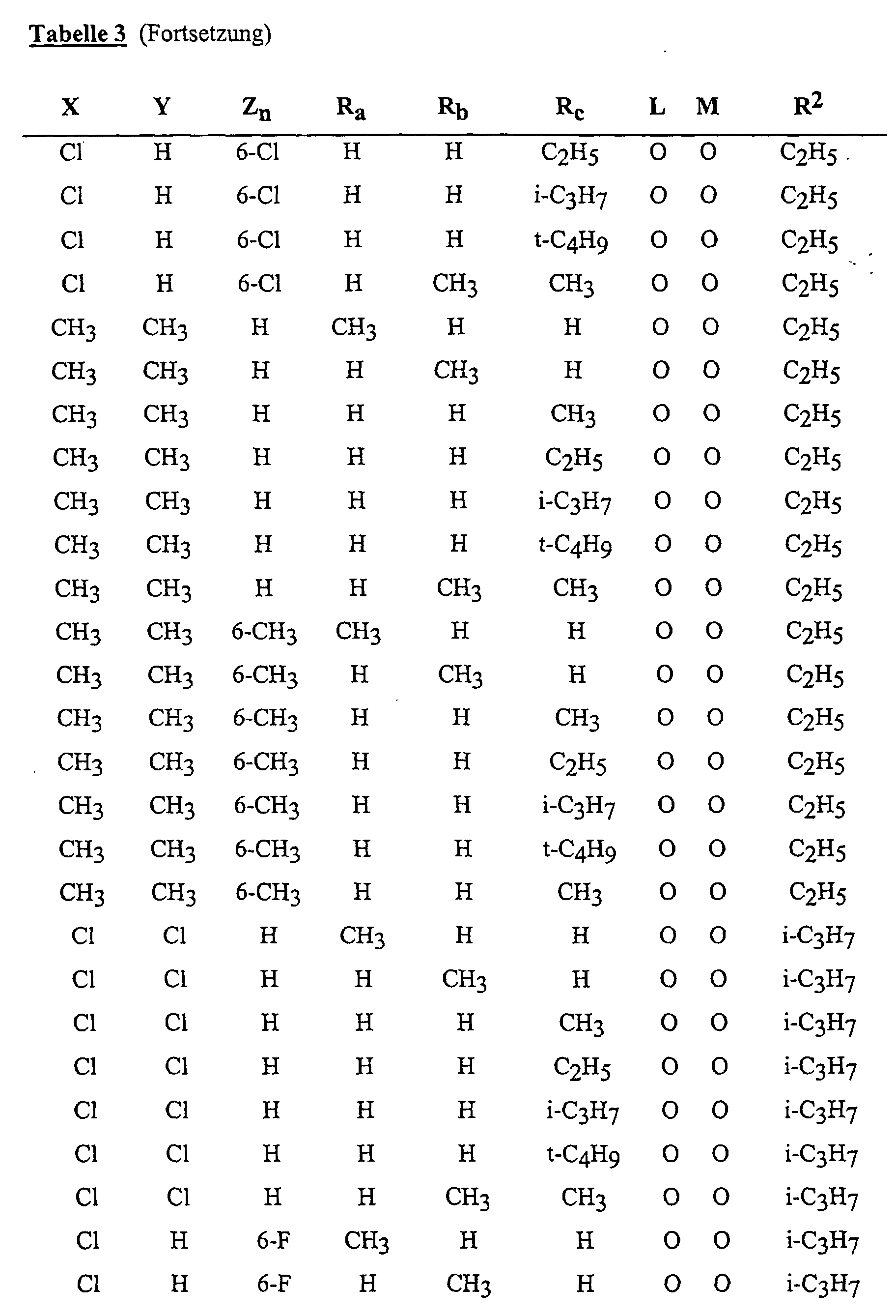

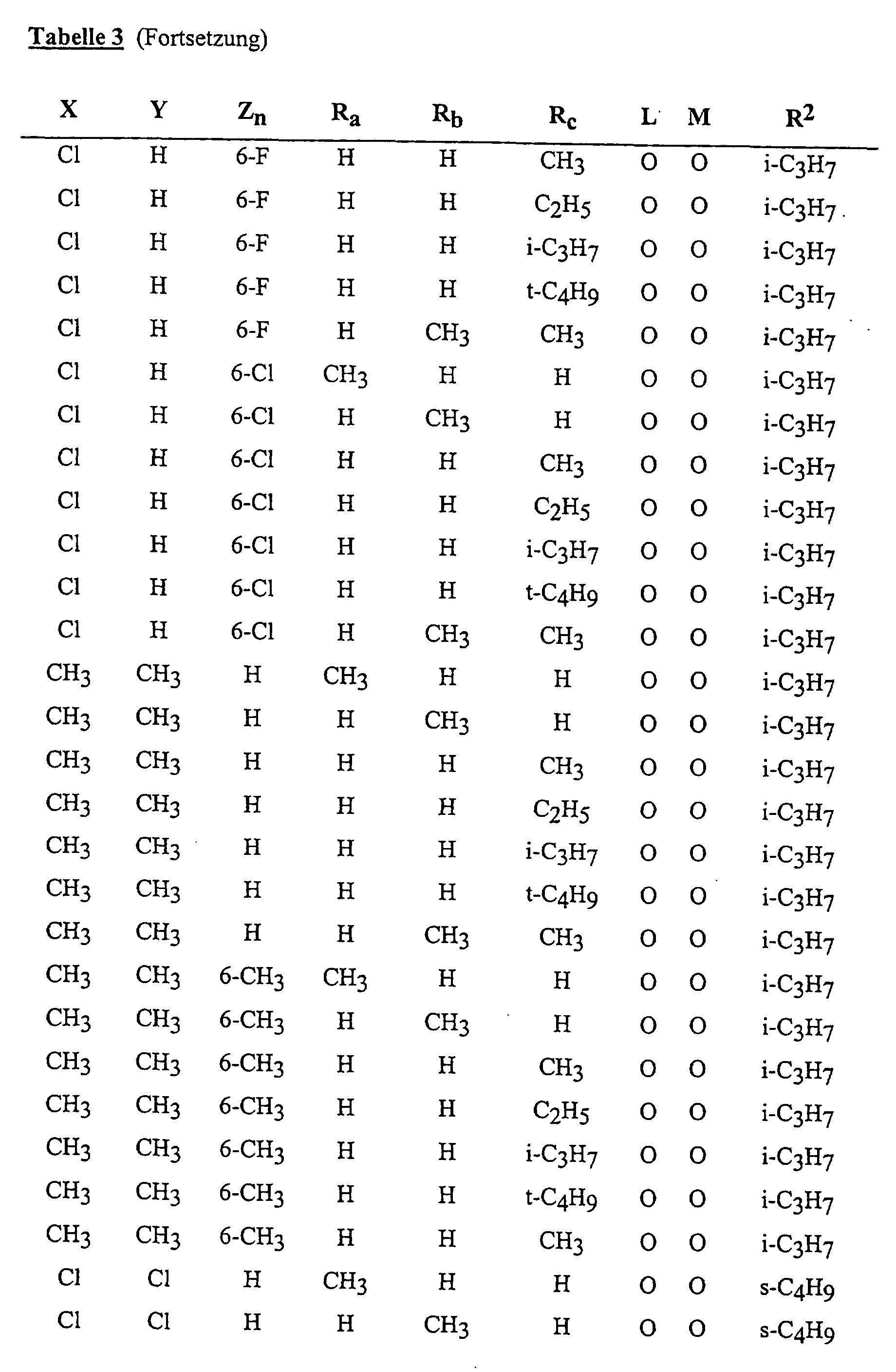

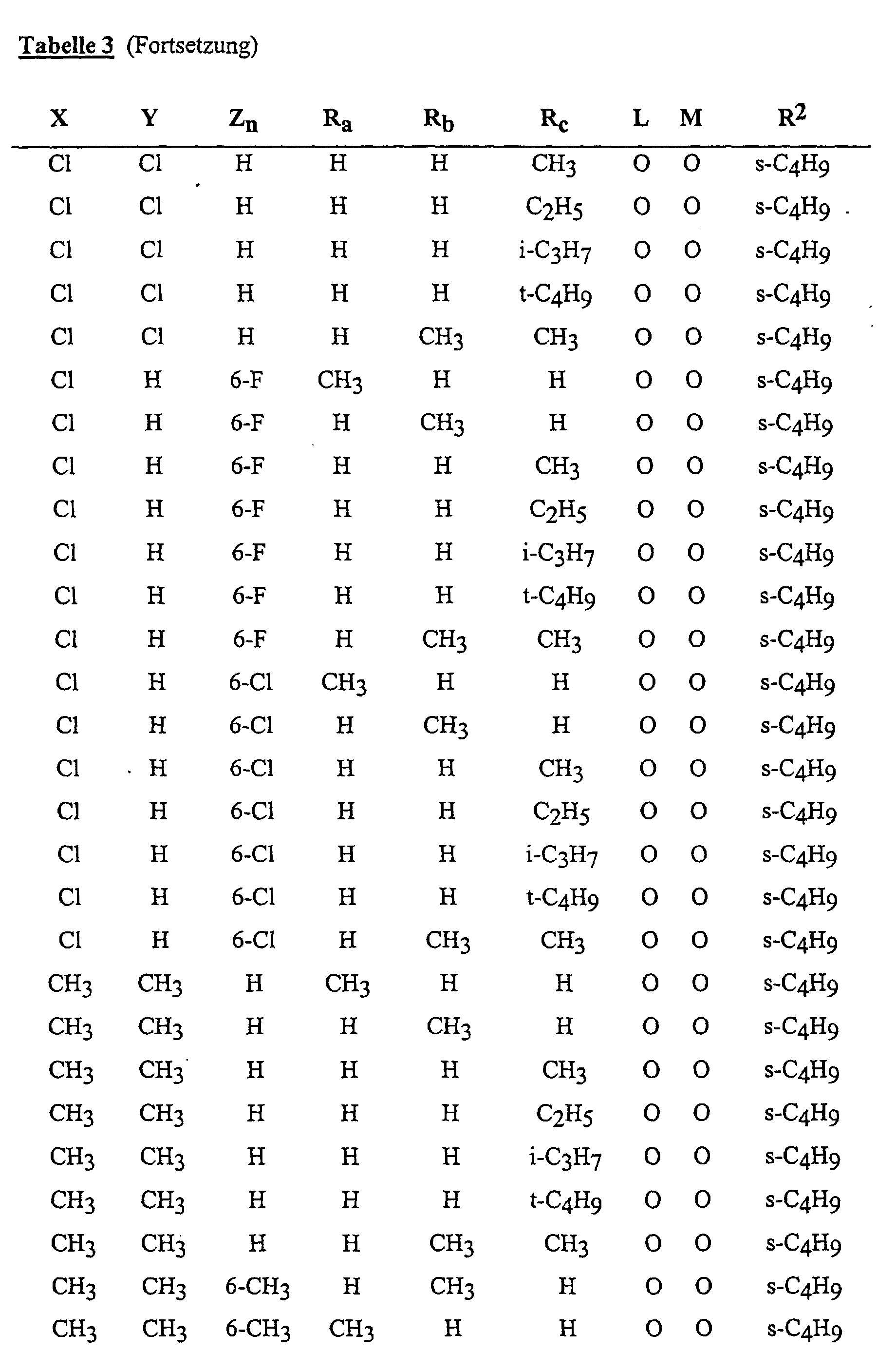

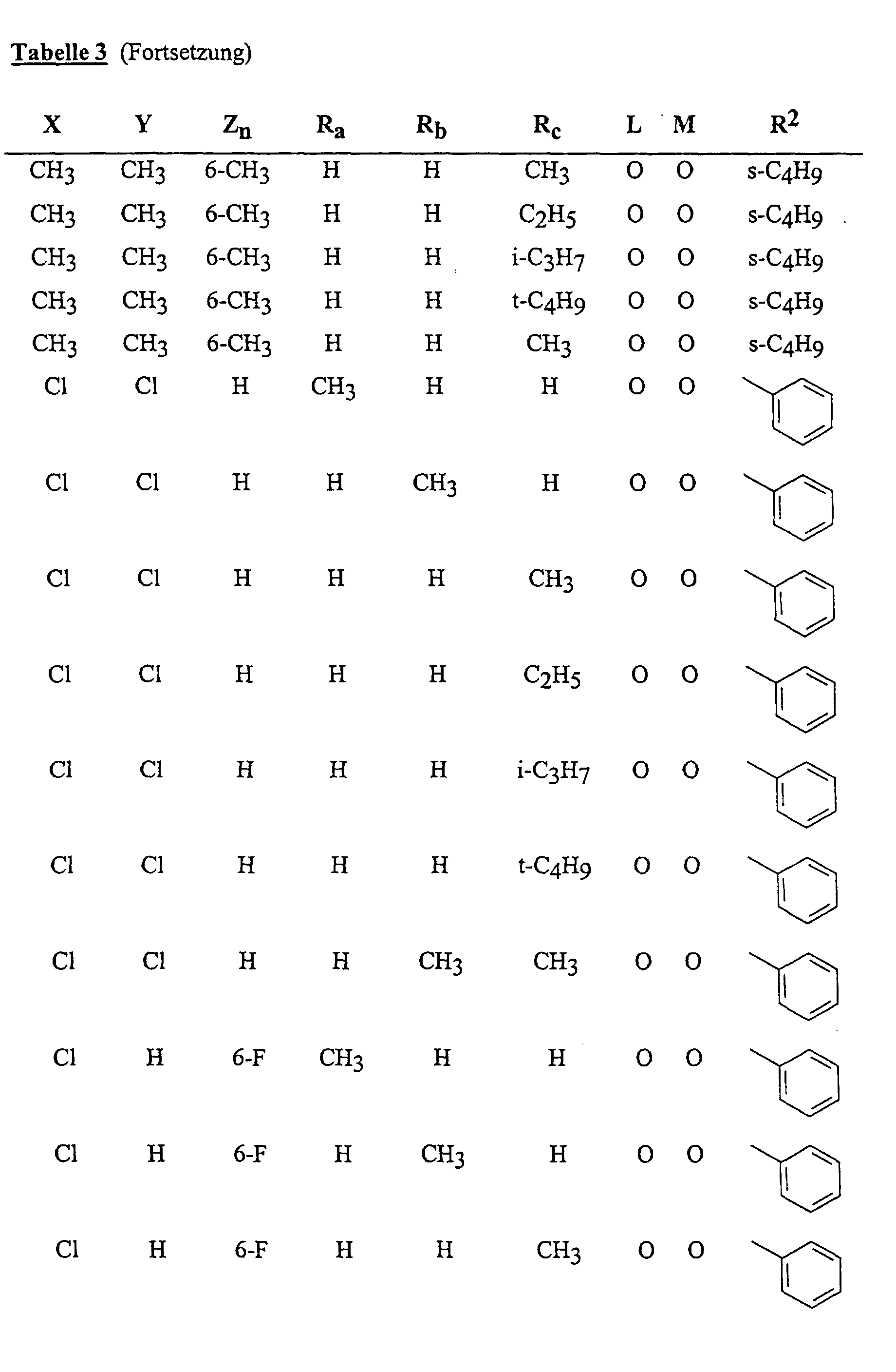

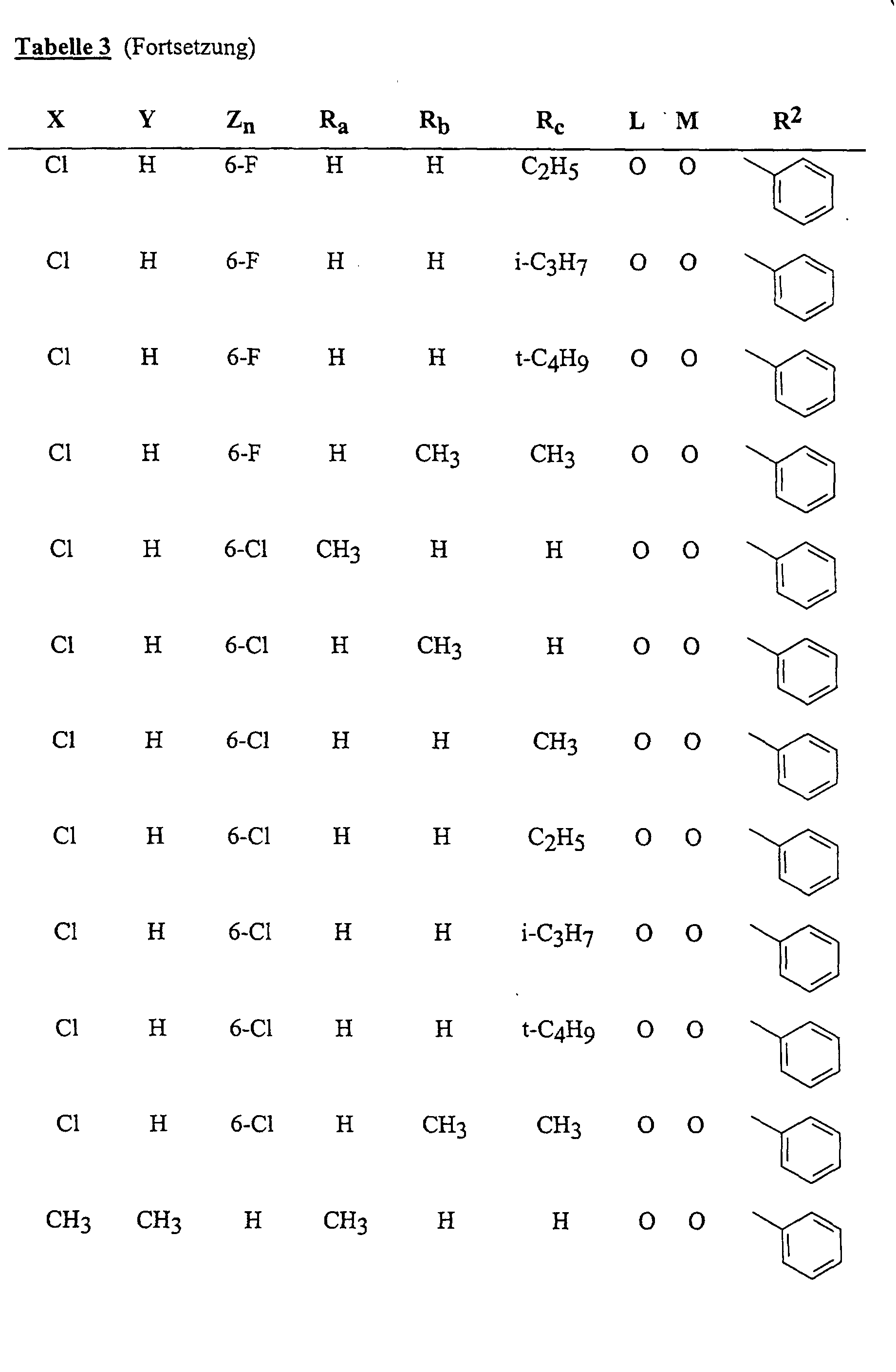

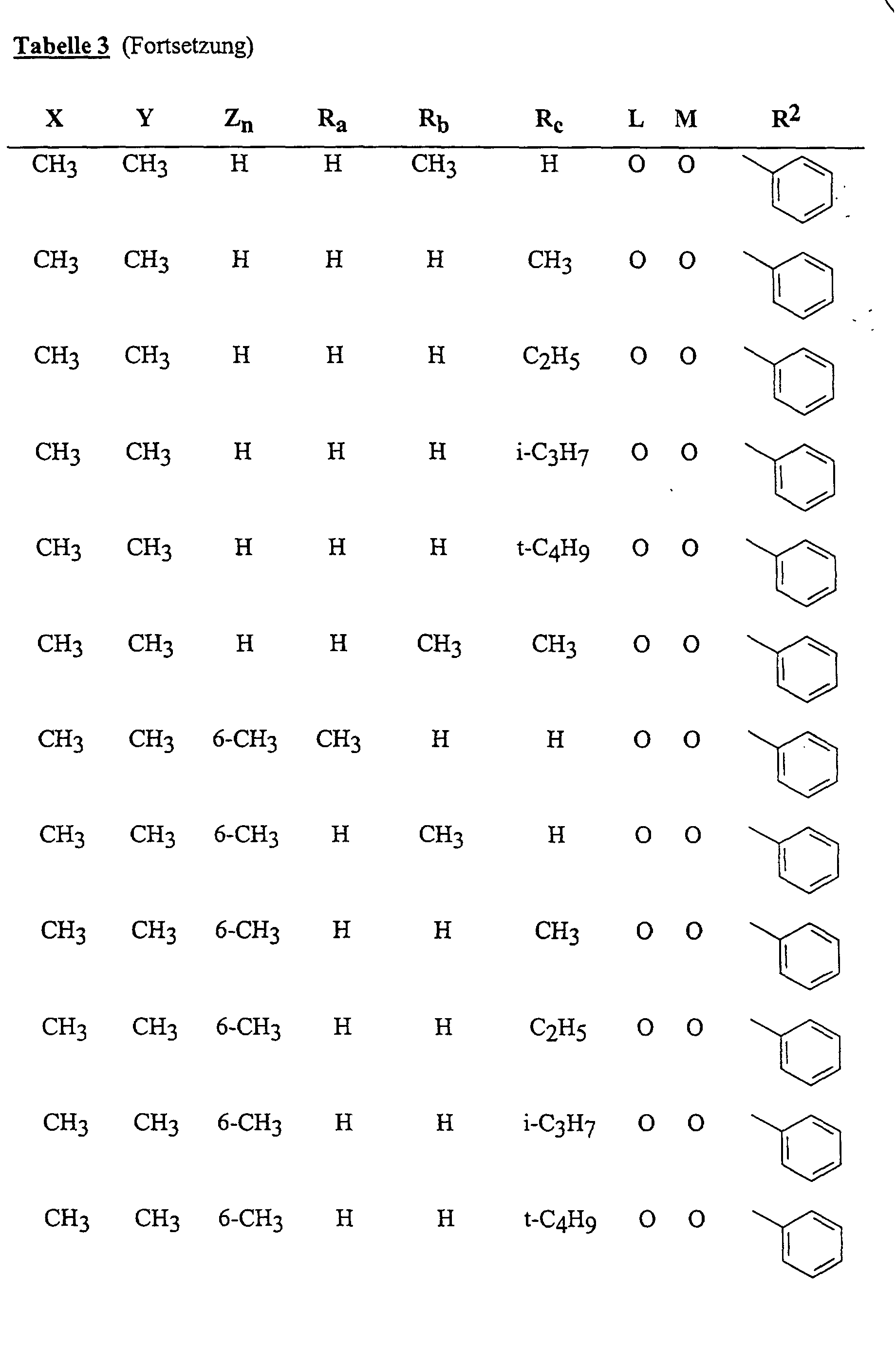

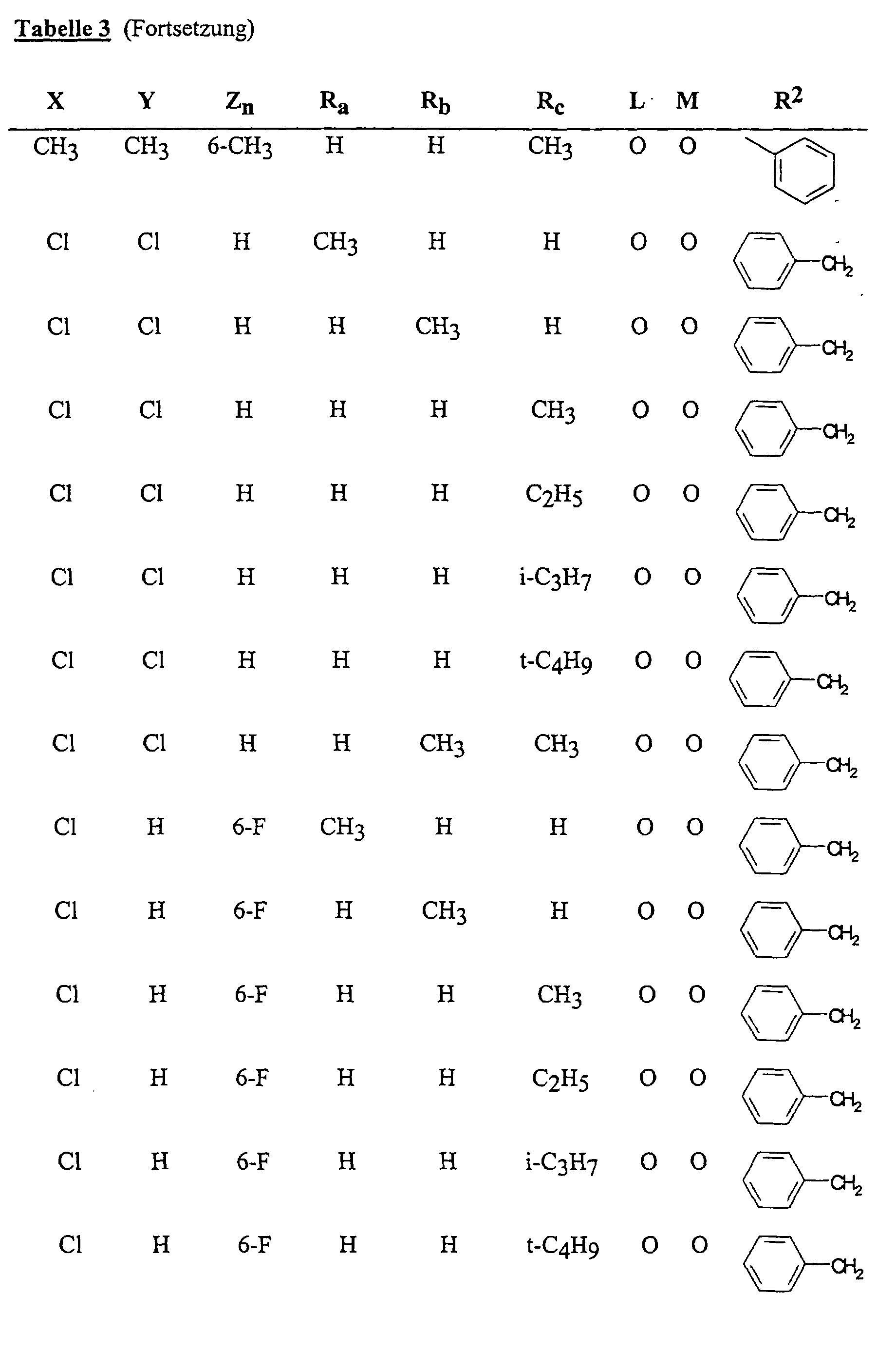

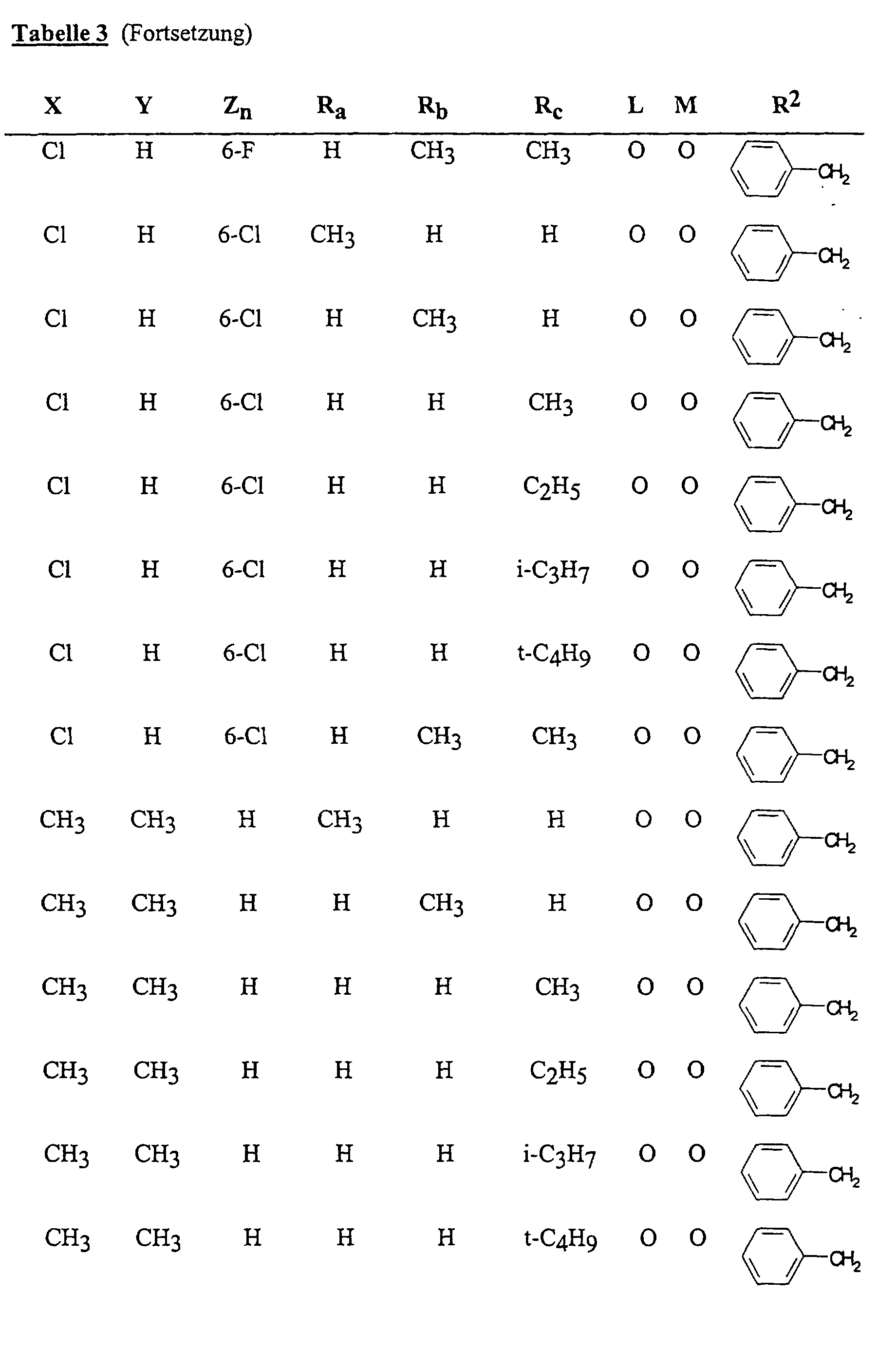

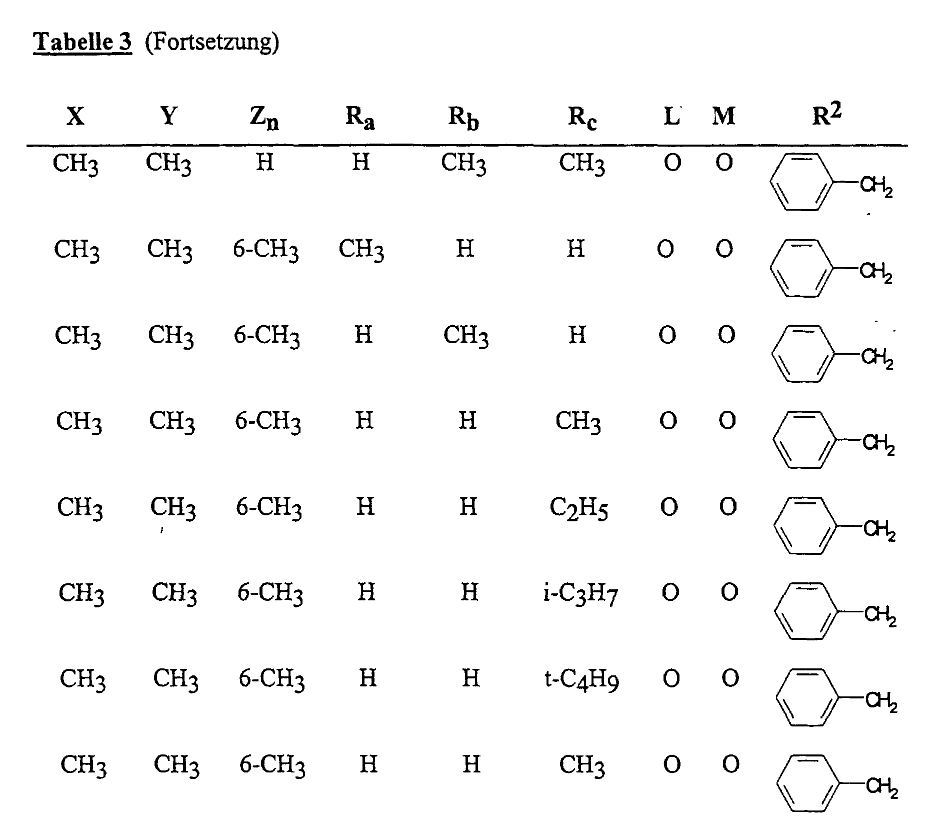

Verwendet man gemäß Verfahren (A) N-2,4-Dichlorphenylacetyl-1-amino-4-ethylcyclohexan-carbonsäureethylester, so kann der Verlauf des erfindungsgemäßen Verfahrens durch folgendes Reaktionsschema wiedergegeben werden:

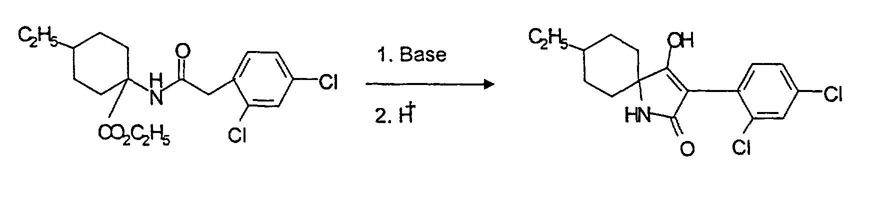

Verwendet man gemäß Verfahren (B) (Variante a) 3-(2,4,6-Trimethylphenyl)-5,5-(3-methyl)-pentamethylen-pyrrolidin-2,4-dion und Pivaloylchlorid als Ausgangsstoffe, so kann der Verlauf des erfindungsgemäßen Verfahrens durch folgendes Reaktionsschema wiedergegeben werden:

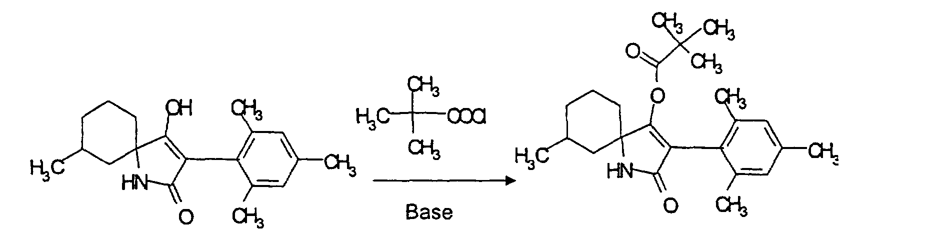

Verwendet man gemäß Verfahren B (Variante β) 3-(2,4,6-Trimethylphenyl)-5,5-(tetramethyl)-dimethylen-pyrrolidin-2,4-dion und Acetanhydrid als Ausgangsverbindungen, so kann der Verlauf des erfindungsgemäßen Verfahrens durch folgendes Reaktionsschema wiedergegeben werden:

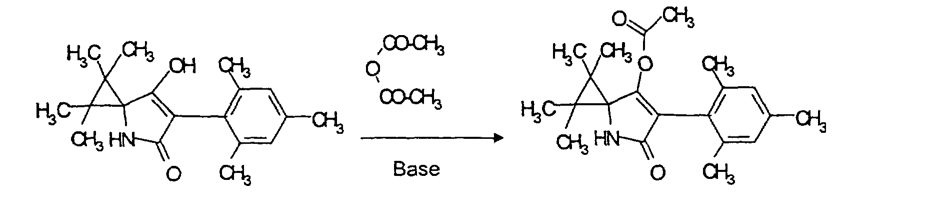

Verwendet man gemäß Verfahren (C) 3-(2,4,6-Trimethylphenyl)-5,5-(3-methyl)-tetramethylen-pyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden.

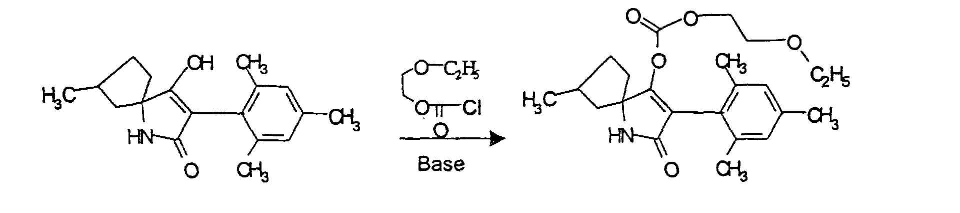

Verwendet man gemäß Verfahren (G) 3-(2,4,6-Trimethylphenyl)-5,5-(4-tert-butyl)-pentamethylen-pyrrolidin-2,4-dion und NaOH als Komponenten, so kann der Verlauf des erfindungsgemäßen Verfahrens durch folgendes Reaktionsschema wiedergegeben werden:

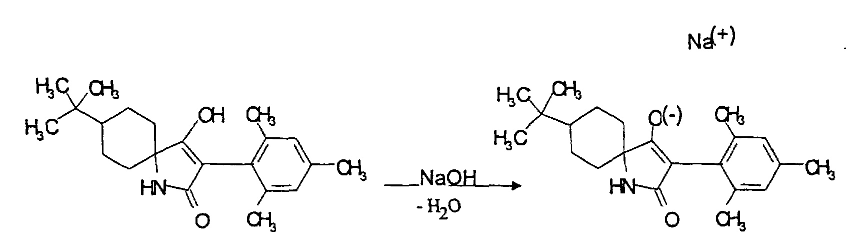

Die bei den erfindungsgemäßen Verfahren (A) als Ausgangsstoffe benötigten Verbindungen der Formel (II) 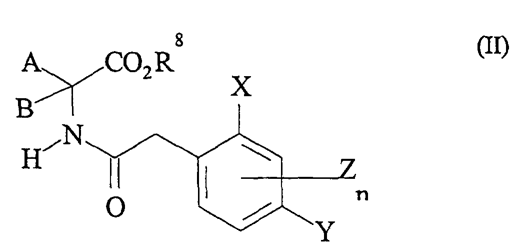 in welcher
- A, B, X, Y, Z, n und R⁸: die oben angegebene Bedeutung haben,
sind neu und Gegenstand einer nicht zum veröffentlichten Stand der Technik gehörenden Patentanmeldung der Anmelderin (vgl. die Deutsche Patentanmeldung P).

Man erhält z.B. Acyl-aminosäureester der Formel (II), wenn man
Aminosäurederivate der Formel (XIV), 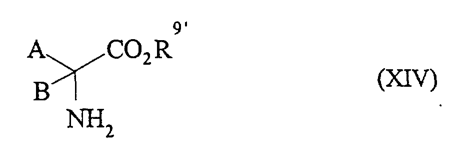 in welcher
- R9': für Wasserstoff (XIVa) und Alkyl (XIVb) steht
und
- A und B: die oben angegebene Bedeutung haben
mit Phenylessigsäurehalogeniden der Formel (XV) 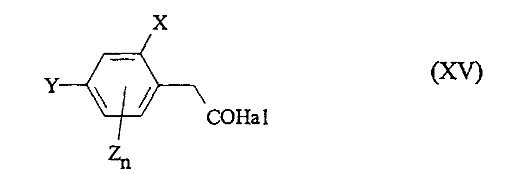 in welcher
- X, Y, Z und n: die oben angegebene Bedeutung haben und
- Hal: für Chlor oder Brom steht,
acyliert (Chem. Reviews 52, 237-416 (1953); Bhattacharya, Indien J. Chem. 6, 341-5, 1968)
oder wenn man Acylaminosäuren der Formel (IIa), 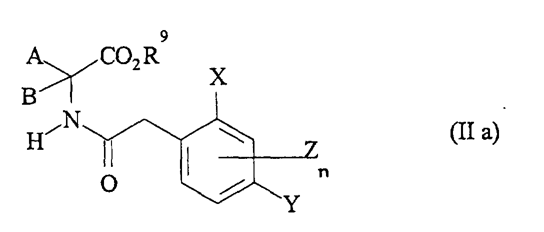 in welcher
- A, B, X, Y, Z und n: die oben angegebene Bedeutung haben,
und
- R⁹: für Wasserstoff steht,
verestert (Chem. Ind. (London) 1568 (1968)).

Die substituierten Cyclohexylaminocarbonsäuren der Formel (XIVa) sind im allgemeinen nach der Bucherer-Bergs-Reaktion oder nach der Strecker-Synthese erhältlich und fallen dabei jeweils in unterschiedlichen Isomerenformen an. So erhält man nach den Bedingungen der Bucherer-Bergs-Reaktion vorwiegend die Isomeren (im folgenden der Einfachheit halber β bezeichnet), in welchen die Reste R und die Carboxylgruppe äquatorial stehen während nach den Bedingungen der Strecker-Synthese vorwiegend die Isomeren (im folgenden der Einfachheit halber als a bezeichnet) anfallen, bei denen die Aminogruppe und die Reste R äquatorial stehen. 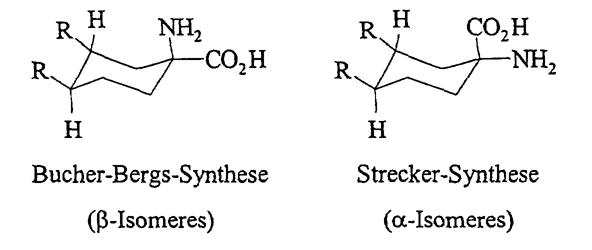 (L. Munday, J. Chem. Soc. 4372 (1961); J.T. Eward, C. Jitrangeri, Can. J. Chem. 53, 3339 (1975).

Weiterhin lassen sich die bei den obigen Verfahren (A) verwendeten Ausgangsstoffe der Formel (II) 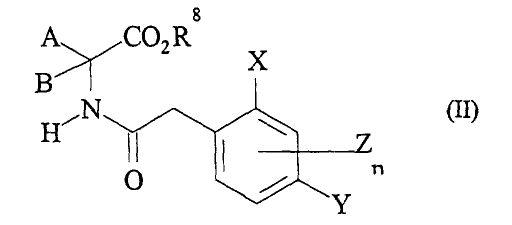 in welcher
- A, B, X, Y, Z, n und R8: die oben angegebene Bedeutung haben,
herstellen, wenn man Aminonitrile der Formel (XVI) 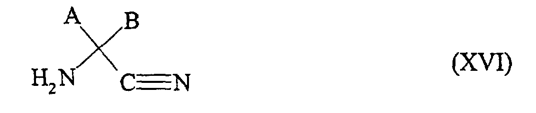 in welcher
- A und B: dier oben angegebene Bedeutung haben,
mit Phenylessigsäurehalogeniden der Formel (XV) 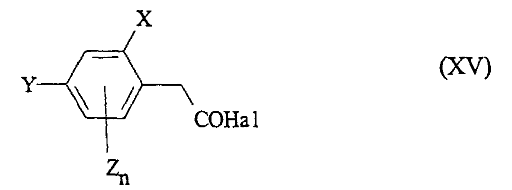 in welcher
- X, Y, Z und n: die oben angegebene Bedeutung haben und
- Hal: für Chlor oder Brom steht,
zu Verbindungen der Formel (XVII) 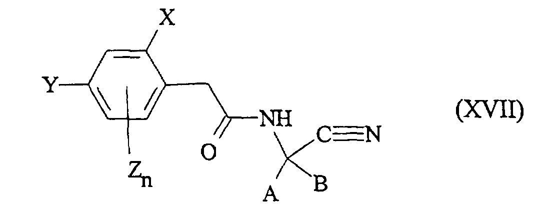 in welcher
- A, B, X, Y, Z und n: die oben angegebene Bedeutung haben,
umsetzt, die anschließend einer schwefelsauren Alkoholyse unterworfen werden.

Die Verbindungen der Formel (XVII) sind ebenfalls neu und Gegenstand einer nicht zum veröffentlichtem Stand der Technik gehörenden Patentanmeldung der Anmelderin (vgl. die Deutsche Patentanmeldung P ).

Beispielhaft aber nicht begrenzend seien außer den bei den Herstellungsbeispielen genannten Zwischenprodukten die folgenden Verbindungen der Formel (II) genannt:
N-(2,4-Dichlorphenylacetyl)-1-amino-2-methyl-cyclohexancarbonsäuremethylester,
N-(2,4-Dichlorphenylacetyl)-1-amino-3-methyl-cyclohexancarbonsäuremethylester,
N-(2,4-Dichlorphenylacetyl)-1-amino-4-methyl-cyclohexancarbonsäuremethylester,
N-(2,4-Dichlorphenylacetyl)-1-amino-3,4-dimethyl-cyclohexancarbonsäuremethylester,
N-(2,4-Dichlorphenylacetyl)-1-amino-4-ethylcyclohexancarbonsäuremethylester,
N-(2,4-Dichlorphenylacetyl)-1-amino-4-isopropyl-cyclohexancarbonsäuremethylester,
N-(2,4-Dichlorphenylacetyl)-1-amino-4-tert-butyl-cyclohexancarbonsäuremethylester,
N-(2,4-Dichlorphenylacetyl)-1-amino-4-phenyl-cyclohexancarbonsäuremethylester,
N-(2,6-Dichlorphenylacetyl)-1-amino-2-methyl-cyclohexancarbonsäuremethylester,
N-(2,6-Dichlorphenylacetyl)-1-amino-3-methyl-cyclohexancarbonsäuremethylester,
N-(2,6-Dichlorphenylacetyl)-1-amino-4-methyl-cyclohexancarbonsäuremethylester,
N-(2,6-Dichlorphenylacetyl)-1-amino-3,4-dimethylcyclohexancarbonsäuremethylester,
N-(2,6-Dichlorphenylacetyl)-1-amino-4-ethyl-cyclohexancarbonsäuremethylester,
N-(2,6-Dichlorphenylacetyl)-1-amino-4-isopropyl-cyclohexancarbonsäuremethylester,
N-(2,6-Dichlorphenylacetyl)-1-amino-4-tert-butyl-cyclohexancarbonsäuremethylester,
N-(2,6-Dichlorphenylacetyl)-1-amino-4-phenyl-cyclohexancarbonsäuremethylester,
N-(2-Chlor-6-fluor-phenyl-acetyl)-1-amino-2-methyl-cyclohexancarbonsäuremethylester,
N-(2-Chlor-6-fluorphenylacetyl)-1-amino-3-methyl-cyclohexancarbonsäuremethylester,
N-(2-Chlor-6-fluorphenylacetyl)-1-amino-4-methyl-cyclohexancarbonsäuremethylester,
N-(2-Chlor-6-fluorphenylacetyl)-1-amino-3,4-dimethyl-cyclohexancarbonsäure-methylester,
N-(2-Chlor-6-fluorphenylacetyl)-1-amino-4-ethyl-cyclohexancarbonsäuremethylester,
N-(2-Chlor-6-fluorphenylacetyl)-1-amino-4-isopropyl-cyclohexancarbonsäuremethylester,
N-(2-Chlor-6-fluorphenylacetyl)-1-amino-4-tert-butyl-cyclohexancarbonsäuremethylester,
N-(2-Chlor-6-fluorphenylacetyl)-1-amino-4-phenyl-cyclohexancarbonsäure-methylester,
N-(2,4,6-Trimethylphenylacetyl)-1-amino-2-methyl-cyclohexancarbonsäure-methylester,
N-(2,4,6-Trimethylphenylacetyl)-1-amino-3-methyl-cyclohexancarbonsäure-methylester,
N-(2,4,6-Trimethylphenylacetyl)-1-amino-4-methyl-cyclohexancarbonsäuremethylester,
N-(2,4,6-Trimethylphenylacetyl)-1-amino-3,4-dimethyl-cyclohexancarbonsäure-methylester,
N-(2,4,6-Trimethylphenylacetyl)-1-amino-4-ethyl-cyclohexancarbonsäure-methylester,
N-(2,4,6-Trimethylphenylacetyl)-1-amino-4-isopropyl-cyclohexancarbonsäuremethylester,
N-(2,4,6-Trimethylphenylacetyl)-1-amino-4-tert-butyl-cyclohexancarbonsäuremethylester,
N-(2,4,6-Trimethylphenylacetyl)-1-amino-4-phenyl-cyclohexancarbonsäure-methylester,
N-(2,4-Dimethylphenylacetyl)-1-amino-2-methyl-cyclohexancarbonsäure-methylester,
N-(2,4-Dimethylphenylacetyl)-1-amino-3-methyl-cyclohexancarbonsäure-methylester,
N-(2,4-Dimethylphenylacetyl)-1-amino-4-methyl-cyclohexancarbonsäure-methylester,
N-(2,4-Dimethylphenylacetyl)-1-amino-3,4-dimethyl-cyclohexancarbonsäuremethylester,
N-(2,4-Dimethylphenylacetyl)-1-amino-4-isopropyl-cyclohexancarbonsäure-methylester,
N-(2,4-Dimethylphenylacetyl)-1-amino-4-tert-butyl-cyclohexancarbonsäure-methylester,
N-(2,4-Dimethylphenylacetyl)-1-amino-4-phenyl-cyclohexancarbonsäure-methylester.

Beispielhaft aber nicht begrenzend seien außer den bei den Herstellungsbeispielen genannten Zwischenprodukten die folgenden Verbindungen der Formel (IIa) genannt:
N-(2,4-Dichlorphenylacetyl)-1-amino-2-methyl-cyclohexancarbonsäure,
N-(2,4-Dichlorphenylacetyl)-1-amino-3-methyl-cyclohexancarbonsäure,
N-(2,4-Dichlorphenylacetyl)-1-amino-4-methyl-cyclohexancarbonsäure,
N-(2,4-Dichlorphenylacetyl)-1-amino-3,4-dimethyl-cyclohexancarbonsäure,
N-(2,4-Dichlorphenylacetyl)-1-amino-4-ethyl-cyclohexancarbonsäure,
N-(2,4-Dichlorphenylacetyl)-1-amino-4-isopropyl-cyclohexancarbonsäure,
N-(2,4-Dichlorphenylacetyl)-1-amino-4-tert-butyl-cyclohexancarbonsäure,
N-(2,4-Dichlorphenylacetyl)-1-amino-4-phenyl-cyclohexancarbonsäure,
N-(2,6-Dichlorphenylacetyl)-1-amino-2-methyl-cyclohexancarbonsäure,
N-(2,6-Dichlorphenylacetyl)-1-amino-3-methyl-cyclohexancarbonsäure,
N-(2,6-Dichlorphenylacetyl)-1-amino-4-methyl-cyclohexancarbonsäure,
N-(2,6-Dichlorphenylacetyl)-1-amino-3,4-dimethyl-cyclohexancarbonsäure,
N-(2,6-Dichlorphenylacetyl)-1-amino-4-ethyl-cyclohexancarbonsäure,
N-(2,6-Dichlorphenylacetyl)-1-amino-4-isopropyl-cyclohexancarbonsäure,
N-(2,6-Dichlorphenylacetyl)-1-amino-4-tert-butyl-cyclohexancarbonsäure,
N-(2,6-Dichlorphenylacetyl)-1-amino-4-phenyl-cyclohexancarbonsäure,
N-(2-Chlor-6-fluorphenylacetyl)-1-amino-2-methyl-cyclohexancarbonsäure,
N-(2-Chlor-6-fluorphenylacetyl)-1-amino-3-methyl-cyclohexancarbonsäure,
N-(2-Chlor-6-fluorphenylacetyl)-1-amino-4-methyl-cyclohexancarbonsäure,
N-(2-Chlor-6-fluorphenylacetyl)-1-amino-3,4-dimethyl-cyclohexancarbonsäure,
N-(2-Chlor-6-fluorphenylacetyl)-1-amino-4-ethyl-cyclohexancarbonsäure,
N-(2-Chlor-6-fluorphenylacetyl)-1-amino-4-isopropyl-cyclohexancarbonsäure,
N-(2-Chlor-6-fluorphenylacetyl)-1-amino-4-tert-butyl-cyclohexancarbonsäure,
N-(2-Chlor-6-fluorphenylacetyl)-1-amino-4-phenyl-cyclohexancarbonsäure,
N-(2,4,6-Trimethylphenylacetyl)-1-amino-2-methyl-cyclohexancarbonsäure,
N-(2,4,6-Trimethylphenylacetyl)-1-amino-3-methyl-cyclohexancarbonsäure,
N-(2,4,6-Trimethylphenylacetyl)-1-amino-4-methyl-cyclohexancarbonsäure,
N-(2,4,6-Trimethylphenylacetyl)-1-amino-3,4-dimethyl-cyclohexancarbonsäure,
N-(2,4,6-Trimethylphenylacetyl)-1-amino-4-ethyl-cyclohexancarbonsäure,
N-(2,4,6-Trimethylphenylacetyl)-1-amino-4-isopropyl-cyclohexancarbonsäure,
N-(2,4,6-Trimethylphenylacetyl)-1-amino-4-tert-butyl-cyclohexancarbonsäure,
N-(2,4,6-Trimethylphenylacetyl)-1-amino-4-phenyl-cyclohexancarbonsäure,
N-(2,4-Dimethylphenylacetyl)-1-amino-2-methyl-cyclohexancarbonsäure,
N-(2,4-Dimethylphenylacetyl)-1-amino-3-methyl-cyclohexancarbonsäure,
N-(2,4-Dimethylphenylacetyl)-1-amino-4-methyl-cyclohexancarbonsäure,
N-(2,4-Dimethylphenylacetyl)-1-amino-3,4-dimethyl-cyclohexancarbonsäure,
N-(2,4-Dimethylphenylacetyl)-1-amino-4-ethyl-cyclohexancarbonsäure,
N-(2,4-Dimethylphenylacetyl)-1-amino-4-isopropyl-cyclohexancarbonsäure,
N-(2,4-Dimethylphenylacetyl)-1-amino-4-tert-butyl-cyclohexancarbonsäure,
N-(2,4-Dimethylphenylacetyl)-1-amino-4-phenyl-cyclohexancarbonsäure.

Verbindungen der Formel (IIa) sind beispielsweise aus den Phenylessigsäurehalogeniden der Formel (XV) und Aminosäuren der Formel (XIVa) nach Schotten-Baumann (Organikum, 9. Auflage, 446 (1970) VEB Deutscher Verlag der Wissenschaften, Berlin) erhältlich.

Die zur Durchführung der erfindungsgemäßen Verfahren (B), (C) und (G), außerdem als Ausgangsstoffe benötigten Säurehalogenide der Formel (III), Carbonsäureanhydride der Formel (IV), Chlorameisensäureester oder Chlorameisensäurethioester der Formel (V) und Metallhydroxide oder Amine der Formel (X) und (XI) sind allgemein bekannte Verbindungen der organischen bzw. anorganischen Chemie.

Das Verfahren (A) ist dadurch gekennzeichnet, daß Verbindungen der Formel (II) in welcher A, B, X, Y, Z, n und R⁸ die oben angegebene Bedeutung haben, in Gegenwart von Basen einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methylpyrrolidon, sowie Alkohole wie Methanol, Ethanol, Propanol, Isopropanol, Butanol, iso-Butanol und tert.-Butanol.

Als Basen (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 oder TDA 1*)eingesetzt werden können. Weiterhin können
Adogen 464 = Methyltrialkyl (C₈-C₁₀)ammoniumchlorid
TDA 1 = Tris-(methoxyethoxyethyl)-amin

Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natriumethylat und Kalium-tert.-butylat einsetzbar.

Die Reaktionstemperaturen können bei der Durchführung des erfmdungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formeln (II) und die deprotonierenden Basen im allgemeinen in etwa doppeltäquimolaren Mengen ein. Es ist jedoch auch möglich, die eine oder andere Komponente in einem größeren Überschuß (bis zu 3 Mol) zu verwenden.

Das Verfahren (Ba) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Ba) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Verwendet man die entsprechenden Carbonsäurehalogenide so kommen als Säurebindemittel bei der Umsetzung nach dem erfindungsgemäßen Verfahren (Ba) alle üblichen Säureakzeptoren in Betracht. Vorzugsweise verwendbar sind tertiäre Amine, wie Triethylamin, Pyridin, Diazabiyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können auch bei dem erfindungsgemäßen Verfahren (Ba) auch bei der Verwendung von Carbonsäurehalogeniden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Ba) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäurehydriden der Formel (IV) umsetzt.

Verwendet man bei dem erfindungsgemäßen Verfahren (Bβ) als Reaktionskomponente der Formel (IV) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäurehydrid gleichzeitig als Verdünnungsmittel fungieren.

Die Reaktionstemperaturen können bei dem erfindungsgemäßen Verfahren (Bβ) auch bei der Verwendung von Carbonsäureanhydriden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestem oder Chlorameisensäurethiolestern der Formel (V) umsetzt.

Verwendet man die entsprechenden Chlorameisensäureester bzw. Chlorameisensäurethiolester so kommen als Säurebindemittel bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) bei Verwendung der Chlorameisensäureester bzw. Chlorameisensäurethiolester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Bei Verwendung der Chlorameisensäureester bzw. Chlorameisensäurethiolester als Carbonsäure-Derivate der Formel (V) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende Chlorameisensäureester bzw. Chlorameisensäurethiolester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Das Verfahren (G) ist dadurch gekennzeichnet, daß man Verbindungen der Formel (Ia) mit Metallhydroxiden (X) oder Aminen (XI) umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (G) wird im allgemeinen unter Normaldruck durchgeführt. Die Reaktionstemperaturen liegen im allgemeinen zwischen -20°C und 100°C, vorzugsweise zwischen 0°C und 50°C.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.
Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp.

Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) gegen die Raupen der Kohlschabe Plutella maculipennis.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachauflaufverfahren. Sie können beispielsweise in Baumwolle oder Zuckerrüben mit sehr gutem Erfolg zur Bekämpfung von Schadgräsern eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Weiterhin kommen 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N- [(4-methoxy-6-methyl-1,3,5-triazin-2-yl)-amino]-carbonyl -benzolsulfonamid (CHLORSULFURON); 2-[4-(2,4-Dichlorphenoxy)-phenoxy]-propionsäure, deren Methyl- oder deren Ethylester (DICLOFOPMETHYL); 4-Amino-6-t-butyl-3-ethylthio-1,2,4-triazin-5(4H)-on (ETHIOZIN); 2- 4-[(6-Chlor-2-benzoxazolyl)-oxy]-phenoxy -propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-lH-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-hamstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-[[((4-Methoxy-6-methyl-1,3,5-triazin-2-yl)-amino)-carbonyl]-amino]-sulfonyl -benzoesäure oder deren (METSULFURON); N-(1-Ethylpropyl)-3,4-dimethyl-2,6-dinitroanilin (PENDIMETHALIN); 0-(6-Chlor-3-phenylpyridazin-4-yl)-S-octylthiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure- (THIAMETURON) in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

### Herstellungsbeispiele:

### Beispiel (Ia-1)

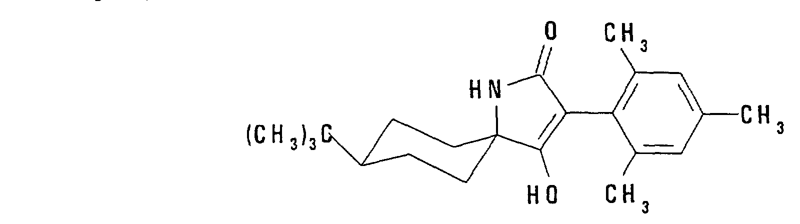

4,14g (0,138 Mol) Natriumhydrid werden in 70 ml absolutem Toluol suspendiert und am Rückfluß erhitzt. Dazu werden 25,6g (0,069 Mol) N-(2,4,6-Trimethylphenylacetyl)-1-amino-4-t.-butyl-cyclohexancarbonsäure-methylester in 140 ml absolutem Toluol zugetropft und anschließend so lange weiter am Rückfluß erhitzt, bis die Reaktion beendet ist. (Dünnschichtchromatographische Kontrolle). Nach dem Abkühlen auf Raumtemperatur wird unter Eiskühlung Ethanol zugetropft, bis kein Wasserstoff mehr entweicht. Danach wird das Lösungsmittel abgedampft, der Rückstand in Ethanol aufgenommen und bei 0-20°C in ca. 10%ige Salzsäure eingerührt. Der ausgefallene Niederschlag wird abgesaugt und getrocknet. Nach dem Umkristallisieren aus Chloroform/Hexan erhält man 14,90g (63% der Theorie) 3-(2,4,6-Trimetylphenyl)-5,5-(4-t.-butyl)-pentamethylen-pyrrolidin-2,4-dion vom Schmelzpunkt Fp.:>220°C. (β-Isomer)

### Beipiel (Ia-2)

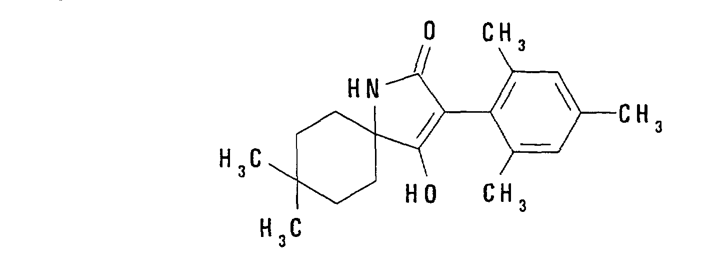

37,2 g (0,331 Mol) Kalium-tert.-butylat werden in 100 ml absolutem Tetrahydrofuran bei Rückflußtemperatur erhitzt. Dazu werden 52 g (0,151 Mol) N-(2,4,6-Trimethyl-phenylacetyl)-1-amino-4,4-dimethyl-cyclohexancarbonsäuremethylester in 510 ml absolutem Toluol zugetropft und 90 Minuten am Rückfluß erhitzt. Nach Beendigung der Reaktion wird der Ansatz auf Raumtemperatur gebracht und 500 ml Wasser dazugegeben. Die wäßrige Phase wird abgetrennt und die Toluolphase mit 220 ml Wasser extrahiert. Die vereinigten wäßrigen Phasen werden mit Toluol gewaschen und anschließend bei Raumtemperatur mit 50 ml konzentrierte Salzsäure versetzt. Der ausgefallene Niederschlag wird abgesaugt, gewaschen und getrocknet. Das Rohprodukt wird zur weiteren Reinigung in 300 ml Methyl-tert.-butylether aufgeschlämt und abgesaugt.

Man erhält 44,9 g (95 % der Theorie) 3-(2,4,6-Trimetylphenyl)-5,5-(4,4-dimethyl)-pentamethylen-pyrrolidin-2,4-dion vom Schmelzpunkt Fp.: >220°C.

Analog zu Beispiel (Ia-1) und (Ia-2) und gemäß den allgemeinen Angaben in der Beschreibung zu den erfindungsgemäßen Verfahren, werden die nachfolgenden in Tabelle 4 aufgeführten Endprodukte der Formel (Ia) als a-Isomer oder β-Isomer erhalten:

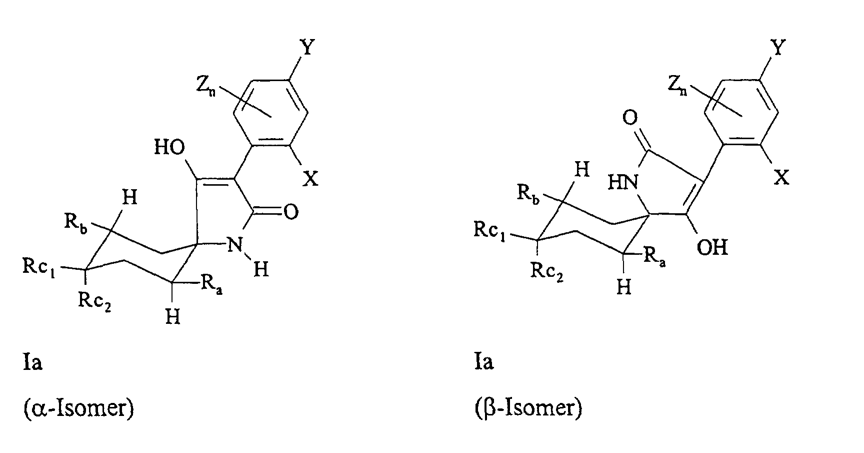

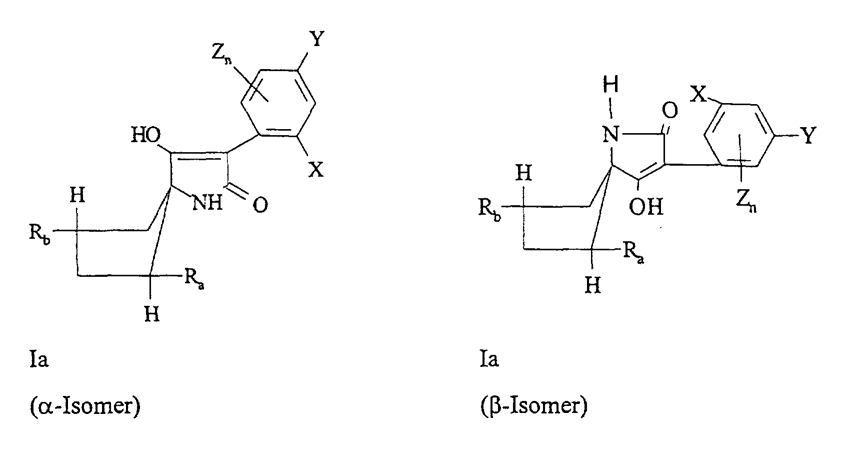

**Tabelle 4:**

| Bsp. Nr. | X | Y | Zₙ | Rₐ | R_{b} | R_{c1} | R_{c2} | phys. Konst. Fp.: °C | Isomer |
|---|---|---|---|---|---|---|---|---|---|
| (Ia-3) | Cl | Cl | H | CH₃ | H | H | H | 146 | α |
| (Ia-4) | CH₃ | CH₃ | 6-CH₃ | CH₃ | H | H | H | >220 | α |
| (Ia-5) | CH₃ | CH₃ | 6-CH₃ | H | H | CH₃ | H | >220 | α |
| (Ia-6) | CH₃ | CH₃ | 6-CH₃ | H | CH₃ | H | H | 217 | α |
| (Ia-7) | Cl | Cl | H | H | CH₃ | CH₃ | H | 130-140 | α |
| (Ia-8) | CH₃ | CH₃ | 6-CH₃ | H | CH₃ | CH₃ | H | >220 | α |
| (Ia-9) | CH₃ | CH₃ | H | H | H | CH₃ | H | 190-198 | α |
| (Ia-10) | CH₃ | CH₃ | 6-CH₃ | H | CH₃ | H | H | 120 | β |
| (Ia-11) | CH₃ | CH₃ | 6-CH₃ | H | H | CH₃ | H | >220 | β |
| (Ia-12) | Cl | Cl | H | H | CH₃ | H | H | >220 | α |
| (Ia-13) | Cl | Cl | H | H | CH₃ | H | H | 207-209 | β |
| (Ia-14) | CH₃ | CH₃ | H | H | CH₃ | H | H | 188-499 | β |
| (Ia-15) | Cl | Cl | H | H | H | CH₃ | H | 205-207 | α |
| (Ia-16) | CH₃ | CH₃ | 6-CH₃ | H | H | C₂H₅ | H | 149-200 | α |
| (Ia-17) | CH₃ | CH₃ | 6-CH₃ | H | H | C₂H₅ | H | 196-202 | β |
| (Ia-18) | Cl | Cl | H | H | H | C₂H₅ | H | 212-213 | α |
| (Ia-19) | Cl | Cl | H | H | H | CH₃ | H | >220 | β |
| (Ia-20) | CH₃ | CH₃ | H | H | H | CH₃ | H | >220 | β |
| (Ia-21) | Cl | Cl | H | H | H | C₂H₅ | H | 235 | β |
| (Ia-22) | CH₃ | CH₃ | 6-CH₃ | -CH₂- | | H | H | >220 | α |
| (Ia-23) | Cl | Cl | H | -CH₂- | | H | H | 215-217 | α |
| (Ia-24) | CH₃ | CH₃ | 6-CH₃ | CH₃ | H | - | - | 183-185 | α |
| (Ia-25) | CH₃ | CH₃ | 6-CH₃ | H | CH₃ | - | - | >220 | α |
| (Ia-26) | CH₃ | CH₃ | 6-CH₃ | H | H | C₆H₁₁ | H | >260 | α |
| (Ia-27) | CH₃ | CH₃ | 6-CH₃ | H | H | C₆H₁₁ | H | 252-253 | β |
| (Ia-28) | CH₃ | CH₃ | 6-CH₃ | H | H | C₆H₅ | H | >230 | *α* |
| (Ia-29) | CH₃ | CH₃ | 6-CH₃ | H | H | -(CH₂)₅- | | >220 | - |
| (Ia-30) | CH₃ | CH₃ | 6-CH₃ | H | H | i-C₃H₇ | H | 239-240 | *α* |
| (Ia-31) | CH₃ | CH₃ | 6-CH₃ | H | H | O-CH₃ | H | >220 | α |
| (Ia-32) | CH₃ | CH₃ | 6-CH₃ | H | H | O-CH₃ | H | >220 | β |
| (Ia-33) | CH₃ | CH₃ | H | H | H | C₂H₅ | H | 186 | β |
| (Ia-34) | Cl | Cl | H | H | H | C₃H₇ | H | 182 | β |
| (Ia-35) | CH₃ | CH₃ | H | H | H | C₃H₇ | H | 197 | β |
| (Ia-36) | CH₃ | CH₃ | 6-CH₃ | H | H | C₃H₇ | H | 206 | β |
| (Ia-37) | CH₃ | CH₃ | H | H | H | i-C₃H₇ | H | 196-205 | β |
| (Ia-38) | CH₃ | CH₃ | 6-CH₃ | H | H | i-C₃H₇ | H | 214 | β |

### Beispiel (Ib-1)

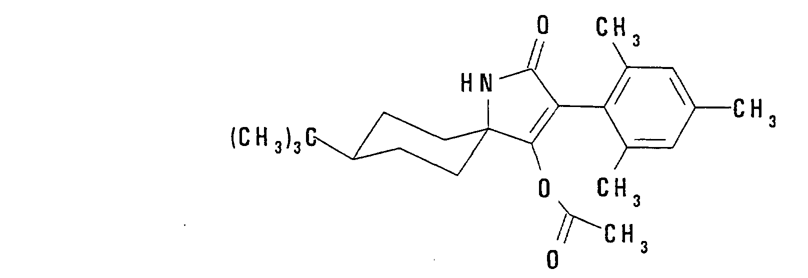

5,12g (0,015 Mol) 3-(2,4,6-Trimethylphenyl)-5,5-(4-t.-butyl)-pentamethylen-pyrrolidin-2,4-dion werden in 60 ml absolutem Dichlormethan gelöst und mit 2,1 ml Triethylamin versetzt. Bei 0 bis 10°C werden 1,13 ml Acetylchlorid in 5 ml absolutem Dichlormethan zugegeben. Das Ende der Reaktion wird dünnschichtchromatographisch ermittel. Anschließend wird zweimal mit jeweils 100 ml 0,5 N Natronlauge gewaschen und die organische Phase über Magnesiumsulfat getrocknet. Der nach dem Abdampfen des Lösungsmittels erhaltene Rückstand wird aus Essigester/n-Hexan umkristallisiert.

Man erhält 2 g (35% der Theorie) 3-(2,4,6-Trimethylphenyl)-5,5-(4-t.-butyl)-pentamethylen-4-acetyloxy-Δ³-pyrolin-2-on vom Schmelzpunkt Fp.:>220°C. (b-Isomer).

### Beispiel (Ib-2)

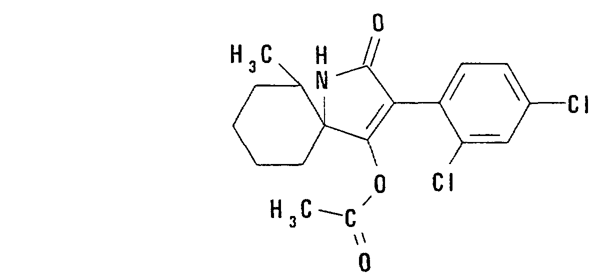

Analog zu Beispiel (Ib-1) erhält man 3-(2,4,6-Trimetylphenyl)-5,5-(2-methyl)-pentamethylen-4-acetyloxy-Δ³-pyrrolin-2-on vom Schmelzpunkt Fp.: 138°C. (Isomerengemisch)

Analog zu Beispiel (Ib-1) und (Ib-2) und gemäß den allgemeinen Angaben in der Beschreibung zu den erfindungsgemäßen Verfahren werden die nachfolgend in Tabelle 5 aufgeführten Endprodukte der Formel (Ib) als α-Isomer oder β-Isomer erhalten.

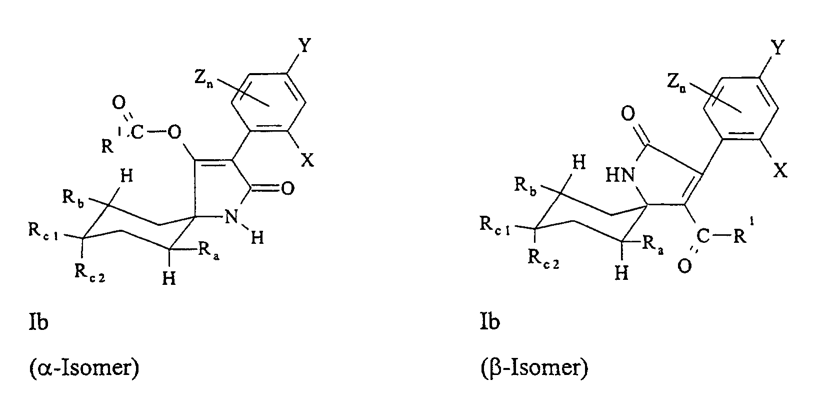

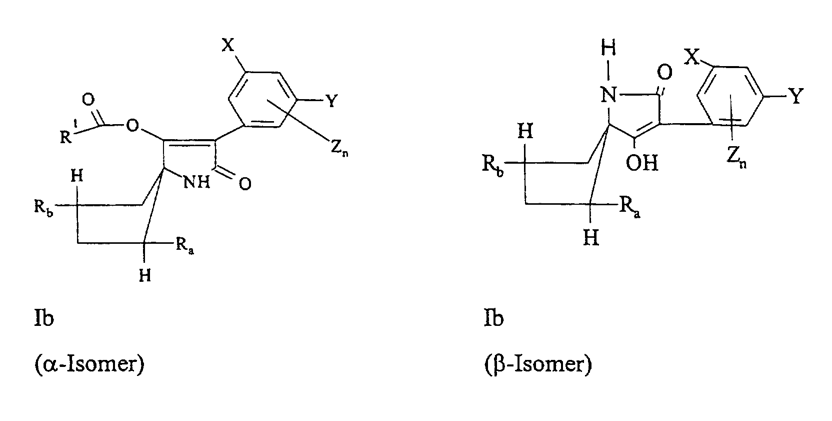

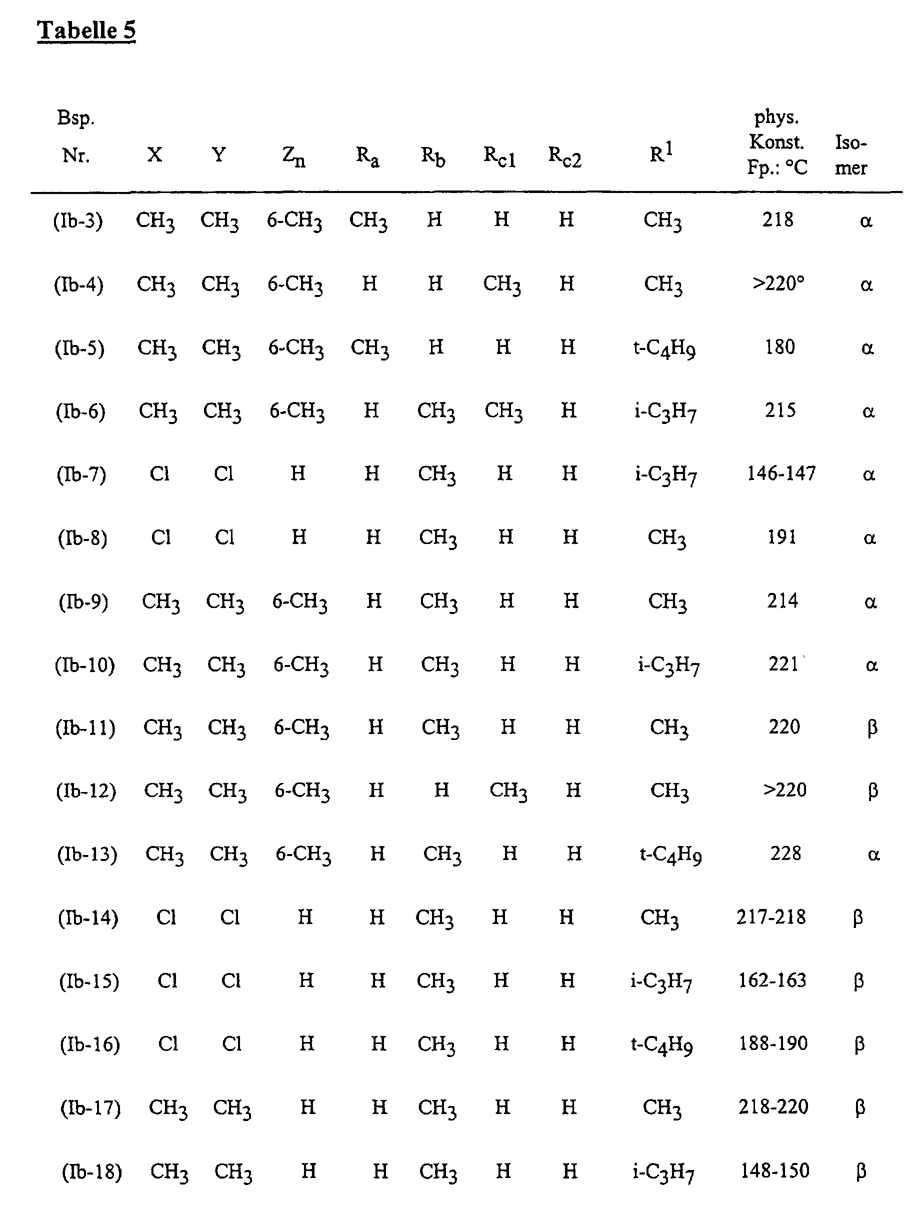

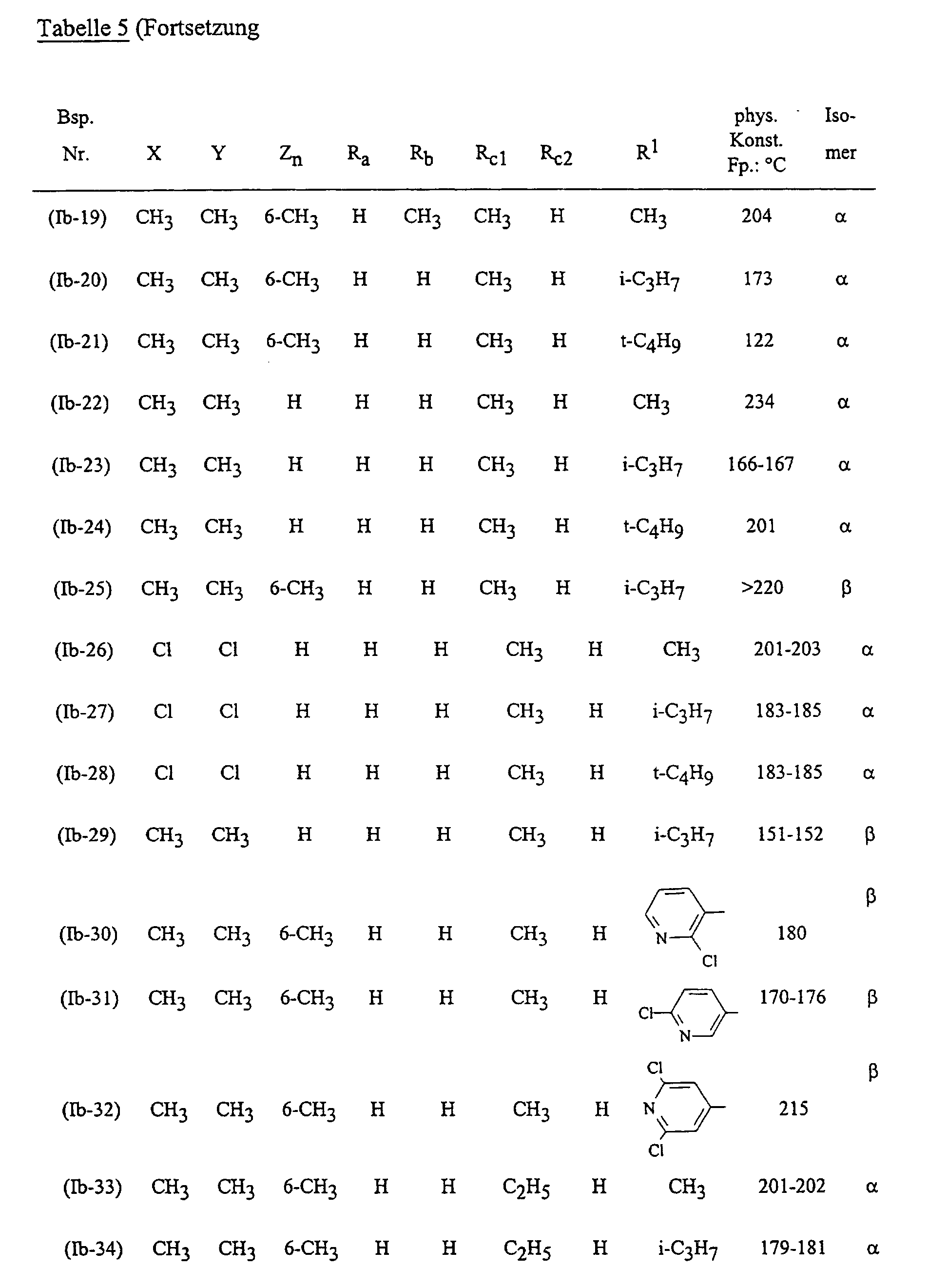

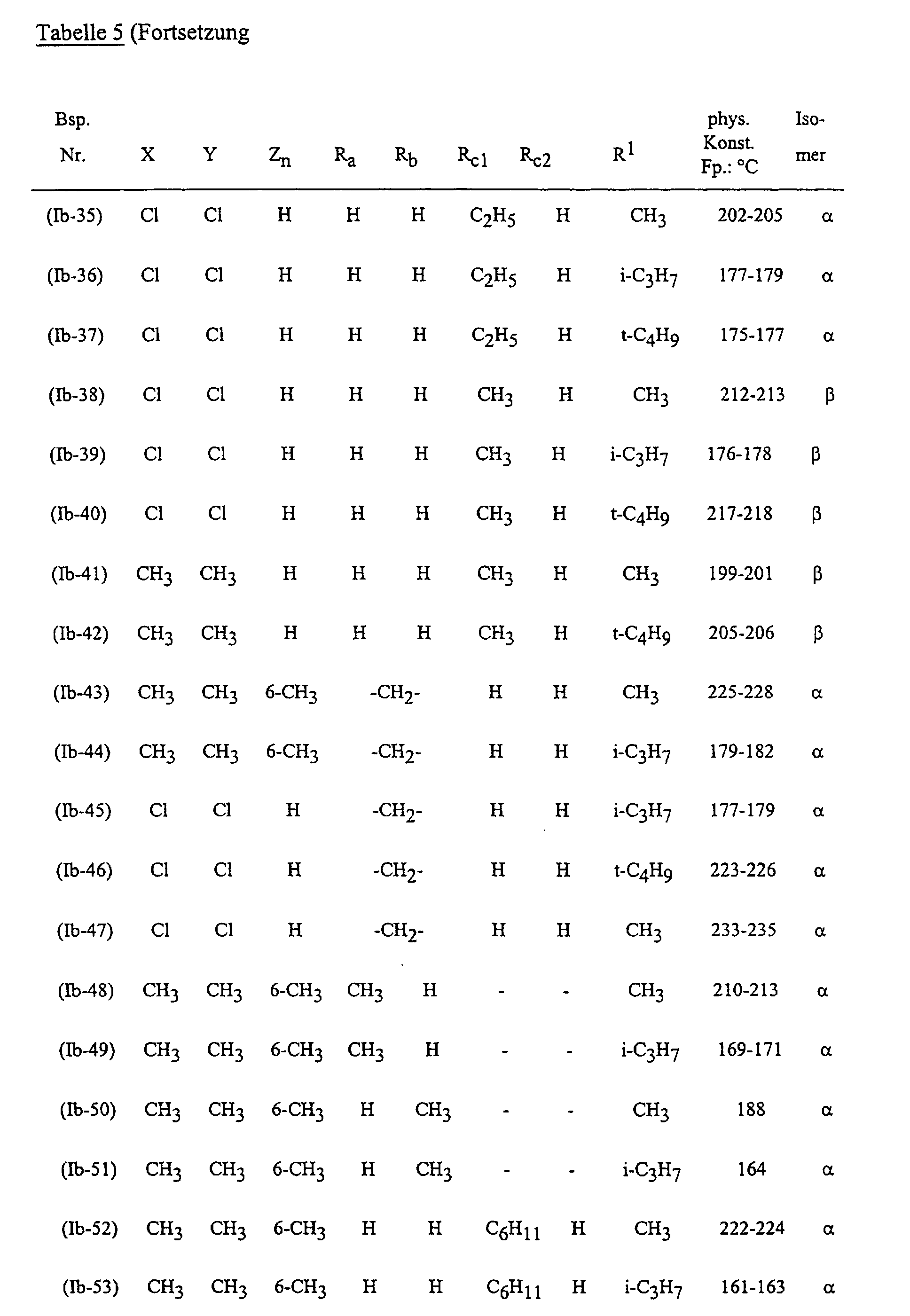

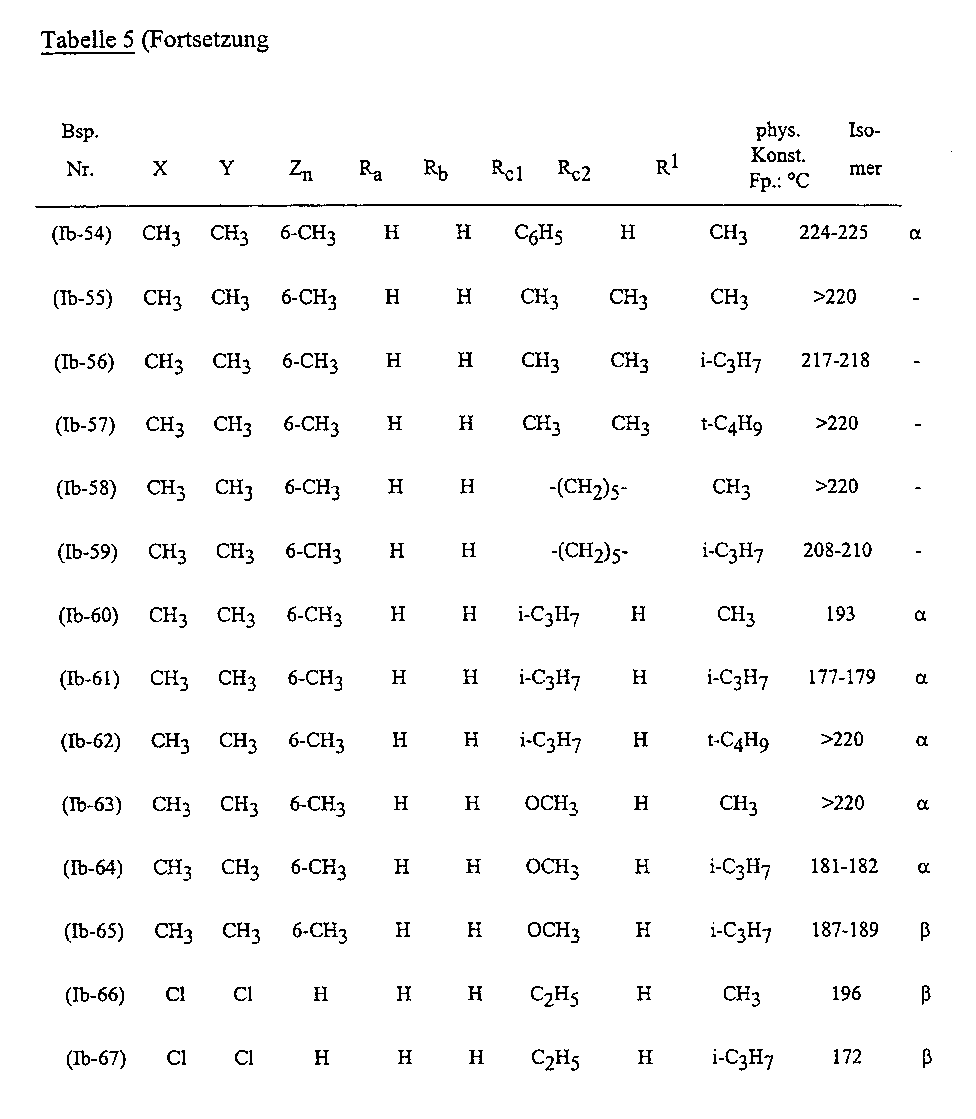

### Beispiel (Ic-1)

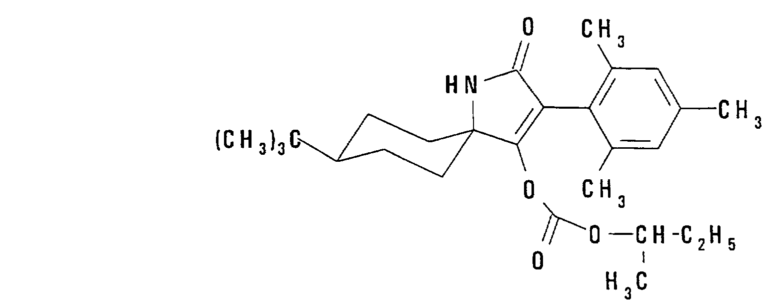

5,12 g (0,015 Mol) 3-(2,4,6-Trimethylphenyl)-5,5-(4-t.-butyl)-pentamethylenpyrrolidin-2,4-dion werden in 60 ml absolutem Dichlormethan gelöst und mit 2,1 ml Triethylamin versetzt. Bei 0-10°C werden 2,05 g Chlorameisensäure-sec-butylester in 5 ml absolutem Dichlormethan zugegeben und der Ansatz bei Raumtemperatur weitergerührt. Das Ende der Reaktion wird dünnschichtchromatographisch ermittel. Anschließend wird zweimal mit jeweils 100 ml 0,5 N Natronlauge gewaschen und die organische Phase über Magnesiumsulfat getrocknet. Der nach dem Abdampfen des Lösungsmittels erhaltene Rückstand wird aus Essigester/n-Hexan umkristallisiert.
Man erhält 4,4 g (66% der Theorie) Kohlensäure-O-(sec.-butyl)-ester-O-[3-(2,4,6-trimethylphenyl)-5,5-(4-t.-butyl)-pentamethylen-Δ³-pyrrolin-4-yl-2-on] vom Schmelzpunkt Fp.:>220°C. (β-Isomer).

### Beispiel (Ic-2)

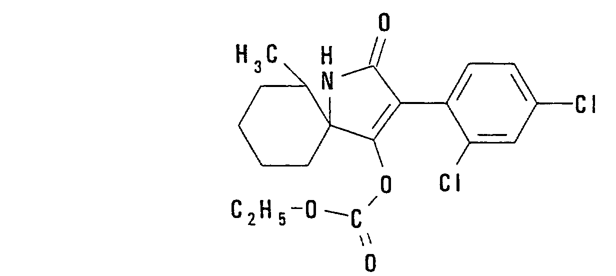

Analog zu Beispiel (Ic-1) erhält man Kohlensäure-O-(sec.-butyl)-ester-O-[3-(2,4,6-trimethylphenyl)-5,5-(4-t.-butyl)-pentamethylen-Δ³-pyrrolin-4-yl-2-on] vom Schmelzpunkt Fp.: 170°C (Isomerengemisch)

Analog zu Beispiel (Ic-1) und (Ic-2) und gemäß den allgemeinen Angaben in der Beschreibung zu den erfindungsgemäßen Verfahren, werden die nachfolgend in Tabelle 6 aufgeführten Endprodukte der Formel (Ic) als α-Isomer oder β-Isomer erhalten.

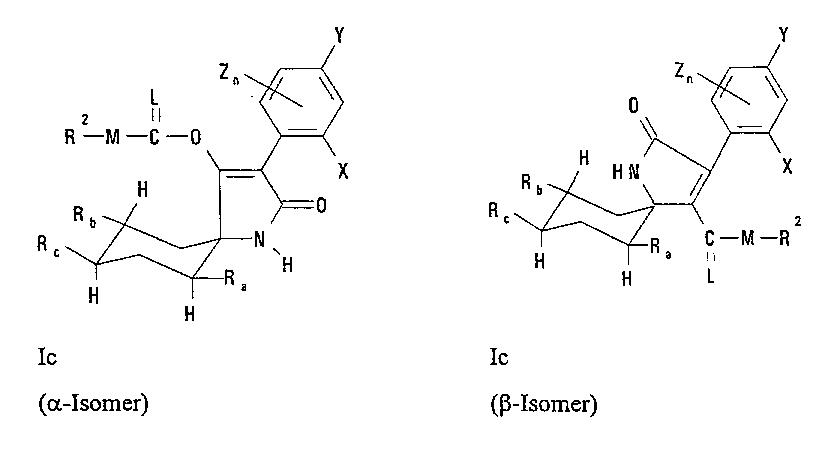

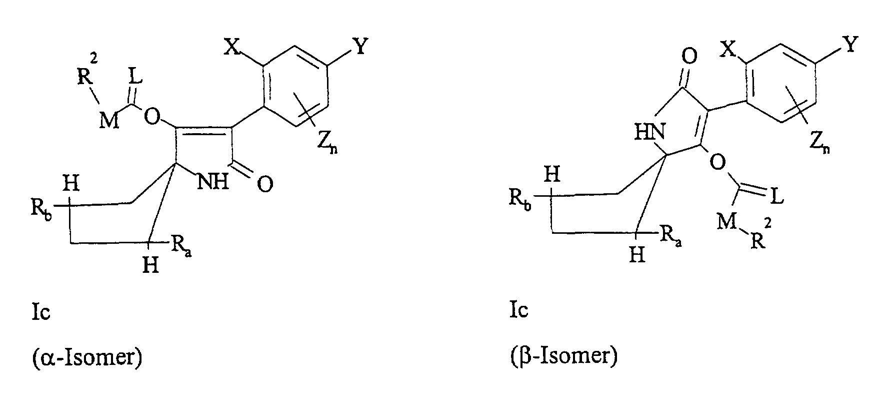

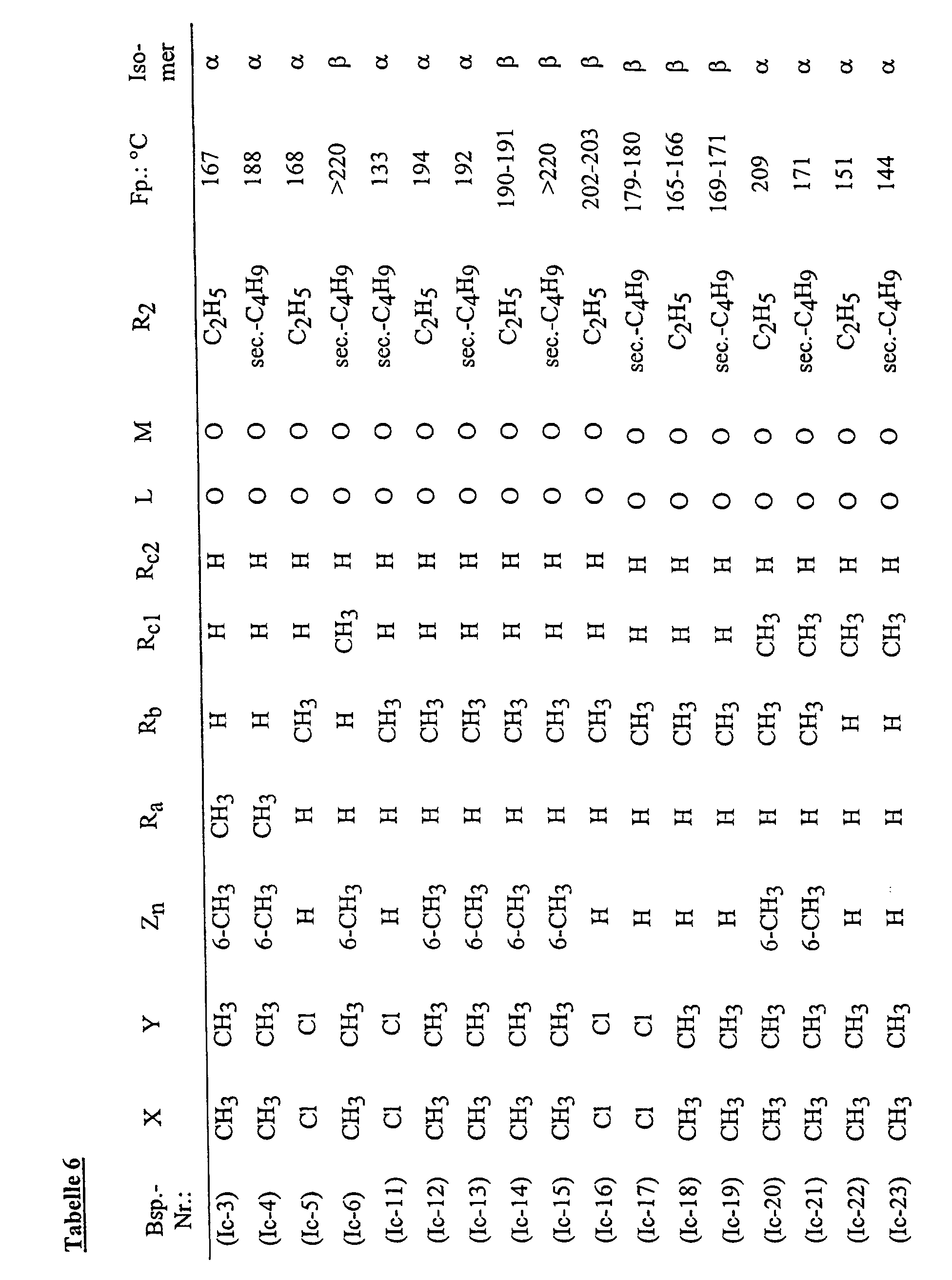

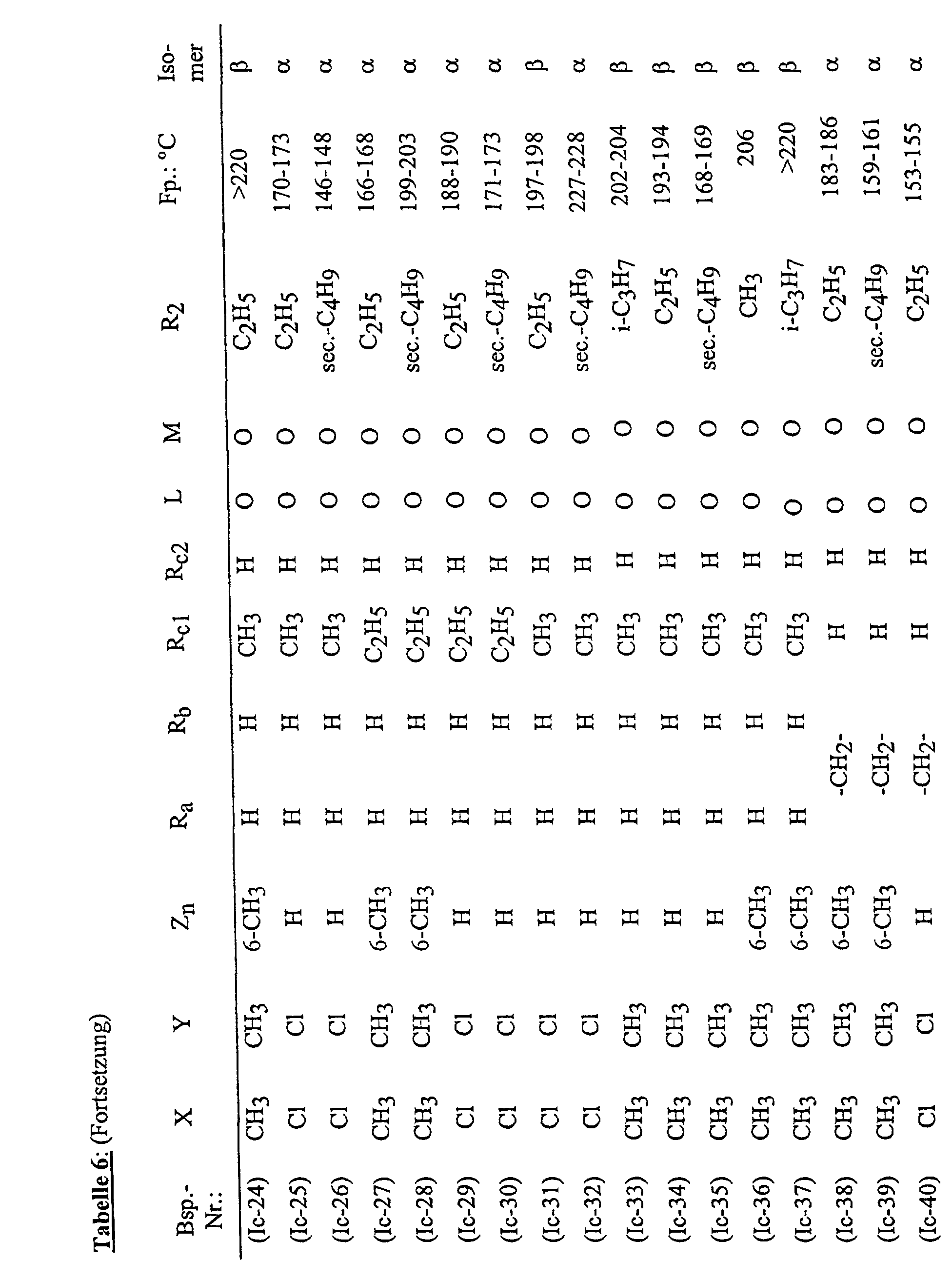

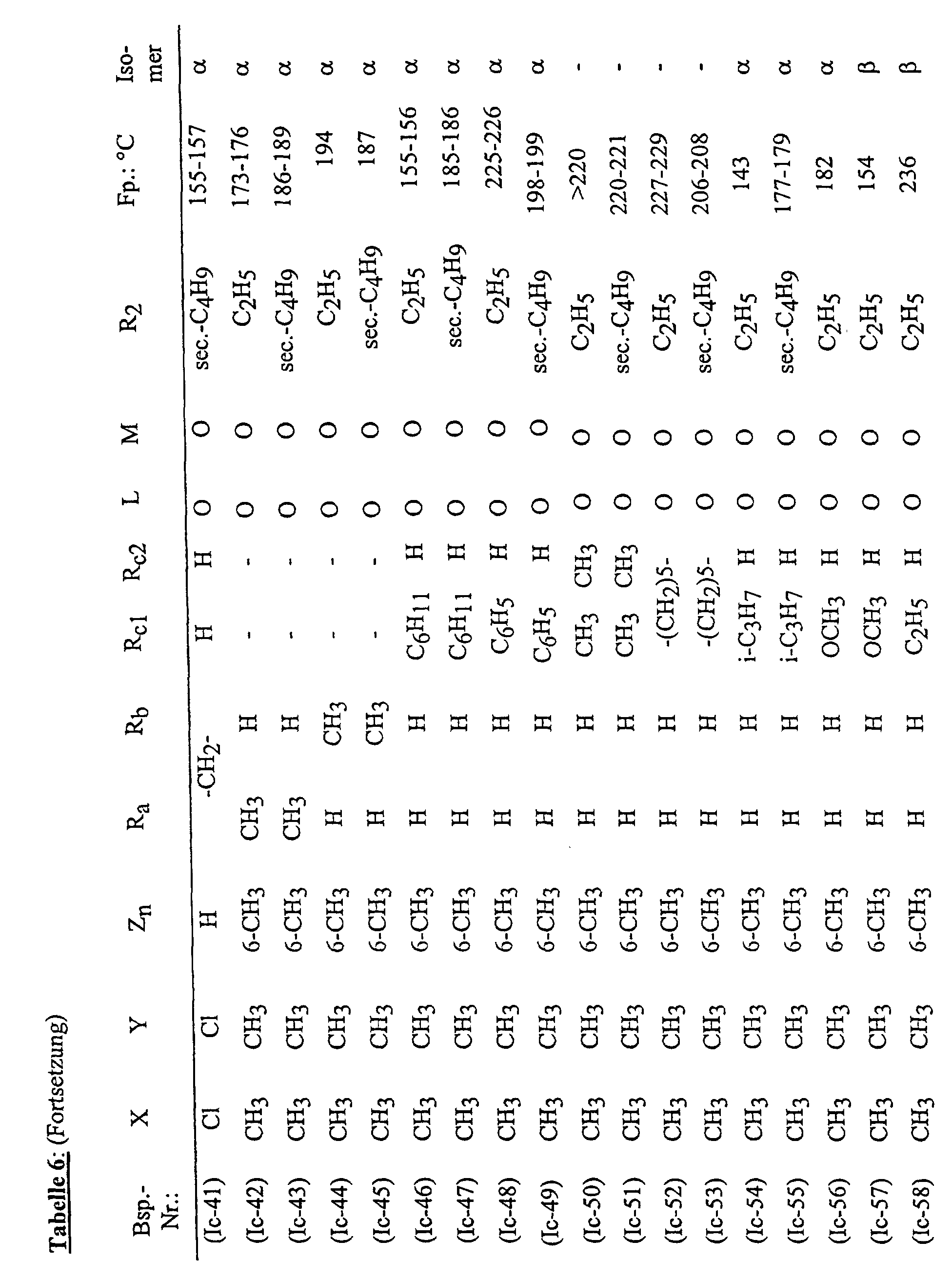

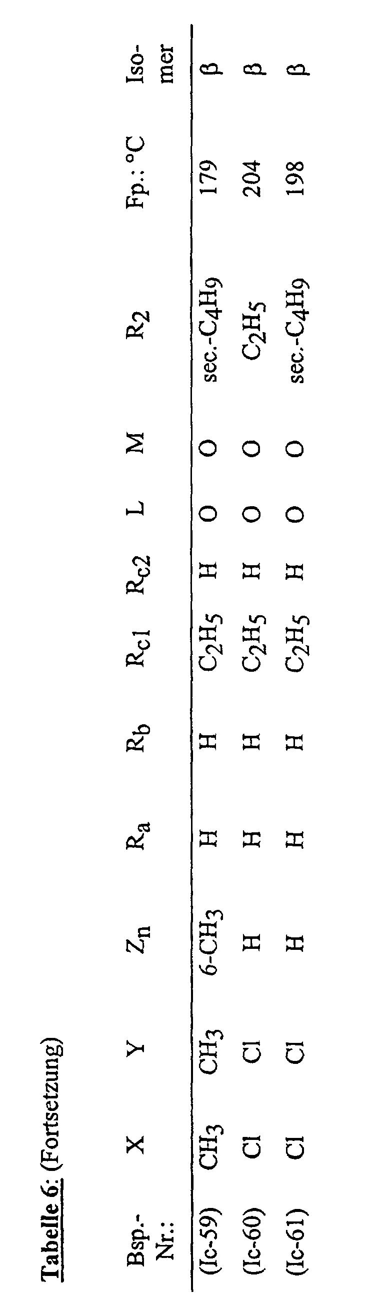

### Beispiel (If-1)

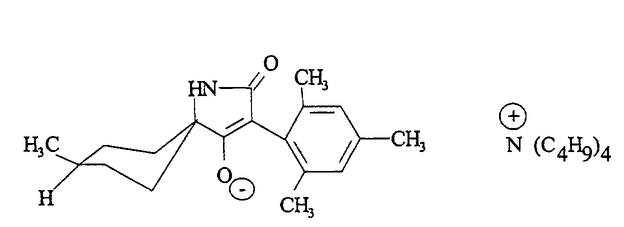

2,99 g (10mmol) 3-(2,4,6-Trimethylphenyl)-5,5-(4-methyl)-pentamethylen-pyrrolidin-2,4-dion werden in 40ml absolutem Methylenchlorid suspendiert. Nach Zugabe von 6,4 g Tetrabutylamminoumhydroxydlösung (40%-ig) wird 15 Min. nachgerührt, das Lösungsmittel abgedampft und der Rückstand mit Diisopropylether zur Kristallation gebracht. Nach Absaugen erhält man 5,1 g (94 % der Theorie) der oben gezeigten Verbindung vom Schmelzpunkt Fp.: 125°C (β-Isomer).

### Beispiel (If - 2)

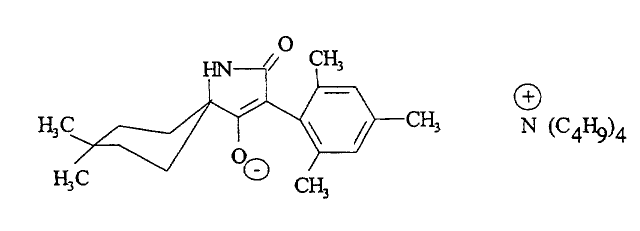

Analog zu Beispiel If-1 erhält man die Verbindung I f-2 vom Schmelzpunkt Fp.: 110°C.

### Herstellung der Ausgangsverbindungen

### Beispiel (II-1)

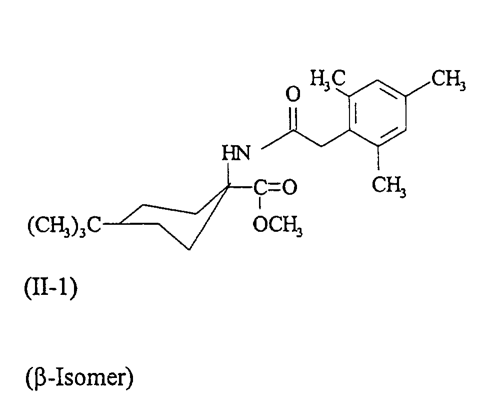

29,1 g (0,117 Mol) 1-Amino-1-cyclohexancarbonsäure-4-(t-butyl)-methylesterhydrochlorid werden in 300 ml Tetrahydrofuran suspendiert und mit 34,4 ml (0,246 Mol) Triethylamin versetzt. Bei 0 bis 10°C werden 23 g Mesitylenessigsäurechlorid in 20 ml absolutem Tetrahydrofuran zugetropft und der Ansatz bei Raumtemperatur weitergerührt. Das Reaktionsende wird dünnschichtchromatographisch bestimmt. Der Ansatz wird in 0,5 l Eiswasser mit 100 ml 1 N Salzsäure eingerührt und mit Dichlormethan extrahiert. Nach dem Waschen mit Natriumhydrogencarbonat-Lösung und trocknen der organischen Phase wird das Lösungsmittel abgezogen. Das Rohprodukt wird aus Toluol/n-Hexan umkristallisiert. Man erhält 25,6 g (59 % der Theorie) N-(2,4,6-Trimethylphenylacetyl)-1-amino-4-tert.-butyl-cyclohexancarbonsäuremethylester vom Schmelzpunkt Fp.: 153-154°C. (β-Isomer)

### Beispiel (II-2)

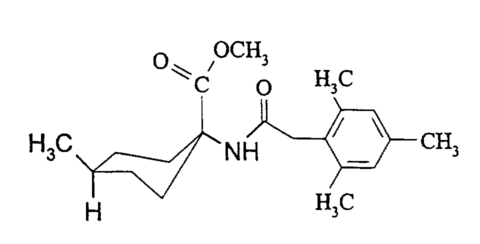

Man legt 148 g (1,52 Mol) konz. Schwefelsäure vor und tropft bei 30 bis 40°C eine Lösung von 90,9 g (0,30 Mol) N-(2,4,6-Trimethylphenylacetyl)-4-methyl-1-aminocyclohexannitril in 600 ml Methylenchlorid zu. Nach 2 h werden 212 ml abs. Methanol bei 40°C zugetropft und noch 6 h bei 40 bis 70°C nachgerührt.

Zur Aufarbeitung rührt man das Reaktionsgemisch in Eiswasser ein, extrahiet mit Methylenchlorid, wäscht die org. Phase mit NaHCO₃-Lösung, trocknet und rotiert ein. Die weitere Reinigung erfolgt durch Umkristalisation aus Toluol/n-Hexan.

Ausbeute: 73,9 g (73 % d. Th.) N-(2,4,6-Trimethylphenylacetyl)-4-methyl-1-aminocyclohexancarbonsäuremethylester vom Fp. 146°C.

Analog zu Beispiel (II-1) und gemäß den allgemeinen Angaben in der Beschreibung zu den erfindungsgemäßen Verfahren, werden die nachfolgend in Tabelle 8 aufgeführten Ausgangsverbindungen der Formel (II) als (α-Isomer) oder (β-Isomer) erhalten:

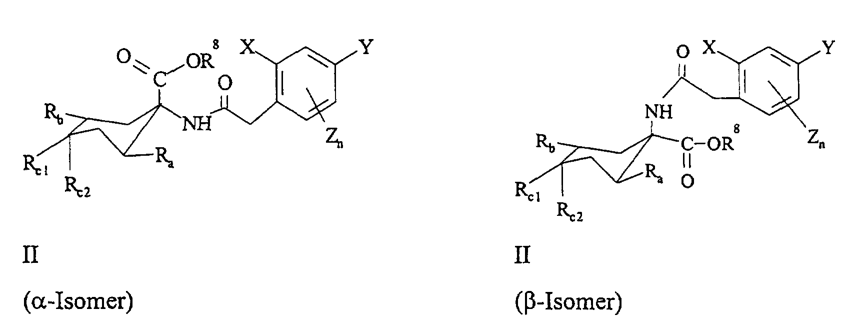

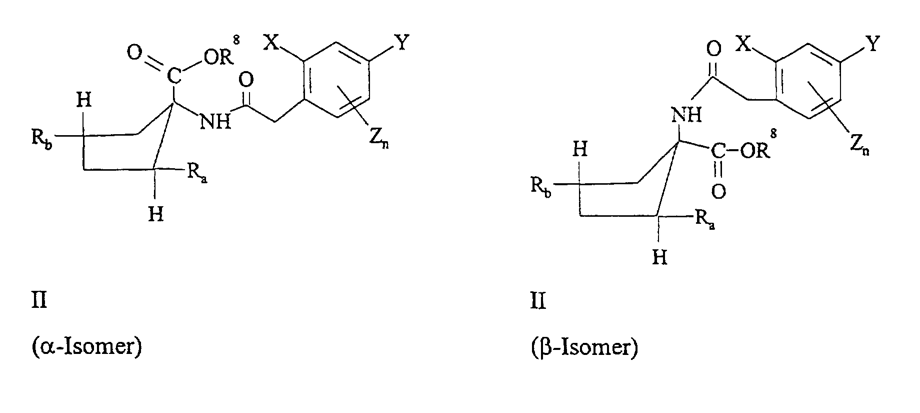

**Tabelle 8**

| Besp. Nr. | Rₐ | R_{b} | R_{c1} | R_{c2} | X | Y | Zₙ | R⁸ | phys. Konst. Fp.: °C | Isomer |
|---|---|---|---|---|---|---|---|---|---|---|
| (II-2) | H | H | CH₃ | H | CH₃ | CH₃ | 6-CH₃ | CH₃ | 146 | α |
| (II-3) | H | H | CH₃ | H | Cl | Cl | H | CH₃ | 118 | α |
| (II-4) | H | H | CH₃ | H | CH₃ | CH₃ | 6-CH₃ | CH₃ | 149-151 | β |
| (II-5) | CH₃ | H | H | H | CH₃ | CH₃ | 6-CH₃ | CH₃ | 103-104 | α |
| (II-6) | CH₃ | H | H | H | Cl | Cl | H | CH₃ | 156 | α |
| (II-7) | H | CH₃ | H | H | CH₃ | CH₃ | 6-CH₃ | CH₃ | 123-124 | β |
| (II-8) | H | CH₃ | H | H | Cl | Cl | H | CH₃ | 144 | α |
| (II-9) | H | H | CH₃ | CH₃ | CH₃ | CH₃ | 6-CH₃ | CH₃ | 142 | - |
| (II-10) | H | CH₃ | H | H | CH₃ | CH₃ | 6-CH₃ | CH₃ | 160 | α |
| (II-11) | H | H | CH₃ | H | CH₃ | CH₃ | H | CH₃ | 127-128 | α |
| (II-12) | H | CH₃ | CH₃ | H | CH₃ | CH₃ | 6-CH₃ | CH₃ | 147 | α |
| (II-13) | H | CH₃ | CH₃ | H | Cl | Cl | H | CH₃ | 138 | α |
| (II-14) | H | H | i-C₃H₇ | H | CH₃ | CH₃ | 6-CH₃ | CH₃ | 137-139 | α |
| (II-15) | H | H | C₂H₅ | H | Cl | Cl | H | CH₃ | 103-105 | α |
| (II-16) | H | H | C₂H₅ | H | CH₃ | CH₃ | 6-CH₃ | CH₃ | 144-145 | α |
| (II-17) | H | H | C₂H₅ | H | CH₃ | CH₃ | 6-CH₃ | CH₃ | 129-130 | β |
| (II-18) | H | H | OCH₃ | H | CH₃ | CH₃ | 6-CH₃ | CH₃ | 116-118 | α |
| (II-19) | H | H | C₆H₁₁ | H | CH₃ | CH₃ | 6-CH₃ | CH₃ | 208-210 | α |
| (II-20) | H | H | C₆H₅ | H | CH₃ | CH₃ | 6-CH₃ | CH₃ | 207 | α |
| (II-21) | H | H | -(CH₂)₅- | | CH₃ | CH₃ | 6-CH₃ | CH₃ | 153-154 | - |
| (II-22) | H | CH₃ | H | H | Cl | Cl | H | CH₃ | 147-148 | β |
| (II-23) | H | H | CH₃ | H | Cl | Cl | H | CH₃ | 151-152 | β |
| (II-24) | H | H | CH₃ | H | CH₃ | CH₃ | H | CH₃ | 123-124 | β |
| (II-25) | H | CH₃ | H | H | CH₃ | CH₃ | H | CH₃ | 131 | β |
| (II-26) | H | H | C₂H₅ | H | Cl | Cl | H | CH₃ | 106 | β |
| (II-27) | H | CH₃ | - | - | CH₃ | CH₃ | 6-CH₃ | CH₃ | 83-85 | α |
| (II-28) | CH₃ | H | - | - | CH₃ | CH₃ | 6-CH₃ | CH₃ | 107-108 | α |
| (II-29) | H | H | C₂H₅ | H | CH₃ | CH₃ | H | CH₃ | 105 | β |
| (II-30) | H | H | C₃H₇ | H | CH₃ | CH₃ | 6-CH₃ | CH₃ | 155 | β |
| (II-31) | H | H | C₃H₇ | H | CH₃ | CH₃ | H | CH₃ | 131 | β |
| (II-32) | H | H | C₃H₇ | H | | | H | CH₃ | 106 | β |
| (II-33) | H | H | i-C₃H₇ | H | CH₃ | CH₃ | 6-CH₃ | CH₃ | 145 | β |
| (II-34) | H | H | i-C₃H₇ | H | CH₃ | CH₃ | H | CH₃ | 118 | β |

### Beispiele für die Herstellung der Ausgangsverbindungen der Formel XVII:

### Beipiel XVII-1

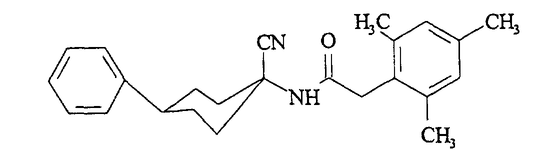

Zu 40g (0,2 Mol) 1-Amino-4-phenyl-cyclohexancarbonsäurenitril und 28ml (0,2 Mol) Triethylamin in 400 ml absolutem Tetrahydrofuran gibt man bei 0°C bis 10°C tropfenweise unter Rühren 39,3 g (0,2 Mol) Mesitylenessigsäurechlorid (vergl. z.B. Tetrahedron 31, 2691-2694 [1975]) in 40 ml absolutem Tetrahydrofuran und rührt nach beendeter Zugabe bei Raumtemperatur bis im Dünnschichtchromatogramm kein Ausgangsprodukt mehr nachweisbar ist. Zur Aufarbeitung gibt man die Reaktionsmischung unter Rühren in eine Mischung aus 1000 ml Eiswasser und 200 ml 1N-Salzsäure, saugt ausgefallenen Feststoff ab, löst den Rückstand in Dichlormethan, trennt die wässrige Phase ab, trocknet die organische Phase über Magnesiumsulfat und entfernt das Lösungsmittel im Vakuum.

Man erhält 65 g (91 % der Theorie) an N-(2,4,6-Trimethylphenyl-acetyl)-4-phenylcyclohexancarbonsäurenitril vom Schmelzpunkt 235-238°C.

Analog zu Beispiel XVII-1 und gemäß den allgemeinen Angaben in der Beschreibung zu den erfindungsgemäßen Verfahren, werden die nachfolgend in der Tabelle 9 aufgeführten Ausgangsverbindungen der Formel XVII erhalten:

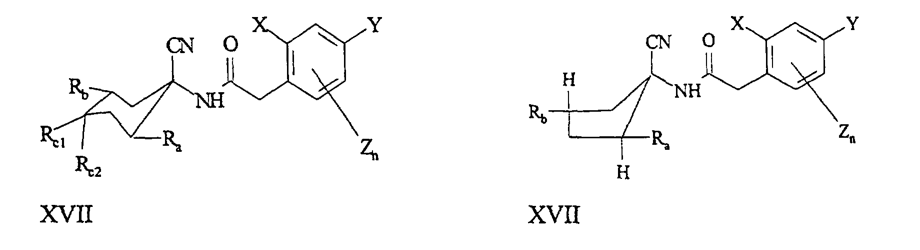

**Tabelle 9**

| Bsp.-Nr.: | Rₐ | R_{b} | R_{c1} | R_{c2} | X | Y | Zₙ | Fp.:°C |
|---|---|---|---|---|---|---|---|---|
| XVII-2 | CH₃ | H | H | H | CH₃ | CH₃ | 6-CH₃ | 183-184 |
| XVII-3 | CH₃ | H | H | H | Cl | Cl | H | 153 |
| XVII-4 | H | H | CH₃ | H | CH₃ | CH₃ | 6-CH₃ | 202 |
| XVII-5 | H | H | CH₃ | H | Cl | Cl | H | 203 |
| XVII-6 | H | CH₃ | H | H | Cl | Cl | H | 189 |
| XVII-7 | H | CH₃ | H | H | CH₃ | CH₃ | 6-CH₃ | 176 |
| XVII-8 | H | CH₃ | CH₃ | H | CH₃ | CH₃ | 6-CH₃ | 174 |
| XVII-9 | H | CH₃ | CH₃ | H | Cl | Cl | H | 153 |
| XVII-10 | H | H | CH₃ | H | CH₃ | CH₃ | H | 198 |
| XVII-11 | H | H | i-C₃H₇ | H | CH₃ | CH₃ | 6-CH₃ | 204-206 |
| XVII-12 | H | H | C₂H₅ | H | CH₃ | CH₃ | 6-CH₃ | 185 |
| XVII-13 | H | H | C₂H₅ | H | Cl | Cl | H | 194 |
| XVII-14 | H | H | CH₃ | CH₃ | CH₃ | CH₃ | 6-CH₃ | 190-192 |
| XVII-15 | H | H | OCH₃ | H | CH₃ | CH₃ | 6-CH₃ | 159-160 |
| XVII-16 | H | H | C₆H₁₁ | H | CH₃ | CH₃ | 6-CH₃ | 208 |
| XVII-17 | H | H | -(CH₂)₅- | | CH₃ | CH₃ | 6-CH₃ | 198-201 |
| XVII-18 | H | CH₃ | - | - | CH₃ | CH₃ | 6-CH₃ | 139 |
| XVII-19 | CH₃ | H | - | - | CH₃ | CH₃ | 6-CH₃ | 142 |

### Beispiel A

### Phaedon-Larven-Test

| | | | |
|---|---|---|---|
| Lösungsmittel | 7 | Gewichtsteile | Dimethylformamid |
| Emulgator | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet; daß keine Käfer-Larven abgetötet wurden.

Bei diesem Test zeigen z.B. die folgenden Verbindungen der Herstellungsbeispiele überlegende Wirksamkeit gegenüber dem Stand der Technik: (Ia-10), (Ia-11), (Ib-11),(Ib-12).

### Beispiel B

### Plutella-Test

| | | | |
|---|---|---|---|
| Lösungsmittel | 7 | Gewichtsteile | Dimethylformamid |
| Emulgator | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet; daß keine Raupen abgetötet wurden.

Bei diesem Test zeigen z.B. die folgenden Verbindungen der Herstellungsbeispiele überlegende Wirksamkeit gegenüber dem Stand der Technik: (Ia-10), (Ia-11), (Ib-12).

### Beispiel C

### Nephotettix-Test

| | | | |
|---|---|---|---|
| Lösungsmittel | 7 | Gewichtsteile | Dimethylformamid |
| Emulgator | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade (Nephotettix cincticeps) besetzt, solange die Blätter noch feucht sind.

Nach der gewünschten Zeit wird die Abtötung in % bestimmt. Dabei bedeutet 100 %, daß alle Zikaden abgetötet wurden; 0 % bedeutet; daß keine Zikaden abgetötet wurden.

Bei diesem Test zeigen z.B. die folgenden Verbindungen der Herstellungsbeispiele überlegende Wirksamkeit gegenüber dem Stand der Technik: (Ia-10), (Ia-11),(Ib-11), (Ib-12).

### Beispiel D

### Pre-emergence-Test

| | | | |
|---|---|---|---|
| Lösungsmittel | 5 | Gewichtsteile | Aceton |
| Emulgator | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonititert in % Schädigung im Vergleich zur Entwicklung der unbehandelten Kontrolle.

Es bedeuten:
- 0%: = keine Wirkung (wie unbehandelte Kontrolle)
- 100 %: = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß Herstellungsbeispiel: (Ia-10), (Ia-11), (Ia-12), (Ib-11), (Ic-6).

### Beispiel E

### Post-emergence-Test

| | | | |
|---|---|---|---|
| Lösungsmittel | 5 | Gewichtsteile | Aceton |
| Emulgator | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung spritzt man Testpflanzen welche eine Höhe von 5 - 15 cm haben, so, daß die jeweils gewünschten Wirkstoffmengen pro Flächeneinheit ausgebracht werden. Nach drei Wochen wird der Schädigungsgrad der Pflanzen bonititert in % Schädigung im Vergleich zur Entwicklung der unbehandelten Kontrolle.

Es bedeuten:
- 0%: = keine Wirkung (wie unbehandelte Kontrolle)
- 100 %: = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß Herstellungsbeispiel: (Ia-10), (Ia-11),(Ib-11), (Ib-12).

## Patentansprüche

1. Substituierte l-H-3-Arylpyrrolidin-2,4-dion Derivate der Formel (I) 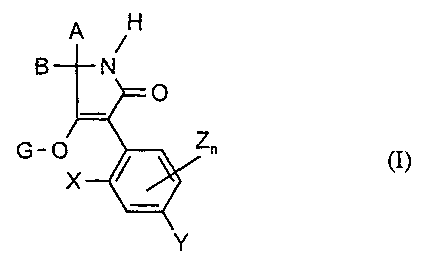 in welcher
A, B und das Kohlenstoffatom an das sie gebunden sind für einen C₃-C₆-Spirocyclus, der einfach oder mehrfach durch C₁-C₆-Alkyl, C₃-C₈-Cycloalkyl, C₁-C₃Haloalkyl, C₁-C₆-Alkoxy, C₁-C₄-Thioalkyl, Fluor, Chlor oder Phenyl substituiert sein kann stehen oder
A, B und das Kohlenstoffatom, an das sie gebunden sind für einen C₃-C₆-Spirocyclus stehen, der mit einer gegebenenfalls durch ein oder zwei Sauerstoff- oder Schwefelatome unterbrochenen Alkylendiyl- oder durch eine Alkylendioxyl- oder durch eine Alkylendithiooxyl-Gruppe substiuiert ist, die mit dem Kohlenstoffatom, an das sie gebunden ist, einen weiteren fünf- bis siebengliedrigen Spirocyclus bildet oder
A,B und das Kohlenstoffatom, an das sie gebunden sind für einen C₃-C₆-Spirocyclus stehen, bei dem zwei Substituenten gemeinsam mit den C-Atomen, an die sie gebunden sind für eine durch Alkyl(C₁-C₃), Alkoxy(C₁-C₃) oder Fluor, Chlor, Brom substituierten gesättigten oder ungesättigten Cyclus stehen, der durch Sauerstoff oder Schwefel unterbrochen sein kann.
X für C₁-C₄-Alkyl, Halogen oder C₁-C₄-Alkoxy steht,
Y für Wasserstoff, Methyl, Ethyl, Propyl, i-Propyl, Butyl, i-Butyl, Fluor, Chlor, Brom, Methoxy, Ethoxy oder Trifluormethyl steht,
Z für Methyl, Ethyl, Propyl, i-Propyl, Butyl, i-Butyl, tert.-Butyl, Fluor, Chlor, Brom, Methoxy oder Ethoxy steht,
n für eine Zahl von 0 - 2 steht,
G für Wasserstoff (a) oder für die Gruppen der Formel 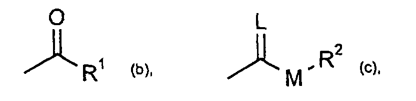 oder E (f)
steht, in welcher
E für ein Metallionäquivalent oder ein Ammoniumion steht und
L und M für Sauerstoff stehen,
R¹ für gegebenenfalls durch Halogen substituiertes C₁-C₁₆-Alkyl, C₂-C₁₆-Alkenyl, C₁-C₆-Alkoxy-C₂-C₆-alkyl, C₁-C₁₆-Alkylthio-C₂-C₆-alkyl, C₁-C₆-Polyalkoxy-C₂-C₆-alkyl oder Cycloalkyl mit 3 bis 7 Ringatomen, das durch 1 bis 2 Sauerstoff- und/oder Schwefelatome unterbrochen sein kann, steht,für gegebenenfalls durch Halogen-, Nitro-, C₁-C₄-Alkyl, C₁-C₄-Alkoxy, C₁-C₃-Halogenalkyl-, C₁-C₃-Halogenalkoxy-substituiertes Phenyl,
für gegebenenfalls durch Halogen-, C₁-C₄-Alkyl, C₁-C₄-Alkoxy, C₁-C₃-Halogenalkyl-, C₁-C₃-Halogenalkoxy-substituiertes Phenyl-C₁-C₄-alkyl steht, für gegebenenfalls durch Fluor, Chlor, Brom- und/oder C₁-C₄-Alkyl-substituiertes Hetaryl steht,
für gegebenenfalls durch Fluor, Chlor, Brom und C₁-C₄-Alkyl-substituiertes Phenoxy-C₁-C₅-alkyl steht,
für gegebenenfalls durch Fluor, Chlor, Brom, Amino und C₁-C₄-Alkyl-substituiertes Hetaryloxy-C₁-C₅-alkyl steht,
R² für gegebenenfalls durch Halogen substituiertes C₁-C₁₆-Alkyl, C₃-C₁₆-Alkenyl, C₁-C₆-Alkoxy-C₂-C₆-alkyl, C₁-C₆-Polyalkoxy-C₂-C₆-alkyl steht,
für gegebenenfalls durch Halogen, Nitro, C₁-C₄-Alkyl-, C₁-C₃-Alkoxy-, C₁-C₃-Halogenalkyl-substituiertes Phenyl oder Benzyl steht,

2. Verbindungen der allgemeinen Formel (I) gemäß Anspruch 1, in welcher
A, B und das Kohlenstoffatom an das sie gebunden sind für einen C₃-C₆-Spirocyclus, der mindestens einfach oder mehrfach durch Methyl, Ethyl, Propyl, Isopropyl, Butyl, iso-Butyl, sec.-Butyl, tert,-Butyl, Cyclohexyl, Trifluormethyl, Methoxy, Ethoxy, Propoxy, Isopropoxy, Butoxy, Isobutoxy, sek.-Butoxy, tert.-Butoxy, Methylthio, Fluor, Chlor oder Phenyl substituiert sein kann stehen oder
A, B und das Kohlenstoffatom, an das sie gebunden sind für einen C₃-C₆-Spirocyclus stehen, der mit einer gegebenenfalls durch ein Sauerstoff- oder Schwefelatom unterbrochenen Alkylendiyl- oder durch eine Alkylendioxyl-Gruppe substituiert ist, die mit dem Kohlenstoffatom, an das sie gebunden ist, einen weiteren fünf- bis siebengliedrigen Spirocyclus bildet oder
A,B und das Kohlenstoffatom, an das sie gebunden sind für einen C₃-C₆-Spirocyclus stehen, bei dem zwei Substituenten gemeinsam mit den C-Atomen, an die sie gebunden sind für einen gesättigten oder ungesättigten fünf- oder sechsgliedrigen Cyclus stehen, der durch Sauerstoff oder Schwefel unterbrochen sein kann.
X für Methyl, Ethyl, Propyl, 2-Propyl, Fluor, Chlor, Brom, Methoxy oder Ethoxy steht,
Y für Wasserstoff, Methyl, Ethyl, Propyl, i-Propyl, Butyl, i-Butyl, tert.-Butyl, Fluor, Chlor, Brom, Methoxy, Ethoxy oder Trifluormethyl steht,
Z für Methyl, Ethyl, Propyl, i-Propyl, Butyl, i-Butyl, tert.-Butyl, Fluor, Chlor, Brom, Methoxy oder Ethoxy steht,
n für eine Zahl von 0 - 1 steht,
G für Wasserstoff (a) oder für die Gruppen der Formeln 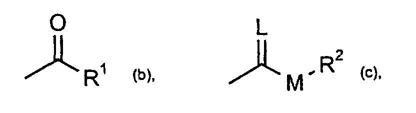 oder E (f) steht, in welchen
E für ein Metallionäquivalent oder ein Ammoniumion steht und
L und M für Sauerstoff stehen,
R¹ für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Alkylthio-C₂-C₆-alkyl, C₁-C₄-Polyalkoxy-C₂-C₄-alkyl oder Cycloalkyl mit 3 bis 6 Ringatomen, das durch 1 bis 2 Sauerstoff- und/oder Schwefelatome unterbrochen sein kann, steht,
für gegebenenfalls durch Fluor, Chlor, Brom, Methyl, Ethyl, Propyl, i-Propyl, Methoxy, Ethoxy, Trifluormethyl, Trifluormethoxy, Nitro-substituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy-substituiertes Phenyl-C₁-C₃-alkyl steht,
für gegebenenfalls durch Fluor, Chlor, Brom, Methyl, Ethyl-substituiertes Furanoyl, Pyridyl, Pyrimidyl, Thiazolyl und Pyrazolyl steht,
für gegebenenfalls durch Fluor, Chlor, Methyl, Ethyl-substituiertes Phenoxy-C₁-C₄-alkyl steht,
für gegebenenfalls durch Fluor, Chlor, Amino, Methyl-, Ethyl, substituiertes Pyridyloxy-C₁-C₄-alkyl, Pyrimidyloxy-C₁-C₄-alkyl und Thiazolyloxy-C₁-C₄-alkyl steht,
R² für gegebenenfalls durch Fluor oder Chlor substituiertes C₁-C₁₄-Alkyl, C₃-C₁₄-Alkenyl, C₁-C₄-Alkoxy-C₂-C₆-alkyl, C₁-C₄-Polyalkoxy-C₂-C₆-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl oder Benzyl steht,

3. Verfahren zur Herstellung der substituierten 1-H-3-Aryl-pyrrolidin-2,4-dion-Derivate der Formel (I) gemäß Anspruch 1, **dadurch gekennzeichnet, daß** man
(A) im Fall der 1-H-3-Aryl-pyrrolidin-2,4-dione bzw. deren Enole der Formel (Ia) 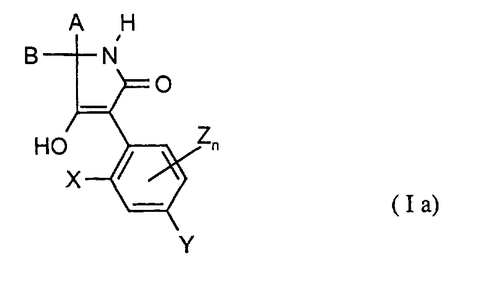 in welcher
A, B, X, Y, Z und n die in Anspruch 1 angegebene Bedeutung haben,
N-Acylaminosäureester der Formel (II) 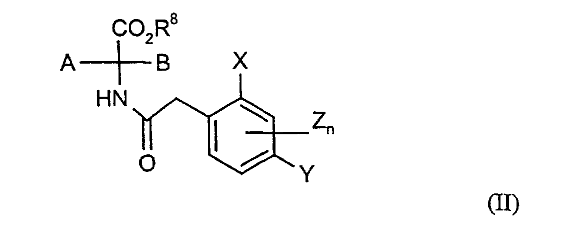 in welcher
A, B, X, Y, Z und n die oben angegebene Bedeutung haben,
und
R⁸ für Alkyl steht,
in Gegenwart eines Verdünnungsmittels und in Gegenwart einer Base intramolekular kondensiert;
oder
(B) im Fall der Verbindungen der Formel (Ib) 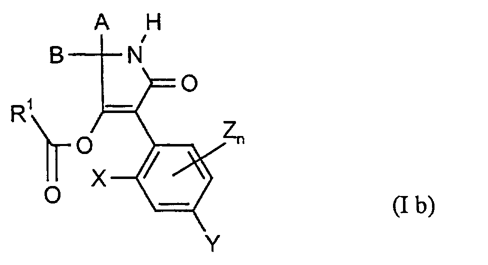 in welcher
A, B, X, Y, Z, R¹ und n die oben angegebene Bedeutung haben,
Verbindungen der Formel (Ia), 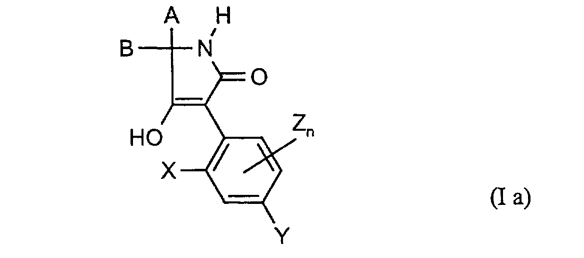
in welcher
A,B,X,Y,Z und n die oben angegebene Bedeutung haben,
α) mit Säurehalogeniden der allgemeinen Formel (III) 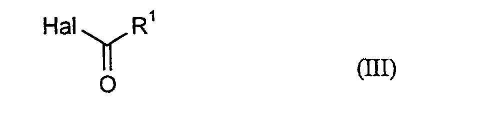 in welcher
R¹ die oben angegebene Bedeutung hat und
Hal für Halogen, insbesondere Chlor und Brom steht,
gegebenenfalls in Gegenwart eines Verdünnungsmittels und gegebenenfalls in Gegenwart eines Säurebindemittels umsetzt
oder
β) mit Carbonsäureanhydriden der allgemeinen Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels,
umsetzt;
oder
(C) im Falle der Verbindungen der Formel (Ic-1) 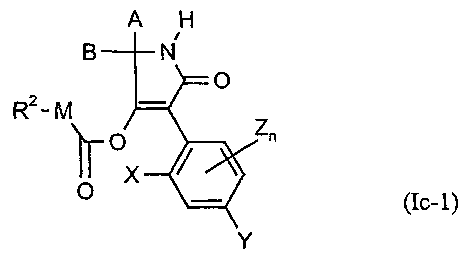 in welcher
A, B, X, Y, Z, R² und n die oben angegebene Bedeutung haben,
und
M für Sauerstoff steht,
Verbindungen der Formel (Ia) 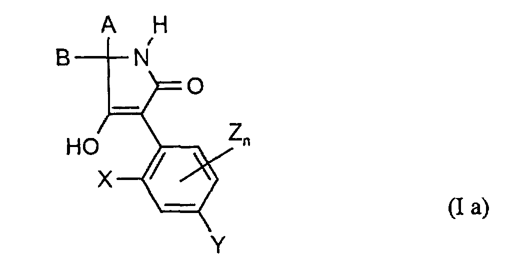 in welcher
A, B, X, Y, Z und n die oben angegebene Bedeutung haben,
mit Chlorameisensäureester oder Chlorameisensäurethiolester der allgemeinen Formel (V)
R²-M-CO-Cl (V)
in welcher
R² und M die oben angegebene Bedeutung haben,
gegebenenfalls in Gegenwart eines Verdünnungsmittels und gegebenenfalls in Gegenwart eines Säurebindemittels umsetzt;
oder
(G) im Fall der Verbindungen der Formel (If) 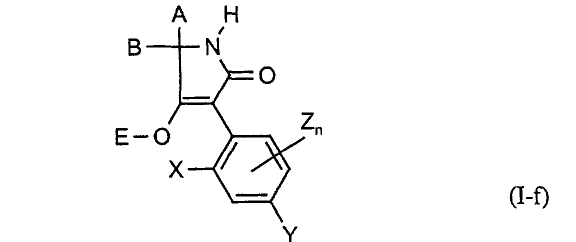 in welcher
A, B, X, Y, Z und n die oben angegebene Bedeutung haben,
und
E für ein Metallionäquivalent oder für ein Ammoniumion steht,
Verbindungen der Formel (Ia) 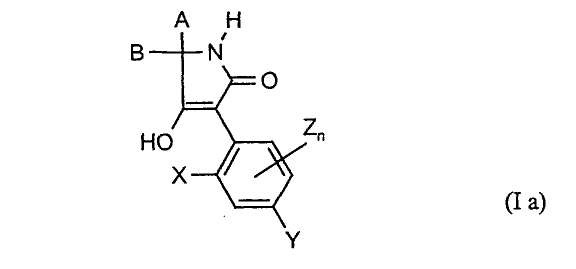 in welcher
A,B,X,Y,Z und n die oben angegebene Bedeutung haben,
mit Metallhydroxiden oder Aminen der allgemeinen Formeln (X) und (XI)
Meₛ OHₜ (X) 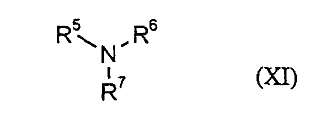
in welchen
Me für ein- oder zweiwertige Metallionen,
s und t für die Zahl 1 und 2 und
R⁵, R⁶ und R⁷ unabhängig voneinander für Wasserstoff und Alkyl stehen,
gegebenenfalls in Gegenwart eines Verdünnungsmittels, umsetzt,

4. Verbindungen der Formel (II) 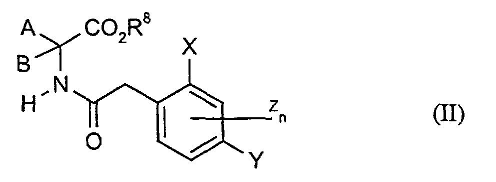 in welcher
A, B, X, Y, Z und n die in Anspruch 1 und R⁸ die in Anspruch 4 angegebene Bedeutung haben.

5. Verfahren zur Herstellung der Verbindungen der Formel (II), **dadurch gekennzeichnet, daß** man Aminosäurederivate der Formel (XIV), 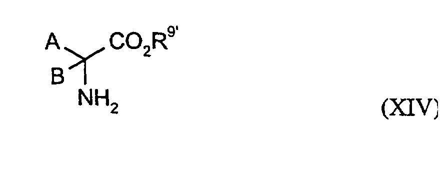 in welcher
R9' für Wasserstoff (XIVa) und Alkyl (XIVb) steht
und
A und B die oben angegebene Bedeutung haben
mit Phenylessigsäurehalogeniden der Formel (XV) 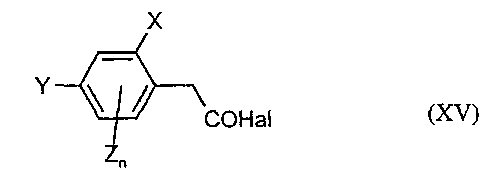 in welcher
X, Y, Z und n die oben angegebene Bedeutung haben und
Hal für Chlor oder Brom steht,
acyliert
oder Acylaminosäuren der Formel (IIa), 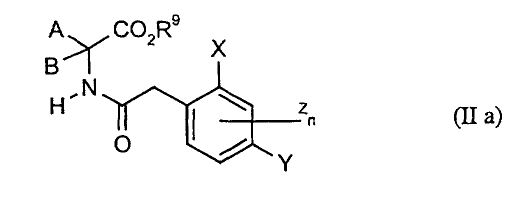 in welcher
A, B, X, Y, Z und n die oben angegebene Bedeutung haben,
und
R⁹ für Wasserstoff steht,
verestert,
oder Aminonitrile der Formel (XVI) 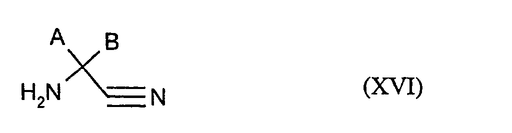 in welcher
A und B die oben angegebene Bedeutung haben,
mit Phenylessigsäurehalogeniden der Formel (XV) 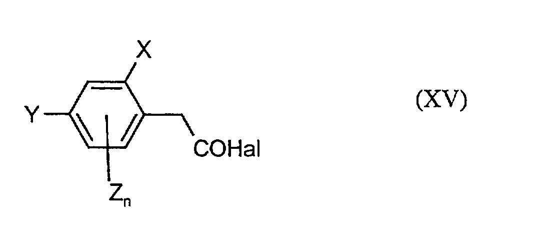 in welcher
X, Y, Z und n die oben angegebene Bedeutung haben und
Hal für Chlor oder Brom steht,
zu Verbindungen der Formel (XVII) 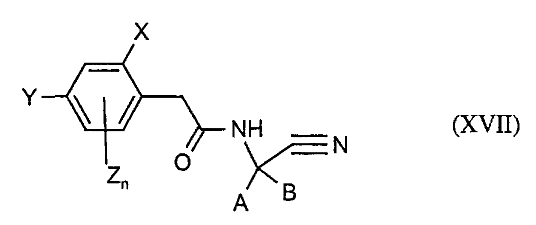 in welcher
A, B, X, Y, Z und n die oben angegebene Bedeutung haben,
umsetzt, die anschließend einer schwefelsauren Alkoholyse unterworfen werden.

6. Verbindungen der Formel (XVII) 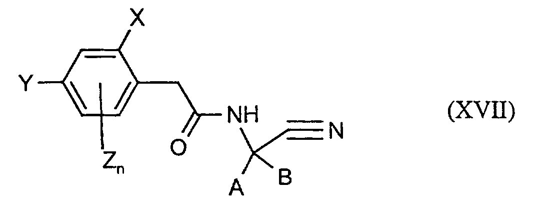 in welcher
A, B, X, Y, Z und n die in Anspruch 1 angegebene Bedeutung haben.

7. Verfahren zur Herstellung der Verbindungen der Formel (XVII) gemäß Anspruch 9, **dadurch gekennzeichnet, daß** man Aminonitrile der Formel (XVI) 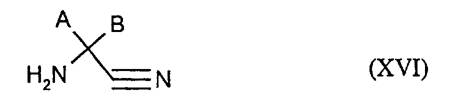 in welcher
A und B die in Anspruch 9 angegebene Bedeutung haben,
mit Phenylessigsäurehalogeniden der Formel (XV) 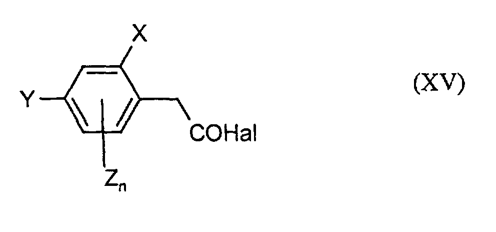 in welcher
X, Y, Z und n die in Anspruch 9 angegebene Bedeutung haben und
Hal für Chlor oder Brom steht,
umsetzt.

8. Schädlingsbekämpfungsmittel und/oder Unkrautbekämpfungsmittel, **gekennzeichnet durch** einen Gehalt an mindestens einer Verbindung der Formel (I) gemäß Anspruch 1.

9. Arthropodizide, nematizide und herbizide Mittel, **gekennzeichnet durch** einen Gehalt an mindestens einer Verbindung der Formel (I).

10. Verwendung von Verbindungen der Formel (I) gemäß Anspruch 1 zur Bekämpfung von Schädlingen im Pflanzenschutz, Haushaltsbereich, Hygienebereich und Vorratsschutz.

11. Verwendung von Verbindungen der Formel (I) gemäß Anspruch 1 zur Bekämpfung von Arthropoden, Nematoden und unerwünschtem Pflanzenbewuchs im Pflanzenschutz, Haushaltsbereich, Hygienebereich und Vorratsschutz.

12. Verfahren zur Bekämpfung von Schädlingen im Pflanzenschutz, Haushaltsbereich, Hygienebereich und Vorratsschutz, **dadurch gekennzeichnet, daß** man Verbindungen der Formel (I) gemäß Anspruch 1 auf die Schädlinge und/oder ihren Lebensraum einwirken läßt.

13. Verfahren zur Herstellung von Schädlingsbekämpfungsmitteln und/oder Unkrautbekämpfungsmitteln, **dadurch gekennzeichnet, daß** man Verbindungen der Formel (I) gemäß Anspruch 1 mit Streckmitteln und/oder oberflächenaktiven Mitteln vermischt.

14. Verwendung von Verbindungen der Formel (I) gemäß Anspruch 1 zur Herstellung von Schädlingsbekämpfungsmitteln zur Bekämpfung von Ekto- und Endoparasiten.

15. Verfahren zur Bekämpfung von unerwünschtem Pflanzenbewuchs, **dadurch gekennzeichnet, daß** man Verbindungen der Formel (I) gemäß Anspruch 1 auf Unkräuter und/oder ihren Lebensraum einwirken läßt.

## Claims

1. Substituted 1H-3-arylpyrrolidine-2,4-dione derivatives of the formula (I) 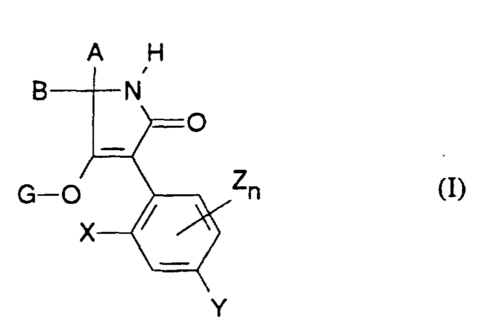 in which
A, B and the carbon atom to which they are bonded represent a C₃-C₆-spirocycle which can be monosubstituted or polysubstituted by C₁-C₆-alkyl, C₃-C₈-cycloalkyl, C₁-C₃-haloalkyl, C₁-C₆-alkoxy, C₁-C₄-thioalkyl, fluorine, chlorine or phenyl, or
A, B and the carbon atom to which they are bonded represent a C₃-C₆-spirocycle which is substituted by an alkylenediyl group which is optionally interrupted by one or two oxygen or sulphur atoms or by an alkylenedioxyl or an alkylenedithiooxyl group which, together with the carbon atom to which it is bonded, forms a further five- to seven-membered spirocycle, or
A, B and the carbon atom to which they are bonded represent a C₃-C₆-spirocycle in which two substituents together with the carbon atoms to which they are bonded represent a saturated or unsaturated carbocycle which is substituted by alkyl(C₁-C₃), alkoxy(C₁-C₃) or fluorine, chlorine or bromine and which can be interrupted by oxygen or sulphur,
X represents C₁-C₄-alkyl, halogen or C₁-C₄-alkoxy,
Y represents hydrogen, methyl, ethyl, propyl, i-propyl, butyl, i-butyl, fluorine, chlorine, bromine, methoxy, ethoxy or trifluoromethyl,
z represents methyl, ethyl, propyl, i-propyl, butyl, i-butyl, tert.-butyl, fluorine, chlorine, bromine, methoxy or ethoxy,
N represents a number from 0 - 2,
G represents hydrogen (a) or the groups of the formula 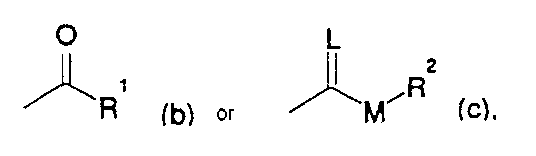
E (f)
in which
E represents a metal ion equivalent or an ammonium ion and
L and M represent oxygen,
R¹ represents optionally halogen-substituted C₁-C₁₆-alkyl, C₂-C₁₆-alkenyl, C₁-C₆-alkoxy-C₂-C₆-alkyl, C₁-C₁₆-alkylthio-C₂-C₆-alkyl, C₁-C₆-polyalkoxy-C₂-C₆-alkyl or cycloalkyl which has 3 to 7 ring atoms and which can be interrupted by 1 to 2 oxygen and/or sulphur atoms, or represents phenyl which is optionally substituted by halogen, nitro, C₁-C₄-alkyl, C₁-C₄-alkoxy, C₁-C₃-halogenoalkyl or C₁-C₃-halogenoalkoxy,
or represents phenyl-C₁-C₄-alkyl which is optionally substituted by halogen, C₁-C₄-alkyl, C₁-C₄-alkoxy, C₁-C₃-halogenoalkyl or C₁-C₃-halogenoalkoxy, or represents hetaryl which is optionally substituted by fluorine, chlorine, bromine and/or C₁-C₄-alkyl,
or represents phenoxy-C₁-C₅-alkyl which is optionally substituted by fluorine, chlorine, bromine and C₁-C₄-alkyl,
or represents hetaryloxy-C₁-C₅-alkyl which is optionally substituted by fluorine, chlorine, bromine, amino and C₁-C₄-alkyl,
R² represents in each case optionally halogen-substituted C₁-C₁₆-alkyl, C₃-C₁₆-alkenyl, C₁-C₆-alkoxy-C₂-C₆-alkyl or C₁-C₆-Polyalkoxy-C₂-C₆-alkyl,
or represents phenyl or benzyl, in each case optionally substituted by halogen, nitro, C₁-C₄-alkyl, C₁-C₃-alkoxy or C₁-C₃-halogenoalkyl.

2. Compounds of the general formula (I) according to Claim 1 in which
A, B and the carbon atom to which they are bonded represent a C₃-C₆-spirocycle which can be at least monosubstituted or polysubstituted by methyl, ethyl, propyl, isopropyl, butyl, isobutyl, sec.-butyl, tert.-butyl, cyclohexyl, trifluoromethyl, methoxy, ethoxy, propoxy, isopropoxy, butoxy, isobutoxy, sec.-butoxy, tert.-butoxy, methylthio, fluorine, chlorine or phenyl, or
A, B and the carbon atom to which they are bonded represent a C₃-C₆-spirocycle which is substituted by an alkylenediyl group which is optionally interrupted by an oxygen or sulphur atom or by an alkylenedioxyl group which group, together with the carbon atom to which it is bonded, forms a further five- to seven-membered spirocycle,
A, B and the carbon atom to which they are bonded represent a C₃-C₆-spirocycle in which two substituents together with the carbon atoms to which they are bonded represent a saturated or unsaturated five- or six-membered carbocycle which can be interrupted by oxygen or sulphur.
X represents methyl, ethyl, propyl, 2-propyl, fluorine, chlorine, bromine, methoxy or ethoxy,
Y represents hydrogen, methyl, ethyl, propyl, i-propyl, butyl, i-butyl, tert.-butyl, fluorine, chlorine, bromine, methoxy, ethoxy or trifluoromethyl,
Z represents methyl, ethyl, propyl, i-propyl, butyl, i-butyl, tert.-butyl, fluorine, chlorine, bromine, methoxy or ethoxy,
n represents a number from 0 - 1,
G represents hydrogen (a) or the groups of the formulae 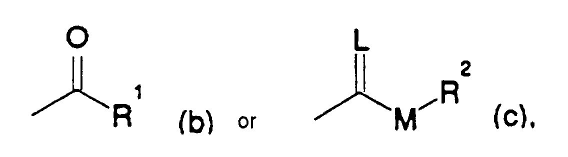
E (F)
in which
E represents a metal ion equivalent or an ammonium ion and
L and M represent oxygen and/or sulphur,
R¹ represents in each case optionally fluorine- or chlorine-substituted C₁-C₁₄-alkyl, C₂-C₁₄-alkenyl, C₁-C₄-alkoxy-C₂-C₆-alkyl, C₁-C₄-alkylthio-C₂-C₆-alkyl, C₁-C₄-polyalkoxy-C₂-C₄-alkyl or cycloalkyl which has 3 to 6 ring atoms and which can be interrupted by 1 to 2 oxygen and/or sulphur atoms,
or represents phenyl which is optionally substituted by fluorine, chlorine, bromine, methyl, ethyl, propyl, i-propyl, methoxy, ethoxy, trifluoromethyl, trifluoromethoxy or nitro,
or represents phenyl-C₁-C₃-alkyl which is optionally substituted by fluorine, chlorine, bromine, methyl, ethyl, propyl, i-propyl, methoxy, ethoxy, trifluoromethyl or trifluoromethoxy,
or represents furanoyl, pyridyl, pyrimidyl, thiazolyl and pyrazolyl, in each case optionally substituted by fluorine, chlorine, bromine, methyl or ethyl,
or represents phenoxy-C₁-C₄-alkyl which is optionally substituted by fluorine, chlorine, methyl or ethyl,
or represents pyridyloxy-C₁-C₄-alkyl, pyrimidyloxy-C₁-C₄-alkyl and thiazolyloxy-C₁-C₄-alkyl, in each case optionally substituted by fluorine, chlorine, amino, methyl or ethyl,
R² represents in each case optionally fluorine- or chlorine substituted C₁-C₁₄-alkyl, C₃-C₁₄-alkenyl, C₁-C₄-alkoxy-C₂-C₆-alkyl or C₁-C₄-polyalkoxy-C₂-C₆-alkyl,
or represents phenyl or benzyl, in each case optionally substituted by fluorine, chlorine, nitro, methyl, ethyl, propyl, i-propyl, methoxy, ethoxy or trifluoromethyl.

3. Process for the preparation of the substituted 1-H-3-aryl-pyrrolidine-2,4-dione derivatives of the formula (I) according to Claim 1, **characterised in that**
(A) in the case of the 1-H-3-aryl-pyrrolidine-2,4-diones or of their enols of the formula (Ia) 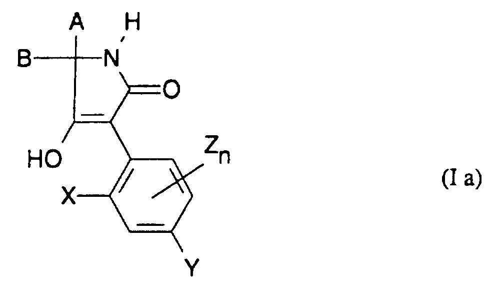 in which
A, B, X, Y, N and n have the meaning given in Claim 1,
N-acylamino acid esters of the formula (II) 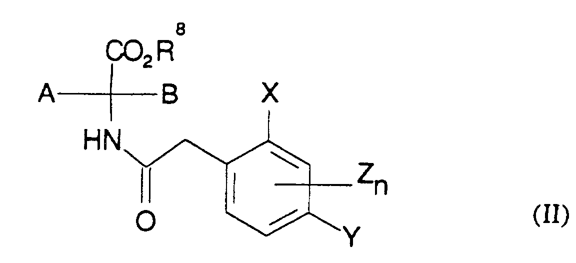 in which
A, B, X, Y, Z and n have the abovementioned meaning and
R⁸ represents alkyl,
are subjected to an intramolecular condensation reaction in the presence of a diluent and in the presence of a base;
or
(B) in the case of the compounds of the formula (Ib), 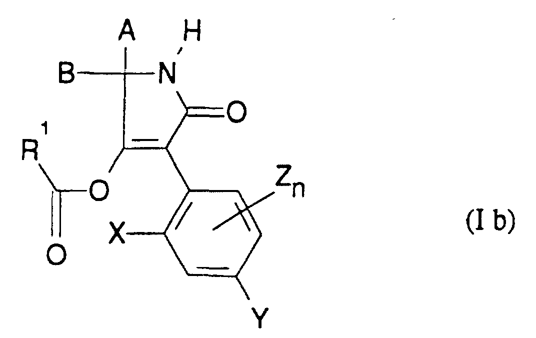 in which
A, B, X, Y, Z, R¹ and n have the abovementioned meaning,
compounds of the formula (Ia) 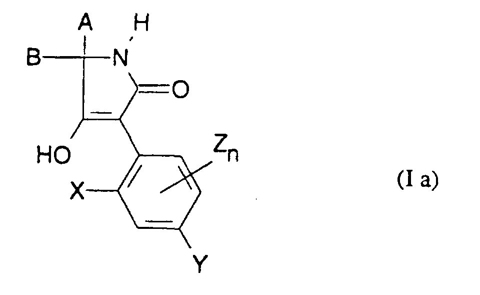 in which
A, B, Y, Z and n have the abovementioned meaning
α) are reacted with acid halides of the general formula (III) 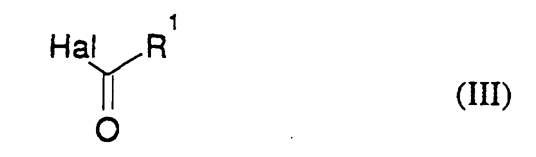 in which
R¹ has the abovementioned meaning and
Hal represents halogen, in particular chlorine and bromine,
if appropriate in the presence of a diluent and if appropriate in the presence of an acid-binding agent,
or
β) are reacted with carboxylic anhydrides of the general formula (IV)
R¹-CO-O-CO-R¹ (IV)
in which
R¹ has the abovementioned meaning,
if appropriate in the presence of a diluent and if appropriate in the presence of an acid-binding agent;
or
(C) in the case of the compounds of the formula (Ic-1) 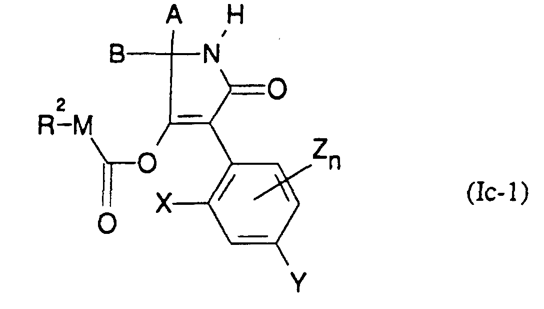 in which
A, B, X, Y, Z, R² and n have the abovementioned meaning
and
M represents oxygen,
compounds of the formula (Ia) 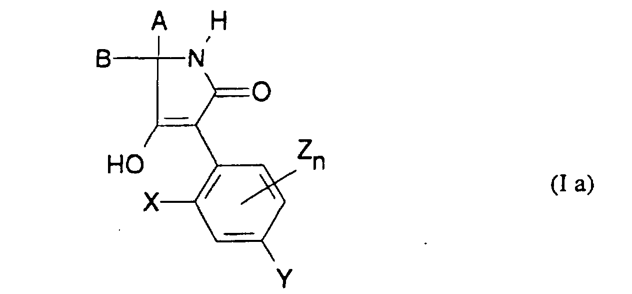 in which
A, B, X, Y, Z and n have the abovementioned meaning, are reacted with chloroformic esters or chloroformic thioesters of the general formula (V)
R²-M-CO-Cl (V)
in which
R² and M have the abovementioned meaning,
if appropriate in the presence of a diluent and if appropriate in the presence of an acid-binding agent;
or
(G)in the case of the compounds of the formula (If) 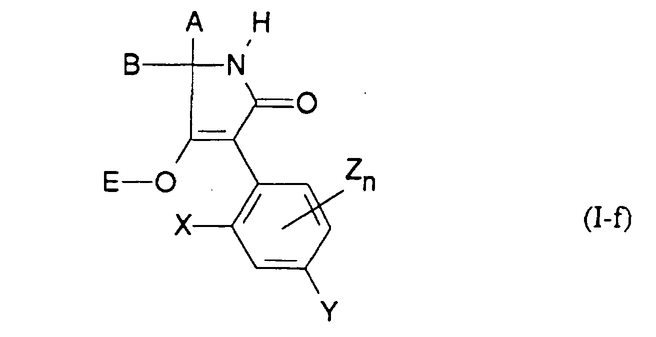 in which
A, B, X, Y, Z and n have the abovementioned meaning
and
E represents a metal ion equivalent or an ammonium ion
compounds of the formula (Ia) 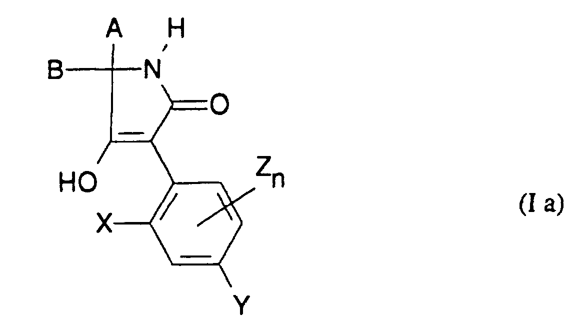 in which
A, B, X, Y, Z and n have the abovementioned meaning
are reacted with metal hydroxides or amines of the general formulae (X) and (XI)
MeₛOHₜ (X) 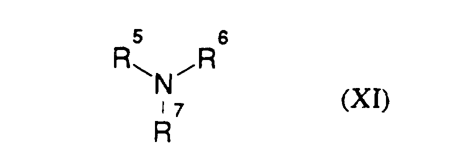
in which
Me represents mono- or divalent metal ions,
s and t represent the numbers 1 and 2 and
R⁵, R⁶ and R⁷ independently of one another represent hydrogen and alkyl,
if appropriate in the presence of a diluent.

4. Compounds of the formula (II) 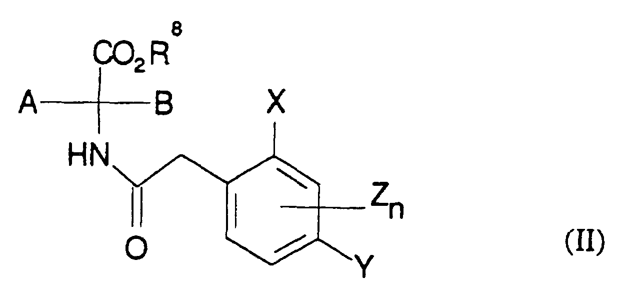 in which
A, B, X, Y, Z and n have the meaning given in Claim 1 and R⁸ has the meaning given in Claim 4.

5. Process for the preparation of the compounds of the formula (II), **characterised in that** amino acid derivatives of the formula (XIV) 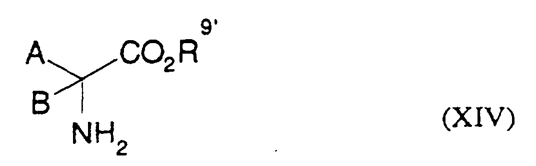 in which
R9' represents hydrogen (XIVa) and alkyl (XIVb)
and
A and B have the abovementioned meaning
are acylated with phenylacetyl halides of the formula (XV) 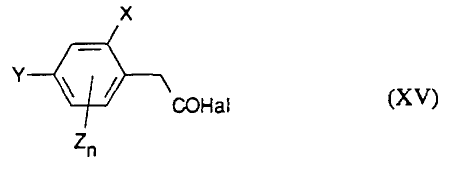 in which
X, Y, Z and n have the abovementioned meaning and
Hal represents chlorine or bromine
or acylamino acids of the formula (IIa) 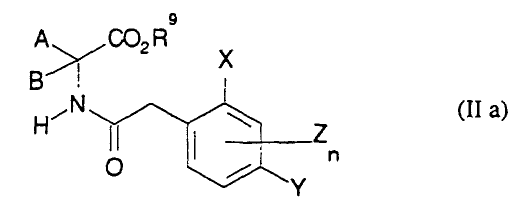 in which
A, B, X, Y, Z and n have the abovementioned meaning and
R9 represents hydrogen
are esterified,
or aminonitriles of the formula (XVI) 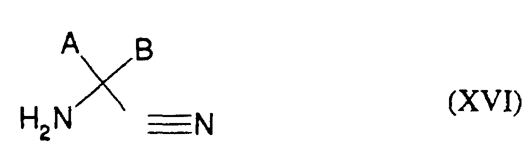 in which
A and B have the abovementioned meaning
are reacted with phenylacetyl halides of the formula (XV) 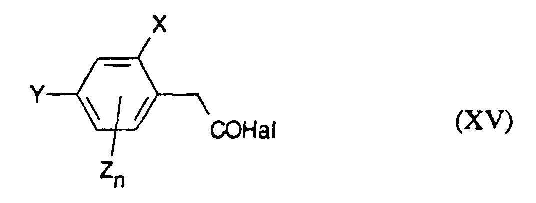
in which
X, Y, Z and n have the abovementioned meaning and
Hal represents chlorine or bromine
to give compounds of the formula (XVII) 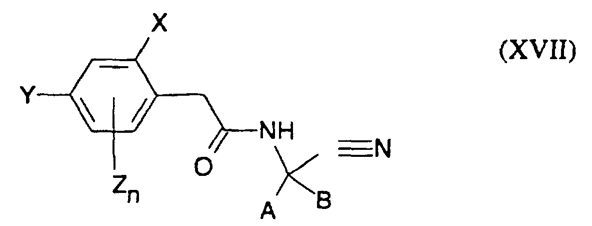 in which
A, B, X, Y, Z and n have the abovementioned meaning
and these compounds are subsequently subjected to alcoholysis in sulphuric acid.

6. Compounds of the formula (XVII) 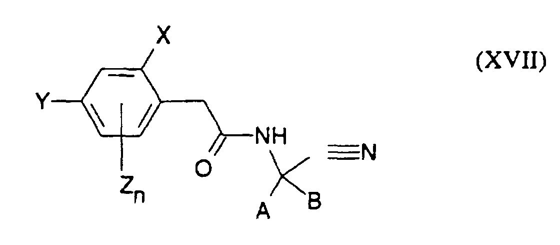 in which
A, B, X, Y, Z and n have the meaning given in Claim 1.

7. Process for the preparation of the compounds of the formula (XVII) according to Claim 9, **characterised in that** aminonitriles of the formula (XVI) 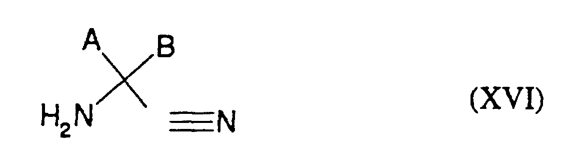 in which
A and B have the meaning given in Claim 9 are reacted with phenylacetyl halides of the formula (XV) 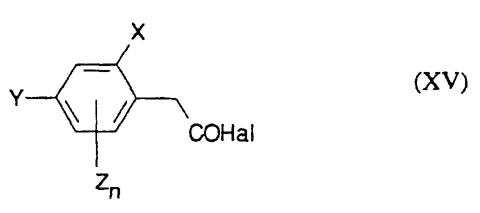 in which
X, Y, Z and n have the meaning given in Claim 9 and
Hal represents chlorine or bromine.

8. Pesticides and/or weed-killers, **characterised in that** they contain at least one compound of the formula (I) according to Claim 1.

9. Arthropodicidal, nematicidal and herbicidal agents, **characterised in that** they contain at least one compound of the formula (I).

10. Use of compounds of the formula (I) according to Claim 1 for combating pests in plant protection, on domestic premises, in the hygiene field and in the protection of stored products.

11. Use of compounds of the formula (I) according to Claim 1 for combating arthropods, nematodes and undesired vegetation in plant protection, on domestic premises, in the hygiene field and in the protection of stored products.

12. Method of combating pests in plant protection, on domestic premises, in the hygiene field and in the protection of stored products, **characterised in that** compounds of the formula (I) according to Claim 1 are allowed to act on the pests and/or their environment.

13. Process for the preparation of pesticides and/or weed-killers, **characterised in that** compounds of the formula (I) according to Claim 1 are mixed with extenders and/or surface-active agents.

14. Use of compounds of the formula (I) according to Claim 1 for the preparation of pesticides for combating ecto- and endoparasites.

15. Method of combating undesired vegetation, **characterized in that** compounds of the formula (I) according to Claim 1 are allowed to act on weeds and/or their environment.

## Revendications

1. Dérivés substitués de l-H-3-arylpyrrolidine-2,4-diones de formule (I) 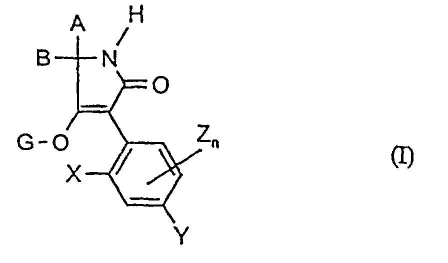 dans laquelle
A, B et l'atome de carbone auquel ils sont liés représentent un cycle spiranique en C₃ à C₆ qui peut être substitué une ou plusieurs fois par un groupe alkyle en C₁ à C₆, cycloalkyle en C₃ à C₈, halogénalkyle en C₁ à C₃, alkoxy en C₁ à C₆, thioalkyle en C₁ à C₄, du fluor, du chlore ou un groupe phényle, ou bien
A, B et l'atome de carbone auquel ils sont liés représentent un cycle spiranique en C₃ à C₆ qui est substitué par un groupe alkylènediyle éventuellement interrompu par un ou deux atomes d'oxygène ou de soufre ou par un groupe alkylènedioxyle ou par un groupe alkylènedithio-oxyle qui forme un autre cycle spiranique pentagonal à heptagonal avec l'atome de carbone auquel il est lié, ou bien
A, B et l'atome de carbone auquel ils sont liés représentent un cycle spiranique en C₃ à C₆ dans lequel deux substituants forment conjointement avec les atomes de carbone auxquels ils sont liés un cycle saturé ou non saturé, substitué par un radical alkyle en C₁ à C₃, alkoxy en C₁ à C₃ ou par du fluor, du chlore ou du brome, qui peut être interrompu par de l'oxygène ou du soufre,
X représente un groupe alkyle en C₁ à C₄, un halogène ou un groupe alkoxy en C₁ à C₄,
Y représente l'hydrogène, un groupe méthyle, éthyle, propyle, isopropyle, butyle, isobutyle, le fluor, le chlore, le brome, un groupe méthoxy, éthoxy ou trifluorométhyle
z est un groupe méthyle, éthyle, propyle, isopropyle, butyle, isobutyle, tertio-butyle, du fluor, du chlore, du brome, un groupe méthoxy ou éthoxy,
n représente un nombre de 0 à 2,
G représente l'hydrogène (a) ou les groupes de formule 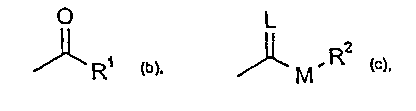 ou E (f)
dans laquelle
E représente un équivalent d'ion métallique ou un ion ammonium et
L et M représentent l'oxygène,
R¹ est un groupe, éventuellement substitué par un halogène, alkyle en C₁ à C₁₆, alcényle en C₂ à C₁₆, (alkoxy en C₁ à C₆)-(alkyle en C₂ à C₆), (alkylthio en C₁ à C₁₆)-(alkyle en C₂ à C₆), poly-(alkoxy en C₁ à C₆)-(alkyle en C₂ à C₆) ou un groupe cycloalkyle à noyau de 3 à 7 atomes qui peut être interrompu par un ou deux atomes d'oxygène et/ou de soufre, un groupe phényle éventuellement substitué par un radical halogéno, nitro, alkyle en C₁ à C₄, alkoxy en C₁ à C₄, halogénalkyle en C₁ à C₃, halogénalkoxy en C₁ à C₃,
un groupe phényl-(alkyle en C₁ à C₄) éventuellement substitué par un radical halogéno, alkyle en C₁ à C₄, alkoxy en C₁ à C₄, halogénalkyle en C₁ à C₃, ou halogénalkoxy en C₁ à C₃, un groupe hétaryle éventuellement substitué par un radical fluoro, chloro, bromo et/ou alkyle en C₁ à C₄,
un groupe phénoxy-(alkyle en C₁ à C₅) éventuellement substitué par des radicaux fluoro, chloro, bromo et alkyle en C₁ à C₄,
un groupe hétaryloxy-(alkyle en C₁ à C₅) éventuellement substitué par des radicaux fluoro, chloro, bromo, amino et alkyle en C₁ à C₄,
R² représente un groupe, éventuellement substitué par un halogène, alkyle en C₁ à C₁₆, alcényle en C₃ à C₁₆, (alkoxy en C₁ à C₆)-(alkyle en C₂ à C₆), poly-(alkoxy en C₁ à C₆)-(alkyle en C₂ à C₆),
un groupe phényle ou benzyle éventuellement substitué par un radical halogéno, nitro, alkyle en C₁ à C₄, alkoxy en C₁ à C₃, halogénalkyle en C₁ à C₃.

2. Composés de formule générale (I) suivant la revendication 1, dans laquelle
A, B et l'atome de carbone auquel ils sont liés représentent un cycle spiranique en C₃ à C₆ qui peut être substitué au moins une ou plusieurs fois par un radical méthyle, éthyle, propyle, isopropyle, butyle, iso-butyle, sec.-butyle, tertio-butyle, cyclohexyle, trifluorométhyle, méthoxy, éthoxy, propoxy, isopropoxy, butoxy, isobutoxy, sec.-butoxy, tertiobutoxy, méthylthio, fluoro, chloro ou phényle, ou bien
A, B et l'atome de carbone auquel ils sont liés représentent un cycle spiranique en C₃ à C₆ qui est substitué avec un groupe alkylènediyle éventuellement interrompu par un atome d'oxygène ou de soufre, ou par un groupe alkylènedioxyle qui forme un autre cycle spiranique pentagonal à heptagonal avec l'atome de carbone auquel il est lié,
A, B et l'atome de carbone auquel ils sont liés représentent un cycle spiranique en C₃ à C₆ dans lequel deux substituants forment conjointement avec les atomes de carbone auxquels ils sont liés un cycle pentagonal ou hexagonal saturé ou non saturé qui peut être interrompu par de l'oxygène ou du soufre,
X représente un groupe méthyle, éthyle, propyle, 2-propyle, du fluor, du chlore, du brome, un groupe méthoxy ou éthoxy,
Y représente l'hydrogène, un groupe méthyle, éthyle, propyle, isopropyle, butyle, isobutyle, tertio-butyle, du fluor, du chlore, du brome, un groupe méthoxy, éthoxy ou trifluorométhyle,
Z représente un groupe méthyle, éthyle, propyle, isopropyle, butyle, isobutyle, tertio-butyle, du fluor, du chlore, du brome, un groupe méthoxy ou éthoxy,
n représente un nombre de 0-1,
G représente l'hydrogène (a) ou les groupes de formule 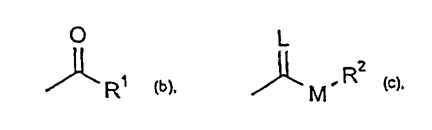 ou E (f)
dans laquelle
E représente un équivalent d'ion métallique ou un ion ammonium et
L et M représentent de l'oxygène,
R¹ représente un groupe, éventuellement substitué par du fluor ou du chlore, alkyle en C₁ à C₁₄, alcényle en C₂ à C₁₄, (alkoxy en C₁ à C₄)-(alkyle en C₂ à C₆), (alkylthio en C₁ à C₄)-(alkyle en C₂ à C₆), poly-(alkoxy en C₁ à C₄)-(alkyle en C₂ à C₄) ou un groupe cycloalkyle à noyau de 3 à 6 atomes, qui peut être interrompu par un ou deux atomes d'oxygène et/ou de soufre,
un groupe phényle éventuellement substitué par un radical fluoro, chloro, bromo, méthyle, éthyle, propyle, isopropyle, méthoxy, éthoxy, trifluorométhyle, trifluorométhoxy ou nitro,
un groupe phényl-(alkyle en C₁ à C₃) éventuellement substitué par un radical fluoro, chloro, bromo, méthyle, éthyle, propyle, isopropyle, méthoxy, éthoxy, trifluorométhyle, trifluorométhoxy,
un groupe furannoyle, pyridyle, pyrimidyle, thiazolyle ou pyrazolyle éventuellement substitué par un radical fluoro, chloro, bromo, méthyle ou éthyle,
un groupe phénoxy-(alkyle en C₁ à C₄) éventuellement substitué par un radical fluoro, chloro, méthyle ou éthyle,
un groupe, éventuellement substitué par un radical fluoro, chloro, amino, méthyle ou éthyle, pyridyloxy-(alkyle en C₁ à C₄), pyrimidyloxy-(alkyle en C₁ à C₄) ou thiazolyloxy-(alkyle en C₁ à C₄),
R² représente un groupe, éventuellement substitué par du fluor ou du chlore, alkyle en C₁ à C₁₄, alcényle en C₃ à C₁₄, (alkoxy en C₁ à C₄)-(alkyle en C₂ à C₆) ou poly-(alkoxy en C₁ à C₄)-(alkyle en C₂ à C₆),
ou bien un groupe phényle ou benzyle éventuellement substitué par un radical fluoro, chloro, nitro, méthyle, éthyle, propyle, isopropyle, méthoxy, éthoxy ou trifluorométhyle.

3. Procédé de production des dérivés substitués de 1-H-3-aryl-pyrrolidine-2,4-diones de formule (I) suivant la revendication 1, **caractérisé** en que
(A) dans le cas des 1-H-3-aryl-pyrrolidine-2,4-diones ou de leurs énols de formule (Ia) 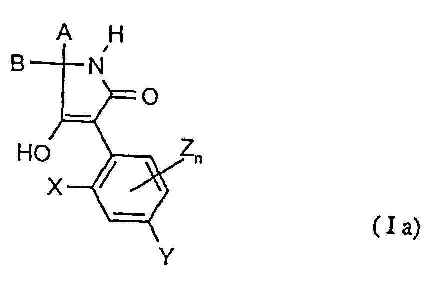 dans laquelle
A, B, X, Y, Z et n ont la définition indiquée dans la revendication 1,
on effectue la condensation intramoléculaire d'esters de N-acylamino-acides de formule (II) 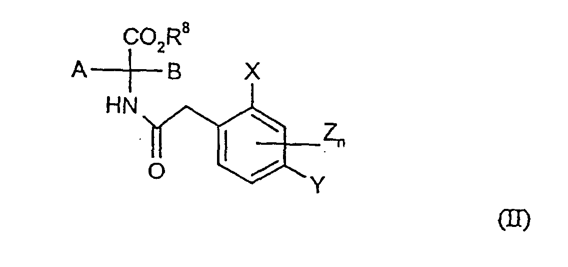 dans laquelle
A, B, X, Y, Z et n ont la définition indiquée ci-dessus
et
R⁸ est un groupe alkyle,
en présence d'un diluant et en présence d'un base ;
ou bien
(B) dans le cas des composés de formule (Ib) 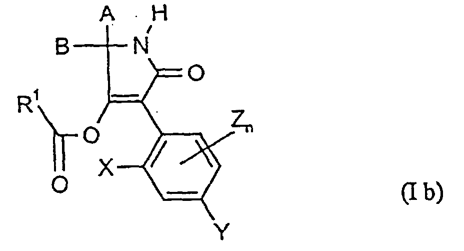 dans laquelle
A, B, X, Y, Z, R¹ et n ont la définition indiquée ci-dessus,
on fait réagir des composés de formule (Ia) 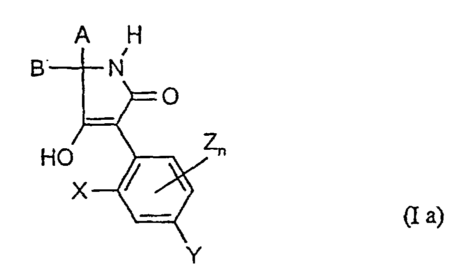 dans laquelle
A, B, X, Y, Z et n ont la définition indiquée ci-dessus,
α) avec des halogénures d'acides de formule générale (III) 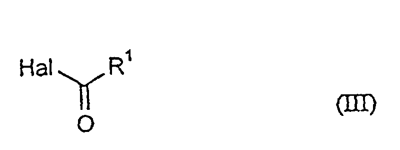 dans laquelle
R¹ a la définition indiquée ci-dessus et
Hal représente un halogène, en particulier le chlore et le brome,
éventuellement en présence d'un diluant et le cas échéant en présence d'un accepteur d'acide,
ou bien
β) avec des anhydrides d'acides carboxyliques de formule générale (IV)
R¹-CO-O-CO-R¹ (IV)
dans laquelle
R¹ a la définition indiquée ci-dessus,
éventuellement en présence d'un diluant et le cas échéant en présence d'un accepteur d'acide ;
ou bien
(C) dans le cas des composés de formule (Ic-1) 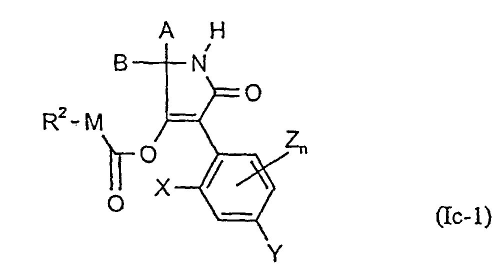 dans laquelle
A, B, X, Y, Z, R² et n ont la définition indiquée ci-dessus,
et
M représente l'oxygène,
on fait réagir des composés de formule (Ia) 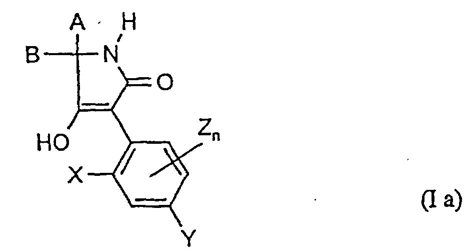 dans laquelle
A, B, X, Y, Z et n ont la définition indiquée ci-dessus,
avec un ester d'acide chloroformique ou un thioester d'acide chloroformique de formule générale (V)
R²-M-CO-Cl (V)
dans laquelle
R² et M ont la définition indiquée ci-dessus,
le cas échéant en présence d'un diluant et en présence éventuelle d'un accepteur d'acide ;
ou bien
(G) dans le cas des composés de formule (If) 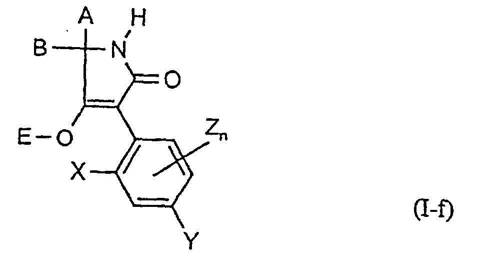 dans laquelle
A, B, X, Y, Z et n ont la définition indiquée ci-dessus,
et
E représente un équivalent d'ion métallique ou un ion ammonium,
on fait réagir des composés de formule (Ia) 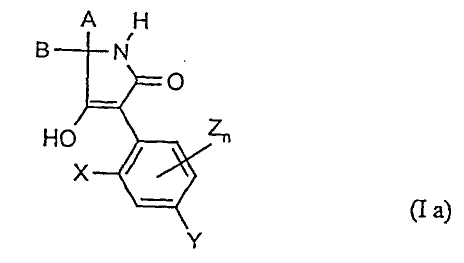 dans laquelle
A, B, X, Y, Z et n ont la définition indiquée ci-dessus,
avec des hydroxydes de métaux ou des amines de formules générales (X) et (XI)
Meₛ OHₜ (X) 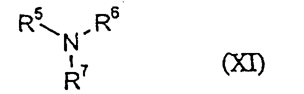
dans lesquels
Me représente des ions métalliques monovalents ou divalents,
s et t représentent les nombres 1 et 2 et
R⁵, R⁶ et R⁷ représentent, indépendamment l'un de l'autre, l'oxygène et un groupe alkyle,
éventuellement en présence d'un diluant.

4. Composés de formule (II) 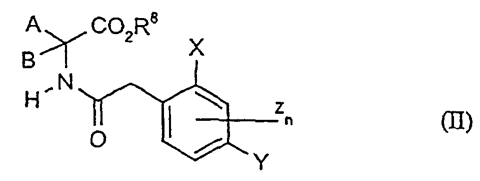 dans laquelle
A, B, X, Y, Z et n ont la définition indiquée dans la revendication 1 et R⁸ a la définition indiquée dans la revendication 4.

5. Procédé de production des composés de formule (II), **caractérisé en ce qu'**on acyle des dérivés d'amino-acides de formule (XIV) 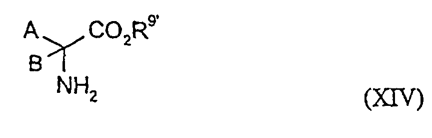 dans laquelle
R^{9'} représente l'hydrogène (XIVa) et un groupe alkyle (XIVb)
et
A et B ont la définition indiquée ci-dessus
avec des halogénures d'acide phénylacétique de formule (XV) 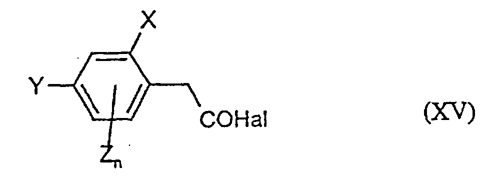 dans laquelle
X, Y, Z et n ont la définition indiquée ci-dessus et
Hal représente le chlore ou le brome
ou bien on estérifie des acylamino-acides de formule (IIa) 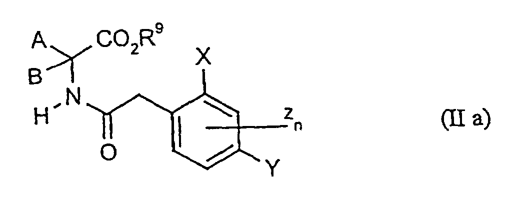 dans laquelle
A, B, X, Y, Z et n ont la définition indiquée ci-dessus, et
R⁹ représente l'hydrogène,
ou bien on fait réagir des aminonitriles de formule (XVI) 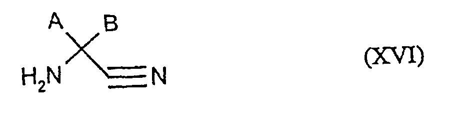 dans laquelle
A et B ont la définition indiquée ci-dessus,
avec des halogénures d'acide phénylacétique de formule (XV) 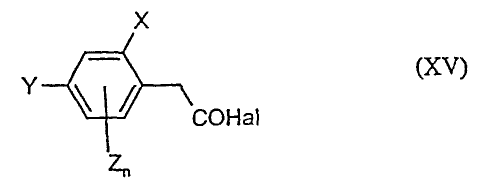 dans laquelle
X, Y, Z et n ont la définition indiquée ci-dessus et
Hal représente le chlore ou le brome,
pour obtenir des composés de formule (XVII) 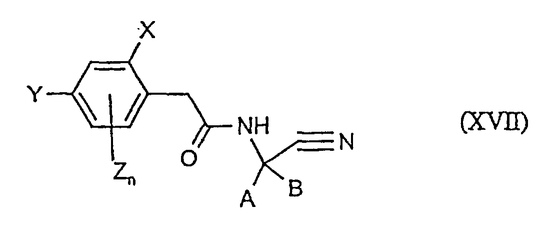 dans laquelle
A, B, X, Y, Z et n ont la définition indiquée ci-dessus, que l'on soumet ensuite à une alcoolyse sulfurique.

6. Composés de formule (XVII) 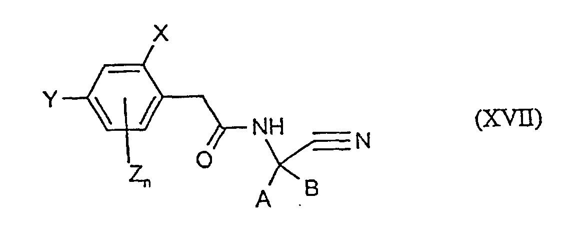 dans laquelle
A, B, X, Y, Z et n ont la définition indiquée dans la revendication 1.

7. Procédé de production des composés de formule (XVII) suivant la revendication 9, **caractérisé en ce qu'**on fait réagir des aminonitriles de formule (XVI) 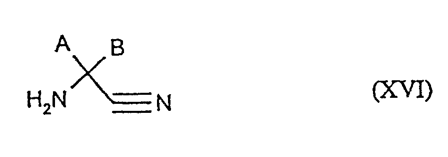 dans laquelle
A et B ont la définition indiquée dans la revendication 9 avec des halogénures d'acide phénylacétique de formule (XV) 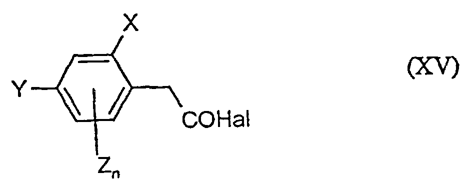 dans laquelle
X, Y, Z et n ont la définition indiquée dans la revendication 9 et
Hal représente le chlore ou le brome.

8. Compositions pesticides et/ou compositions herbicides, **caractérisées par** une teneur en au moins un composé de formule (I) suivant la revendication 1.

9. Compositions arthropodicides, nématicides et herbicides, **caractérisées par** une teneur en au moins un composé de formule (I).

10. Utilisation de composés de formule (I) suivant la revendication 1 pour combattre des parasites dans la protection des plantes, dans le domaine domestique, dans le domaine de l'hygiène et dans la protection des denrées entreposées.

11. Utilisation de composés de formule (I) suivant la revendication 1 pour combattre des arthropodes, des nématodes et la croissance de végétaux indésirables dans la protection des plantes, le domaine domestique, le domaine de l'hygiène et la protection des denrées entreposées.

12. Procédé pour combattre des parasites dans la protection des plantes, dans le domaine domestique, dans le domaine de l'hygiène et dans la protection des denrées entreposées, **caractérisé en ce qu'**on fait agir des composés de formule (I) suivant la revendication 1 sur les parasites et/ou sur leur milieu.

13. Procédé de préparation de compositions pesticides et/ou de compositions herbicides, **caractérisé en ce qu'**on mélange des composés de formule (I) suivant la revendication 1 avec des diluants et/ou des agents tensioactifs.

14. Utilisation de composés de formule (I) suivant la revendication 1 pour la préparation de compositions pesticides destinées à combattre des ectoparasites et des endoparasites.

15. Procédé pour combattre la croissance de plantes indésirables, **caractérisé en ce qu'**on fait agir des composés de formule (I) suivant la revendication 1 sur les mauvaises herbes et/ou sur leur milieu.
